# EUROPEAN PATENT APPLICATION

(11) **EP 4 194 055 A1**
(43) Date of publication of application: **14.06.2023**
(21) Application number: 21854201.7
(22) Date of filing: 09.08.2021
(51) Int. Cl.: A61P 35/00, A61K 35/17, C12N 15/867, C12N 5/10, A61P 35/02, A61P 37/02, C12N 15/113

(54) **ENGINEERED CELLS AND METHOD FOR ENGINEERING CELLS**

(30) Priority: 07.08.2020 CN 202010788848; 16.07.2021 CN 202110807344
(71) Applicant: CRAGE medical Co., Limited, Mongkok Kowloon Hong-Kong (CN)
(72) Inventor: LI, Zonghai, Shanghai 200231 (CN); LIAO, Zhaohui, Shanghai 200231 (CN)
(74) Representative: Lavoix
(86) International application number: PCT/CN2021/111596
(87) International publication number: WO 2022/028623

(57) **Abstract**

Provided are a method for engineering T-cells or pluripotent stem cells, as well as engineered T-cells and pluripotent stem cells. The method specifically comprises: engineering the T cells or pluripotent stem cells to obtain engineered T cell or engineered pluripotent stem cells with reduced expression, activity, and/or signaling of NKG2A. The obtained engineered T cell and a composition containing the engineered T cell can be used to treat diseases, such as cancer (tumor) and autoimmune diseases.

## Description

### RELATED PATENT APPLICATIONS

This patent application claims priority to Chinese patent applications with application number 202010788848.0 filed on August 07, 2020 and the Chinese patent application with application number 202110807344.3 filed on July 16, 2021.

### TECHNICAL FIELD

This application provides engineered T cells and methods for producing engineered T cells. The engineered T cells and compositions comprising the engineered T cells can be used to treat diseases, such as cancer (tumor) and autoimmune diseases.

### BACKGROUND ART

As a cell-based tumor therapy, immune cell therapy is currently the most promising treatment method. Engineered immune cells have the potential to precisely target tumor cells without harming normal tissues; clinical observations indicate that they have significant major anticancer activity. There is still an urgent need to improve immune cell therapy by broadening the applicability and enhancing the efficacy of immune cells.

### CONTENT OF THE APPLICATION

This application provides a method for engineering a T cell or a pluripotent stem cell, and further provides an engineered T cell and a pluripotent stem cell.

In the first aspect of the present application, a method for engineering a T cell or a pluripotent stem cell is provided, the method is: engineering the T cell or pluripotent stem cell to obtain an engineered T cell or engineered pluripotent stem cell with reduced expression, activity and/or signaling of NKG2A.

In some specific embodiments, the engineering transformation comprises modification of a nucleic acid or a protein related to the expression, activity and/or signaling of NKG2A.

In some specific embodiments, the engineering transformation results in a genetic modification that reduces the expression of NKG2A in the engineered T cell or the engineered pluripotent stem cell, or results in a genetic modification that reduces expression of a protein regulating the expression or activity of NKG2A in the engineered T cell or the engineered pluripotent stem cell, or results in a genetic modification that reduces expression of a protein that involved in NKG2A-dependent signaling in the engineered T cell or the engineered pluripotent stem cell.

In some specific embodiments, the engineering transformation is modification of a gene encoding NKG2A, or modification of a gene encoding a protein regulating the expression or activity of NKG2A, or modification of a gene encoding a protein involved in NKG2A-dependent signaling.

In some specific embodiments, the genetic modification comprises deletion, mutation, and/or insertion; preferably, the genetic modification makes changes to the open reading frame of NKG2A, the protein regulating the expression or activity of NKG2A, or the protein involved in NKG2A-dependent signaling.

In some specific embodiments, at least one allele of the NKG2A gene, a gene encoding the protein regulating the expression or activity of NKG2A, or a gene encoding the protein involved in NKG2A-dependent signaling is disrupted; preferably, both the alleles are disrupted.

In some specific embodiments, the engineering transformation comprises administering an effective amount of inhibitory nucleic acid molecules to the T cell or pluripotent stem cell, the inhibitory nucleic acid molecules are capable of inhibiting the expression of NKG2A in the T cell or the pluripotent stem cell, or are capable of inhibiting the expression of a protein capable of upregulating the expression or activity of NKG2A of the T cell or the pluripotent stem cell, or are capable of inhibiting the expression of a protein participating in NKG2A-dependent signaling of the T cell or the pluripotent stem cell; preferably, the inhibitory nucleic acid molecule comprises a sequence complementary to the gene encoding NKG2A, the gene inhibiting the protein up-regulating the expression or activity of NKG2A, or the gene of the protein involved in NKG2A-dependent signaling.

In some specific embodiments, the inhibitory nucleic acid comprises an RNA interfering agent.

In some specific embodiments, the inhibitory nucleic acid comprises an siRNA, shRNA or miRNA.

In some specific embodiments, the genetic modification is modification by gene editing technology; preferably, the gene editing technology is selected from CRISPR/Cas9 technology, artificial Zinc Finger Nucleases (ZFN) technology, transcription activator-like effector (TALE) technology or TALE-CRISPR/Cas9 technology; more preferably, the gene editing technology is CRISPR/Cas9 technology.

In some preferred embodiments, the gRNA used in the CRISPR/Cas9 technology is selected from the sequences as shown by SEQ ID NO: 12, SEQ ID NO: 13, SEQ ID NO:14 or SEQ ID NO:15.

In some specific embodiments, the reduced expression, activity and/or signaling of NKG2A is permanent, transient or inducible.

In some specific embodiments, the T cell is a natural T cell, or a T cell capable of recognizing a specific target antigen; preferably, the T cell is a cell comprising a specific T cell subset; more preferably, the specific T cell subset is a memory stem cell-like T cell (Tscm cell), central memory T cell (Tcm), effector T cell (Tef), regulatory T cell (Tregs), effector memory T cell (Tern) or γ δ T cell.

In some specific embodiments, the pluripotent stem cell is a natural pluripotent stem cell or a pluripotent stem cell capable of recognizing a specific target antigen.

In some specific embodiments, the target antigen is a tumor antigen or a pathogen antigen.

In some specific embodiments, the target antigen is selected from Claudin18.2, Claudin18.1, Claudin 6, vascular endothelial growth factor receptor, glypican-3 (GPC3), B cell maturation antigen (BCMA), carbonic anhydrase 9 (CAIX), tEGFR, CD19, CD20, CD22, mesothelin, CEA and hepatitis B surface antigen, antifolate receptor, CD23, CD24, CD30, CD33, CD38, CD44, EGFR, Epithelial Glycoprotein 2 (EPG-2), Epithelial Glycoprotein 40 (EPG-40), EPHa2, erb-B2, erb-B3, erb-B4, erbB dimer, EGFR vIII, folate binding protein (FBP), FCRL5, FCRH5, fetal acetylcholine receptor, GD2, GD3, HMW-MAA, IL-22R-α, IL-13R-α 2, kinase insertion domain receptor (kdr), κ light chain, Lewis Y, L1 cell adhesion molecule (L1-CAM), melanoma-associated antigen (MAGE)-A1, MAGE-A3, MAGE-A6, preferentially expressed melanoma antigen (PRAME), survivin, TAG72, B7-H6, IL-13 receptor α2 (IL -13Ra2), CA9, GD3, HMW-MAA, CD171, G250/CAIX, HLA-AI MAGEA1, HLA-A2, PSCA, folate receptor-a, CD44v6, CD44v7/8, avb6 integrin, 8H9, NCAM, VEGF Receptor, 5T4, fetal AchR, NKG2D Ligand, CD44v6, dual antigen, cancer-testis antigen, mesothelin, murine CMV, mucin 1 (MUC1), MUC16, PSCA, NKG2D, NY-ESO-1, MART- 1, gp100, G protein-coupled receptor 5D (GPCR5D), ROR1, TAG72, VEGF-R2, carcinoembryonic antigen (CEA), prostate-specific antigen, PSMA, Her2/neu, estrogen receptor, progesterone receptor, Ephrin B2, CD123, c-Met, GD-2, O-acetylated GD2 (OGD2), CE7, Wilms tumor 1 (WT-1), cyclin, cyclin A2, CCL-1, CD138, pathogen-specific antigens, and antigens associated with universal labels.

In some specific embodiments, the T cell recognizing a specific target antigen or the pluripotent stem cell recognizing a specific target antigen express an exogenous protein that recognizes the specific target antigen; preferably, the exogenous protein comprises an antibody or ligand that recognizes the target antigen.

In some specific embodiments, the exogenous protein is a chimeric antigen receptor, chimeric T cell receptor, or T cell antigen coupler (TAC).

In some specific embodiments, the exogenous protein recognizes at least one target antigen.

In some specific embodiments, the exogenous protein recognizes two or three target antigens.

In some specific embodiments, the two or three target antigens are expressed in a same type of cells, or a same type of pathogens, or a same organ.

In some specific embodiments, the two or three target antigens target different cells.

In some specific embodiments, the target antigens comprise NKG2A; preferably, the target antigens further comprise tumor antigens. In specific embodiments, the tumor antigen is BCMA or CD 19.

In some specific embodiments, the T cell carrying an exogenous protein recognizing a target antigen or the pluripotent stem cell carrying an exogenous protein recognizing a target antigen contain a first exogenous protein and a second exogenous protein capable of recognizing different target antigens.

In some specific embodiments, the antigens recognized by the first exogenous protein and the second exogenous protein are expressed in the same type of cells, or the same type of pathogen, or the same organ.

In some specific embodiments, the antigens recognized by the first exogenous protein and the second exogenous protein are expressed in different cells; preferably, the first exogenous protein recognizes a tumor antigen or a pathogen antigen, and the second exogenous protein recognizes NKG2A.

In some specific embodiments, the engineered T cell or engineered pluripotent stem cell comprises a protein recognizing NKG2A.

In some specific embodiments, the T cell is obtained from below sources including: PBMC, bone marrow, lymph node tissue, cord blood, thymus tissue, tissue from a site of infection, ascites, pleural effusion, spleen tissue, or tumor tissue.

In some specific embodiments, the method further comprises endogenous TCR molecular silencing in the engineered T cell or pluripotent stem cell; further preferably, endogenous MHC molecular silencing in the engineered T cell or pluripotent stem cell.

In some specific embodiments, the TCR molecular silencing refers to the silencing of genes encoding one or both of the α and β chains of TCR; preferably, the TCR molecular silencing refers to the silencing of the gene encoding the α chain of TCR (i.e., the TRAC gene); more preferably, the TCR molecular silencing refers to the silencing of the constant region of the gene encoding the α chain of TCR; further preferably, the TCR molecular silencing refers to the silencing of the first exon of the constant region of the gene encoding the α chain of TCR.

In some specific embodiments, the MHC molecule refers to an HLA molecule. In a specific embodiment, the HLA molecule is selected from HLA-I class and/or HLA-II molecules, including at least one of HLA-A, HLA-B, HLA-C, B2M and CIITA molecules. In some specific embodiments, the HLA molecule is a class I HLA molecule. In some specific embodiments, the HLA molecule is a B2M molecule.

In some specific embodiments, gene editing technology is used to silence endogenous TCR molecules and endogenous MHC molecules. In a specific embodiment, the gene editing technology is selected from CRISPR/Cas technology, artificial Zinc Finger Nucleases (ZFN) technology, transcription activator-like effector (TALE) technology or TALE - CRISPR/Cas technology; preferably, CRISPR/Cas technology is used; more preferably, CRISPR/Cas9 technology is used.

In some specific embodiments, when using CRISPR/Cas9 technology for B2M molecular silencing, the sequence of the selected gRNA is as shown by SEQ ID NO:54.

In some specific embodiments, when CRISPR/Cas9 technology is used for α chain molecular silencing of TCR, the sequence of the selected gRNA is as shown by SEQ ID NO:53.

In the second aspect of the present application, provided is a method for gene editing of NKG2A in a cell based on the CRISPR/Cas system, wherein the gRNA used comprises a sequence as shown by SEQ ID NO: 12, SEQ ID NO: 13, SEQ ID NO: 14 or SEQ ID NO: 15.

In some specific embodiments, the Cas enzyme is a Cas9 enzyme or Cas12 enzyme.

In some specific embodiments, the complex of a Cas enzyme and gRNA is introduced into the cell for gene editing, and the enzyme activity of the Cas9 enzyme is 0.1-1 nmol, preferably 0.2-0.7 nmol, more preferably 0.3-0.5 nmol, most preferably 0.37 nmol.

In some specific embodiments, the molar ratio of Cas9 enzyme and gRNA is 1:1∼1:10, preferably 1:3~1:5, more preferably 1:4.

In some specific embodiments, the cell is an eukaryotic cell; preferably the eukaryotic cell is a T cell or pluripotent stem cell.

In some specific embodiments, the TRAC and/or the B2M gene of the cell are further edited.

In some specific embodiments, the T cell or pluripotent stem cell further expresses a chimeric receptor, an exogenous cytokine, an inhibitory/activating receptor or ligand, or a costimulatory factor.

In a third aspect of the present application, provided is an RNA construct comprising a nucleotide sequence selected from SEQ ID NO: 12, SEQ ID NO: 13, SEQ ID NO:14 or SEQ ID NO:15.

In some specific embodiments, the gRNA construct is used to guide the recognition by the Cas enzyme; in a specific embodiment, the Cas enzyme is a natural Cas enzyme or an engineered Cas enzyme that does not have cleavage activity.

In the fourth aspect of the present application, provided is an engineered T cell, wherein the engineered T cell has reduced expression, activity and/or signaling of NKG2A.

In some specific embodiments, the engineering refers to genetic engineering.

In some specific embodiments, the genetic engineering comprises: genetic modification that results in reduced expression of NKG2A in the engineered T cell, a genetic modification that results in reduced expression of a protein regulating the expression or activity of NKG2A, or a genetic modification that results in reduced expression of a protein that is involved in NKG2A-dependent signaling.

In some specific embodiments, the genetic modification that results in reduced expression of NKG2A in the engineered T cell is a modification of a gene encoding NKG2A;
The genetic modification that results in the expression reduction of the protein regulating the expression or activity of NKG2A refers to the modification of the gene encoding the protein regulating the expression or activity of NKG2A;
The genetic modification that reduces the expression of a protein involved in NKG2A-dependent signaling refers to the modification of a gene encoding a protein involved in NKG2A-dependent signaling.

In some specific embodiments, the genetic modification includes deletion, mutation, and/or insertion; preferably, the genetic modification makes changes in the open reading frame of NKG2A, the protein regulating the expression or activity of NKG2A, or the protein involved in NKG2A-dependent signaling.

In some specific embodiments, at least one allele of the NKG2A gene, the gene encoding the protein regulating the expression or activity of NKG2A, and/or the gene encoding the protein involved in NKG2A-dependent signaling is disrupted; preferably, both the alleles are disrupted.

In some specific embodiments, the reduced expression, activity and/or signaling of NKG2A is permanent, transient or inducible.

In some specific embodiments, the engineered T cell further expresses an exogenous protein recognizing a target antigen.

In some specific embodiments, when the engineered T cell is stimulated by the target antigen, the expression of NKG2A is not up-regulated.

In some specific embodiments, preferably, the exogenous protein is a chimeric antigen receptor (CAR), a chimeric T cell receptor (TCR), a T cell antigen coupler (TAC).

In some specific embodiments, the target antigen is a tumor antigen or a pathogen antigen.

In some specific embodiments, when the conditions leading to the up-regulation of NKG2A expression occur, the NKG2A expression of the engineered T cell is not up-regulated or the up-regulation is weaker.

In some specific embodiments, compared with a T cell that has not been engineered for reduced expression, activity, and/or signaling of NKG2A, the expression of NKG2A in the engineered T cell is not up-regulated or the up-regulation is weaker.

In some specific embodiments, the expression of the NKG2A is reduced by more than about 50%, 60%, 70%, 80%, 90%, or 95%.

In some specific embodiments, the expression of NKG2A is determined by immunoblotting or flow cytometry.

In some specific embodiments, compared to a T cell without reduced expression, activity, and/or signaling of NKG2A, the immune activity of the engineered T cell is increased or increased by about 10%, 20%, 30%, 40%, 50%, 60%, 70%, 80%, 90% or 100%.

In some specific embodiments, the tumor antigen is BCMA or CD19; preferably, the exogenous protein is a chimeric antigen receptor containing an antibody that can specifically recognize BCMA; more preferably, the antibody specifically recognizing BCMA comprises HCDR1 as shown by SEQ ID NO:16, HCDR2 as shown by SEQ ID NO:17, HCDR3 as shown by SEQ ID NO:18, and LCDR1 as shown by SEQ ID NO:19, LCDR2 as shown by SEQ ID NO:20, LCDR3 as shown by SEQ ID NO:21; further preferably, the antibody specifically recognizing BCMA or a functional fragment thereof comprises the heavy chain variable region as shown by SEQ ID NO:22 and light chain variable region as shown by SEQ ID NO:23.

In some specific embodiments, the endogenous TCR of the engineered T cell is not expressed;
In some specific embodiments, a gene encoding one or both of the α and β chains of TCR is silenced; preferably, the TCR molecular silencing refers to the silencing of the gene encoding the α chain of TCR (i.e., TRAC gene); more preferably, the TCR molecular silencing refers to the silencing of the gene encoding the α-chain constant region of TCR; further preferably, the TCR molecular silencing refers to the silencing of the first exon of the gene encoding the α-chain constant region of TCR.

In some specific embodiments, the expression, activity and/or signaling of a MHC molecule in the engineered T cell is reduced; preferably, the MHC molecule refers to an HLA molecule; more preferably, the HLA molecule is selected from HLA-class I and/or HLA-II molecules, including at least one of HLA-A, HLA-B, HLA-C, B2M and CIITA molecules.

In some specific embodiments, the HLA molecule is a class I HLA molecules.

In some specific embodiments, the HLA molecule is a B2M molecule.

In some specific embodiments, the engineered T cell further expresses an antibody recognizing NKG2A or a functional fragment thereof.

In some specific embodiments, the antibody recognizing NKG2A comprises HCDR1 as shown by SEQ ID NO:3, HCDR2 as shown by SEQ ID NO:4, HCDR3 as shown by SEQ ID NO:5, LCDR1 as shown by SEQ ID NO:6, LCDR2 as shown by SEQ ID NO:7, LCDR3 as shown by SEQ ID NO:8; preferably, the antibody recognizing NKG2A comprises the heavy chain variable region as shown by SEQ ID NO:1 or the light chain variable region as shown by SEQ ID NO:2.

In some specific embodiments, the engineered T cell further expresses a chimeric antigen receptor (CAR), a chimeric T cell receptor, a T cell antigen coupler (TAC) specifically binding to NKG2A; preferably, the CAR comprises:
(i) an antibody specifically binding to NKG2A or a functional fragment thereof, the transmembrane region of CD28 or CD8, the co-stimulatory signal domain of CD28 and CD3ζ; and/or
(ii) an antibody specifically binding to NKG2A or a functional fragment thereof, the transmembrane region of CD28 or CD8, the co-stimulatory signal domain of CD137 and CD3ζ; and/or
(iii) an antibody specifically binding to NKG2A or a functional fragment thereof, the transmembrane region of CD28 or CD8, the co-stimulatory signal domain of CD28, the costimulatory signal domain of CD137, and CD3ζ.

In some specific embodiments, the engineered T cell expresses a chimeric antigen receptor targeting NKG2A and a chimeric antigen receptor targeting a tumor antigen; preferably, the engineered T cell expresses a chimeric antigen receptor targeting NKG2A and a chimeric antigen receptor targeting BCMA or CD19.

In some specific embodiments, the engineered T cell expresses a chimeric antigen receptor as shown by SEQ ID NO:25 and SEQ ID NO:9.

In some specific embodiments, the engineered T cell expresses an antibody recognizing NKG2A and an antibody recognizing a tumor antigen.

In some specific embodiments, the antibody recognizing NKG2A and the antibody recognizing the tumor antigen are linked to form a tandem antibody.

In some specific embodiments, the antibody recognizing NKG2A and the antibody recognizing the tumor antigen are linked by a connecting chain; preferably, the connecting chain is selected from sequences as shown by SEQ ID NO:34, SEQ ID NO:35, SEQ ID NO:36, SEQ ID NO:37, SEQ ID NO:38, or SEQ ID NO:39.

In some specific embodiments, the linkage mode of the antibody recognizing NKG2A and the antibody recognizing the tumor antigen is:
(1) Light chain/heavy chain (or light chain variable region/heavy chain variable region) of an antibody recognizing NKG2A-heavy chain/light chain (or heavy chain variable region/light chain variable region) of the antibody recognizing NKG2A—the heavy chain/light chain (or heavy chain variable region/light chain variable region) of the antibody recognizing the tumor antigen-the light chain/heavy chain of the antibody recognizing the tumor antigen (or the light chain variable region/heavy chain variable region);
(2) The light chain (or light chain variable region) of the antibody recognizing the tumor antigen-the heavy chain (or heavy chain variable region) of the antibody recognizing NKG2A—the light chain (or light chain variable region) of the antibody recognizing NKG2A—the heavy chain (or heavy chain variable region) of the antibody recognizing the tumor antigen; or
(3) The light chain (or light chain variable region) of the antibody recognizing NKG2A—the heavy chain (or heavy chain variable region) of the antibody recognizing the tumor antigen-the light chain (or light chain variable region) of the antibody recognizing the tumor antigen - the heavy chain (or heavy chain variable region) of the antibody recognizing NKG2A.

In some specific embodiments, the tandem antibody has a transmembrane domain and an intracellular domain linked to form an exogenous receptor expressed on the engineered T cell.

In some specific embodiments, the intracellular domain is a cytoplasmic signaling domain; preferably, the intracellular domain further comprises a co-stimulatory signal domain.

In some specific embodiments, the tumor antigen is BCMA.

In some specific embodiments, the sequence of the tandem antibody comprises VH as shown by SEQ ID NO:1, VL as shown by SEQ ID NO:2, VH as shown by SEQ ID NO:22, and VL as shown by SEQ ID NO: 23.

In some specific embodiments, wherein the amino acid sequence of the exogenous receptor is as shown by SEQ ID NO:26, SEQ ID NO:55 or SEQ ID NO:57.

In the fifth aspect of the present application, provided is the use of a first CAR T cell targeting NKG2A in combination with a second CAR T cell targeting a target antigen for disease treatment, the expression, activity and/or signaling of the endogenous NKG2A is reduced in the first CAR T cell and/or the second CAR T cell; preferably, the target antigen is a tumor antigen, and the disease is a tumor.

In some specific embodiments, the expression, activity and/or signaling of the endogenous NKG2A in the first CAR T cell and/or the second CAR T cell is reduced.

In some specific embodiments, the second CAR T cell recognizes a tumor antigen.

In some specific embodiments, the tumor antigens are CD19 and BCMA.

In some specific embodiments, the TCR molecule of the first CAR T cell and/or the second CAR T cell is silenced.

In some specific embodiments, the TCR molecular silencing refers to the silencing of a gene encoding one or both of the α and β chains of TCR; preferably, the TCR molecular silencing refers to the silencing of the gene encoding the α chain of TCR (i.e., the TRAC gene); more preferably, the TCR molecular silencing refers to the silencing of the constant region of the gene encoding the α chain of TCR; further preferably, the TCR molecular silencing refers to the silencing of the first exon of the constant region of the gene encoding the α chain of TCR.

In some specific embodiments, an endogenous MHC molecule of the first CAR T cell and/or the second CAR T cell is silenced.

In some specific embodiments, the MHC molecule refers to an HLA molecule; preferably, the HLA molecule is selected from HLA-I class and/or HLA-II molecules, including at least one of HLA-A, HLA-B, HLA-C, B2M and CIITA molecules; more preferably, the HLA molecule is a class I HLA molecule.

In some specific embodiments, the HLA molecule is B2M molecule.

In some specific embodiments, gene editing technology is used to silence endogenous TCR molecules, endogenous MHC molecules, or reduce expression, activity and/or signaling of endogenous NKG2A.

In the sixth aspect of the present application, provided is the use of a first CAR T cell targeting NKG2A in combination with a second CAR T cell targeting a target antigen in the treatment of a disease, wherein an inhibition agent of NKG2A protein is administered at the same time.

In some specific embodiments, the TCR molecule of the first CAR T cell and/or the second CAR T cell is silenced.

In some specific embodiments, the TCR molecular silencing refers to the silencing of a gene encoding one or both of the α and β chains of TCR; preferably, the TCR molecular silencing refers to the silencing of the gene encoding the α chain of TCR (i.e., the TRAC gene); more preferably, the TCR molecular silencing refers to the silencing of the constant region of the gene encoding the α chain of TCR; further preferably, the TCR molecular silencing refers to the silencing of the first exon of the constant region of the gene encoding the α chain of TCR.

In some specific embodiments, an endogenous MHC molecule of the first CAR T cell and/or the second CAR T cell is silenced.

In some specific embodiments, the MHC molecule refers to an HLA molecule; preferably, the HLA molecule is selected from HLA-I class and/or HLA-II molecules, including at least one of HLA-A, HLA-B, HLA-C, B2M and CIITA molecules; more preferably, the HLA molecule is a class I HLA molecule.

In specific embodiments, the HLA molecule is the B2M molecule.

In the seventh aspect of the present application, provided is a composition comprising an effective amount of the engineered T cells described in the fourth aspect of the present application. Preferably, a pharmaceutically acceptable carrier is further comprised; preferably, the carrier is saline solution, dextrose solution or 5% human serum albumin. Preferably, a cryoprotectant is also comprised.

In the eighth aspect of the present application, provided is an engineered T cell genetically engineered to reduce the expression, activity and/or signaling of NKG2A in the cell.

In some specific embodiments, the engineered T cell comprises:
gene disruption of a gene encoding NKG2A and/or gene disruption resulting in reduced expression of NKG2A in the engineered T cell;
gene disruption of a gene encoding the expression or activity of NKG2A protein and/or gene disruption resulting in reduced expression of a protein regulating the expression or activity of NKG2A; and/or
gene disruption of a gene encoding a protein involved in NKG2A-dependent signaling and/or gene disruption resulting in reduced expression of a protein involved in NKG2A-dependent signaling.

In some specific embodiments, the gene disruption comprises deletions, mutations and/or insertions resulting in premature termination codons in the genes or frameshift in the open reading frame of the genes.

In some specific embodiments, two alleles of the gene encoding NKG2A, the gene encoding the protein regulating the expression or activity of NKG2A, and/or the gene encoding the protein involved in NKG2A-dependent signaling in the engineered T cell are all destroyed.

In some specific embodiments, the engineered T cell comprises an inhibitory nucleic acid molecule targeting a gene in the T cell, resulting in reduced expression of NKG2A, reduced expression of the protein that regulates expression or activity of NKG2A, and/or reduced expression of the protein involved in NKG2A-dependent signaling.

In some specific embodiments, compared with the expression of the protein in the non-engineered T cell, the expression of NKG2A, the protein regulating the expression or activity of NKG2A, and/or the protein involved in NKG2A-dependent signaling in the engineered T cell is reduced by greater than 50%, 60%, 70%, 80%, 90%, or 95%, or reduced by greater than about 50%, 60%, 70%, 80%, 90%, or 95%.

In some specific embodiments, the expression of NKG2A is reduced in the engineered T cell.

In the ninth aspect of the present application, provided is an engineered T cell comprising gene disruption in a gene encoding NKG2A, wherein the expression of NKG2A in the cell is reduced.

In some specific embodiments, the gene disruption comprises deletions, mutations and/or insertions resulting in premature termination codons in the gene or frameshifts in the open reading frame of the gene.

In some specific embodiments, both alleles of the gene encoding NKG2A are disrupted in the genome of the engineered T cell.

In some specific embodiments, the engineered T cell comprises an inhibitory nucleic acid molecule targeting the gene encoding NKG2A.

In some specific embodiments, the inhibitory nucleic acid molecule comprises a sequence complementary to the gene encoding NKG2A.

In some specific embodiments, the inhibitory nucleic acid comprises an RNA interfering agent.

In some specific embodiments, the inhibitory nucleic acid comprises siRNA, shRNA or miRNA, preferably, the inhibitory nucleic acid sequence comprises the sequence as shown by SEQ ID NO: 12, 13, 14 or 15.

In some specific embodiments, the reduced expression, activity and/or signaling of NKG2A is permanent, transient or inducible.

In some specific embodiments, compared with the expression, activity and/or signaling of NKG2A in non-engineered T cell, the expression, activity and/or signaling of NKG2A in the engineered T cell is reduced by greater than 50%, 60%, 70%, 80%, 90% or 95%, or is reduced by greater than about 50%, 60%, 70%, 80%, 90% or 95%.

In some specific embodiments, the expression of NKG2A in the cells is not detectable in immunoblot assays and/or in flow cytometry.

In some specific embodiments, compared with a non-genetically engineered T cell, the immune activity of the engineered T cell is increased by 10%, 20%, 30%, 40%, 50%, 60%, 70%, %, 80%, 90% or 100%, or is increased by about 10%, 20%, 30%, 40%, 50%, 60%, 70%, %, 80%, 90% or 100%.

In some specific embodiments, compared with a non-genetically engineered T cell, the inhibition of tumor cell proliferation *in vitro,* and/or inhibition of tumor cell proliferation, and tumor volume *in vivo* in the engineered T cell is increased by 10%, 20% %, 30%, 40%, 50%, 60%, 70%, 80%, 90%, or 100%, or increased by about 10%, 20% %, 30%, 40%, 50%, 60%, 70%, 80%, 90%, or 100%.

In some specific embodiments, the cell further expresses a chimeric antigen receptor (CAR), modified T cell (antigen) receptor (TCR), T cell fusion protein (TFP), T cell antigen coupler (TAC), aTCR-T or a combination thereof. Preferably, the CAR comprises:
(i) an antibody specifically binding to a target antigen or a functional fragment thereof, the transmembrane region of CD28 or CD8, the co-stimulatory signal domain of CD28 and CD3ζ; and/or
(ii) an antibody specifically binding to a target antigen or a functional fragment thereof, the transmembrane region of CD28 or CD8, the co-stimulatory signal domain of CD137 and CD3ζ; and/or
(iii) an antibody specifically binding to the target antigen or a functional fragments thereof, the transmembrane region of CD28 or CD8, the costimulatory signal domain of CD28, and the costimulatory signa 1 domain of CD137 and CD3ζ;

Preferably, the target antigens include tumor antigens and pathogen antigens;
Preferably, the tumor antigens include Claudin18.2, Claudin18.1, Claudin 6, vascular endothelial growth factor receptor, glypican-3 (GPC3), B cell maturation antigen (BCMA), carbonic anhydrase 9(CAIX), tEGFR, CD19, CD20, CD22, mesothelin, CEA and hepatitis B surface antigen, antifolate receptor, CD23, CD24, CD30, CD33, CD38, CD44, EGFR, epithelial glycoprotein 2 (EPG -2), epithelial glycoprotein 40 (EPG-40), EPHa2, erb-B2, erb-B3, erb-B4, erbB dimer, EGFR vIII, folate binding protein (FBP), FCRL5, FCRH5, fetal acetylcholine receptor, GD2, GD3, HMW-MAA, IL-22R-α, IL-13R-α2, Kinase Insertion Domain Receptor (kdr), κ light chain, Lewis Y, L1 Cell Adhesion Molecule (L1-CAM), melanoma-associated antigen (MAGE)-A1, MAGE-A3, MAGE-A6, preferentially expressed melanoma antigen (PRAME), survivin, TAG72, B7-H6, IL-13 receptor α 2 (IL-13Ra2), CA9, GD3, HMW-MAA, CD171, G250/CAIX, HLA-AI MAGEA1, HLA-A2, PSCA, folate receptor-a, CD44v6, CD44v7/8, avb6 integrin, 8H9, NCAM, VEGF receptor, 5T4, fetal AchR, NKG2D ligand, CD44v6, dual antigen, cancer-testis antigen, mesothelin, murine CMV, mucin 1 (MUC1), MUC16, PSCA, NKG2D, NY-ESO-1, MART-1, gp100, G protein-coupled receptor 5D (GPCR5D), ROR1, TAG72, VEGF-R2, carcinoembryonic antigen (CEA), prostate-specific antigen, PSMA, Her2/neu, estrogen receptor, progesterone receptor, ephrin B2, CD123, c-Met, GD-2, O-acetylated GD2 (OGD2), CE7, Wilms tumor 1 (WT-1), cyclins, cyclin A2, CCL-1, CD138, pathogen-specific antigens and antigens associated with common labels;
Preferably, the CAR targeting BCMA comprises an antibody capable of specifically recognizing BCMA;
Preferably, the antibody specifically recognizing BCMA comprises HCDR1 as shown by SEQ ID NO: 16, HCDR2 as shown by SEQ ID NO: 17, HCDR3 as shown by SEQ ID NO: 18, and LCDR1 as shown by SEQ ID NO: 19, LCDR2 as shown by SEQ ID NO: 20, LCDR3 as shown by SEQ ID NO: 21;
Preferably, the antibody specifically recognizing BCMA or a functional fragment thereof comprises the heavy chain variable region as shown by SEQ ID NO:22 and the light chain variable region as shown by SEQ ID NO:23.

In some specific embodiments, the cell is an autologous T cell derived from a human; preferably, the cell is a primary T cell.

In a specific embodiment, MHC expression, activity and/or signaling in the cell is reduced; preferably, the MHC is an MHC class I molecule; more preferably, the MHC I is HLA; more preferably, the HLA is a HLA-I; more preferably, the HLA-I is one or more selected from the group consisting of: HLA-A, HLA-B, HLA-C, and B2M; most preferably, HLA-I comprise HLA-A and B2M;
Preferably, the engineered T cell comprises an inhibitory nucleic acid molecule targeting a gene encoding MHC;
Preferably, the inhibitory nucleic acid molecule comprises a sequence complementary to the gene encoding MHC;
Preferably, the inhibitory nucleic acid comprises an RNA interfering agent;
Preferably, the inhibitory nucleic acid comprises siRNA, shRNA or miRNA;
Preferably, the reduction in MHC expression, activity and/or signaling is permanent, transient or inducible;
Preferably, compared with the expression, activity and/or signaling of MHC in a non-genetically engineered T cell, the expression, activity and/or signaling of MHC in the engineered T cell is reduced by greater than 50%, 60%, 70%, 80%, 90%, 95% or 100%, or is reduced by greater than about 50%, 60%, 70%, 80%, 90%, 95% or 100%;
Preferably, the expression of MHC expressed in the engineered T cell is undetectable by immunoblotting assay and/or flow cytometry.

In some specific embodiments, the cell further expresses an antibody recognizing NKG2A or a functional fragment thereof, and/or expresses a chimeric antigen receptor (CAR), a modified T cell (antigen) receptor (TCR), a T cell fusion protein (TFP), a T cell antigen coupler (TAC), aTCR-T, or a combination thereof specifically binding to NKG2A. Preferably, the CAR comprises:
(i) an antibody specifically binding to NKG2A or a functional fragment thereof, the transmembrane region of CD28 or CD8, the co-stimulatory signal domain of CD28 and CD3ζ; and/or
(ii) an antibody specifically binding to NKG2A or a functional fragment thereof, the transmembrane region of CD28 or CD8, the co-stimulatory signal domain of CD 137 and CD3ζ; and/or
(iii) an antibody specifically binding to NKG2A or a functional fragment thereof, the transmembrane region of CD28 or CD8, the costimulatory signal domain of CD28, the costimulatory signal domain of CD137 and CD3ζ;

Preferably, the antibody specifically binding to NKG2A comprises HCDR1 as shown by SEQ ID NO: 3, HCDR2 as shown by SEQ ID NO: 4, HCDR3 as shown by SEQ ID NO: 5, LCDR1 as shown by SEQ ID NO: 6, LCDR2 as shown by SEQ ID NO: 7, LCDR3 as shown by SEQ ID NO: 8;
Preferably, the antibody recognizing NKG2A comprises the heavy chain variable region as shown by SEQ ID NO:1 or the light chain variable region as shown by SEQ ID NO:2.

In some specific embodiments, the cell is derived from an allogeneic T cell; preferably, the cell is derived from a human T cell; preferably, the cell is a primary T cell.

The tenth aspect of the present application provides a method for producing an engineered T cell, comprising genetically engineering the T cell to reduce the expression, activity and/or signaling of NKG2A in the cell. Preferably, reducing expression comprises:
disrupting or inhibiting a gene encoding NKG2A and/or disrupting or inhibiting a gene in the T cell, resulting in reduced expression of NKG2A;
disrupting or inhibiting a gene encoding a protein regulating the expression or activity of NKG2A and/or disrupting or inhibiting a gene in the T cell, resulting in reduced expression of the protein regulating the expression or activity of NKG2A; and/or
disrupting or inhibiting a gene encoding a protein involved in NKG2A-dependent signaling and/or disrupting or inhibiting a gene in the T cell, resulting in reduced expression of the protein involved in NKG2A-dependent signaling;
Preferably, comprising introducing a deletion, mutation or insertion into the gene;
Preferably, the gene encodes NKG2A;
Preferably, the gene is disrupted or inhibited by introducing into the T cell an endonuclease targeting the gene under conditions allowing the gene to be disrupted or inhibited;
Preferably, the endonuclease is selected from TAL nuclease, meganuclease, zinc finger nuclease, Cas9 and Argonaute;
Preferably, disruption or inhibition is achieved by introducing into the T cell an inhibitory nucleic acid targeting the gene under conditions resulting in inhibition of the gene;
Preferably, the inhibitory nucleic acid molecule comprises a sequence complementary to the gene encoding NKG2A;
Preferably, the inhibitory nucleic acid comprises an RNA interfering agent;
Preferably, the nucleic acid is siRNA, shRNA or miRNA;
Preferably, the reduction in expression is permanent, transient or inducible;
Preferably, compared with the expression in a non-genetically engineered T cell, the expression, activity and/or signaling of NKG2A is reduced by greater than or more than about 50%, 60%, 70%, 80%, 90% or 95%;
Preferably, the NKG2A expression level cannot be detected by immunoblotting assay and/or flow cytometry;
Preferably, comprising isolating a T cell from a human prior to the genetic engineering;
Preferably, comprising isolating a T cell from peripheral blood mononuclear cells (PBMC);
Preferably, isolating the T cell comprises selecting the T cell from PBMCs using T cell markers;
Preferably, the T cell markers are CD4, CD8.

In a specific embodiment, the cell is genetically engineered to express a chimeric antigen receptor (CAR), a modified T cell (antigen) receptor (TCR), a T cell fusion protein (TFP), a T cell antigen coupler (TAC), aTCR-T or a combination thereof. Preferably, the CAR comprises:
(i) an antibody specifically binding to a target antigen or a functional fragment thereof, the transmembrane region of CD28 or CD8, the co-stimulatory signal domain of CD28, and CD3ζ; and/or
(ii) an antibody specifically binding to a target antigen or a functional fragment thereof, the transmembrane region of CD28 or CD8, the co-stimulatory signal domain of CD137, and CD3ζ; and/or
(iii) an antibody specifically binding to a target antigen or a functional fragment thereof, the transmembrane region of CD28 or CD8, the costimulatory signal domain of CD28, the costimulatory signal domain of CD137 and CD3ζ;

Preferably, the target antigens comprise tumor antigens and pathogen antigens;
Preferably, the tumor antigens include Claudin18.2, Claudin18.1, Claudin 6, vascular endothelial growth factor receptor, glypican-3 (GPC3), B cell maturation antigen (BCMA), carbonic anhydrase 9 (CAIX), tEGFR, CD19, CD20, CD22, mesothelin, CEA and hepatitis B surface antigen, antifolate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimer, EGFR vIII, folate binding protein (FBP), FCRL5, FCRH5, fetal acetylcholine receptor, GD2, GD3, HMW-MAA, IL-22R-α, IL-13R-α2, Kinase Insertion Domain Receptor (kdr), κ light chain, Lewis Y, L1 Cell Adhesion Molecule (L1-CAM), Melanoma-associated antigen (MAGE)-A1, MAGE-A3, MAGE-A6, preferentially expressed melanoma antigen (PRAME), survivin, TAG72, B7-H6, IL-13 receptor alpha 2 (IL-13Ra2), CA9, GD3, HMW-MAA, CD171, G250/CAIX, HLA-AI MAGEA1, HLA-A2, PSCA, folate receptor-a, CD44v6, CD44v7/8, avb6 integrin, 8H9, NCAM, VEGF receptor, 5T4, Fetal AchR, NKG2D ligand, CD44v6, dual antigen, cancer-testis antigen, mesothelin, murine CMV, mucin 1 (MUC1), MUC16, PSCA, NKG2D, NY-ESO-1, MART-1, gp100, G Protein-coupled receptor 5D (GPCR5D), ROR1, TAG72, VEGF-R2, carcinoembryonic antigen (CEA), prostate-specific antigen, PSMA, Her2/neu, estrogen receptor, progesterone receptor, ephrin B2, CD123, c-Met, GD-2, O-acetylated GD2 (OGD2), CE7, Wilms tumor 1 (WT-1), cyclins, cyclin A2, CCL-1, CD138, pathogen-specific antigens and antigens associated with common labels;
Preferably, the CAR targeting BCMA comprises an antibody capable of specifically recognizing BCMA;
Preferably, the antibody specifically recognizing BCMA comprises HCDR1 as shown by SEQ ID NO: 16, HCDR2 as shown by SEQ ID NO: 17, HCDR3 as shown by SEQ ID NO: 18, LCDR1 as shown by SEQ ID NO: 19, LCDR2 as shown by SEQ ID NO: 20, LCDR3 as shown by SEQ ID NO: 21;
Preferably, the antibody specifically recognizing BCMA or a functional fragment thereof comprises the heavy chain variable region as shown by SEQ ID NO:22 and the light chain variable region as shown by SEQ ID NO:23.
In some specific embodiments, it further comprises genetically engineering the cell to reduce MHC expression, activity and/or signaling in the cell; preferably, the MHC is an MHC class I molecule; preferably, the MHC I is HLA; more preferably, the HLA is HLA-I; preferably, the HLA-I is one or more selected from the group consisting of: HLA-A, HLA-B, HLA-C, and B2M; Preferably, HLA-I comprise HLA-A and B2M;
Preferably, reducing expression comprises:
   disrupting or inhibiting a gene encoding MHC and/or disrupting or inhibiting a gene in the T cell, resulting in reduced expression of MHC;
   disrupting or inhibiting a gene encoding a protein that regulates expression or activity of MHC and/or disrupting or inhibiting a gene in the T cell, resulting in reduced expression of the protein that regulates expression or activity of MHC; and/or
   disrupting or inhibiting a gene encoding a protein involved in MHC-dependent signaling and/or disrupting or inhibiting a gene in the T cell, resulting in reduced expression of the protein involved in MHC-dependent signaling;
   Preferably, including introducing a deletion, mutation or insertion into the gene;
   Preferably, the gene encodes MHC;
   Preferably, the gene is disrupted or inhibited by introducing into the T cell an endonuclease targeting the gene under conditions allowing the gene to be disrupted or inhibited;
   Preferably, the endonuclease is selected from the group consisting of: TAL nuclease, meganuclease, zinc finger nuclease, Cas9 and Argonaute;
   Preferably, disruption or inhibition is achieved by introducing into the T cell an inhibitory nucleic acid targeting the gene under conditions that result in suppression of the gene;
   Preferably, the inhibitory nucleic acid molecule comprises a sequence complementary to the gene encoding MHC;
   Preferably, the inhibitory nucleic acid comprises an RNA interfering agent;
   Preferably, the nucleic acid is siRNA, shRNA or miRNA;
   Preferably, the reduction in expression is permanent, transient or inducible;
   Preferably, compared with the expression in a non-genetically engineered T cell, MHC expression, activity and/or signaling is reduced by greater than or greater than about 50%, 60%, 70%, 80%, 90% or 95%;
   Preferably, the MHC expression level is not detectable by immunoblotting and/or flow cytometry.

In some specific embodiments, the cell is genetically engineered to express an antibody recognizing NKG2A or a functional fragment thereof, and/or express a chimeric antigen receptor (CAR), a modified T cell (antigen) receptor (TCR), a T cell fusion protein (TFP), a T cell antigen coupler (TAC), aTCR-T, or a combination thereof specifically binding to NKG2A;
Preferably, the CAR comprises:
   (i) an antibody specifically binding to NKG2A or a functional fragment thereof, the transmembrane region of CD28 or CD8, the co-stimulatory signal domain of CD28 and CD3ζ; and/or
   (ii) an antibody specifically binding to NKG2A or a functional fragment thereof, the transmembrane region of CD28 or CD8, the co-stimulatory signal domain of CD137 and CD3ζ; and/or
   (iii) an antibody specifically binding to NKG2A or a functional fragment thereof, the transmembrane region of CD28 or CD8, the costimulatory signal domain of CD28, the costimulatory signal domain of CD137 and CD3ζ;
Preferably, the antibody specifically binding to NKG2A comprises HCDR1 as shown by SEQ ID NO: 3, HCDR2 as shown by SEQ ID NO: 4, HCDR3 as shown by SEQ ID NO: 5, LCDR1 as shown by SEQ ID NO: 6, LCDR2 as shown by SEQ ID NO: 7, LCDR3 as shown by SEQ ID NO: 8;
Preferably, the antibody recognizing NKG2A comprises the heavy chain variable region as shown by SEQ ID NO:1 or the light chain variable region as shown by SEQ ID NO:2.

In some specific embodiments, the cell is an autologous T cell derived from a human; preferably, the cell is a primary T cell.

In some specific embodiments, the cell is derived from an allogeneic T cell; preferably, the cell is derived from a human T cell; preferably, the cell is a primary T cell.

The eleventh aspect of the present application provides an engineered T cell produced by the method described in the tenth aspect of the present application.
The twelfth aspect of the present application provides a composition comprising an effective amount of the engineered T cells as described in the eighth, ninth, and eleventh aspects of the present application;
Preferably, a pharmaceutically acceptable carrier is also comprised;
Preferably, the carrier is saline solution, dextrose solution or 5% human serum albumin;
Preferably, a cryoprotectant is also comprised.

The thirteenth aspect of the present application provides a kit, comprising the engineered T cell as described in the eighth, ninth, and eleventh aspects of the present application or the composition as described in the twelfth aspect of the present application and additional agents used for treating diseases.

The fourteenth aspect of the present application provides a method for treating diseases, which includes administering the engineered T cell as described in the eighth, ninth, and eleventh aspects of the present application or the composition as described in the twelfth aspect of the present application or the kit as described in the thirteenth aspect of the present application;
Preferably, before administering the engineered T cell, the engineered T cell is produced by the method according to the tenth aspect of the present application;
Preferably, it further comprises administering an additional agent;
Preferably, the disorder is selected from inflammatory disorders, infections and tumors;
Preferably, the infection is a viral or bacterial infection;
Preferably, wherein the tumor comprises leukemia (such as acute leukemia, acute lymphoblastic leukemia, acute myeloid leukemia, acute myelogenous leukemia, acute promyelocytic leukemia, acute myelomonocytic leukemia, acute monocytic leukemia, acute leukemia, chronic leukemia, chronic myelogenous leukemia, chronic lymphocytic leukemia, polycythemia vera), lymphoma (Hodgkin's disease, non-Hodgkin's disease), primary macroglobulinemia, heavy chain disease, solid tumors such as sarcomas and cancers (e.g., fibrosarcoma, myxosarcoma, liposarcoma, chondrosarcoma, osteosarcoma, chordoma, endothelial sarcoma, lymphangiosarcoma, angiosarcoma, lymphangioendotheliosarcoma, synovial vioma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary gland carcinoma, bronchial carcinoma, medullary carcinoma, renal cell carcinoma, liver carcinoma, Nile duct carcinoma, choriocarcinoma, seminoma, embryonal carcinoma, Wilms tumor, cervical cancer, uterine cancer, testicular cancer, lung cancer, small cell lung cancer, bladder cancer, epithelial cancer, glioma, astrocytoma, medulloblastoma, craniopharyngioma, ependymoma, pineal tumor, hemangioblastoma, acoustic neuroma, oligodendroglioma, Schwannoma, meningioma, melanoma, neuroblastoma, retinoblastoma), esophageal cancer, gallbladder cancer, kidney cancer, multiple myeloma; preferably, the "tumor" includes but is not limited to: pancreas cancer, liver cancer, lung cancer, gastric cancer, esophageal cancer, head and neck squamous cell carcinoma, prostate cancer, colon cancer, breast cancer, lymphoma, gallbladder cancer, kidney cancer, leukemia, multiple myeloma, ovarian cancer, cervical cancer and glioma, and any combination thereof;
Preferably, the subject is a human;
Preferably, wherein the engineered T cell is autologous or allogeneic to the subject.

### BRIEF DESCRIPTION OF THE DRAWINGS

Figure 1: Schematic diagram of BCMA-NKG2A CAR plasmid;
Figure 2: Schematic diagram of BCMA CAR plasmid;
Figure 3: Detection results of CAR positive rate in BCMA CAR-T cells and BCMA-NKG2A CAR-T cells;
Figure 4: B2M and TCR knockout results in UCAR-T cells;
Figure 5: The detection of NKG2A expression after UCAR-T cells exposed to tumor antigens *in vivo* shows that: co-incubation of UCAR-T and tumor cells (target cells) in mice can significantly induce the expression of endogenous NKG2A in UCAR-T cells;
Figure 6: The detection of NKG2A knockout in UCAR-T cells shows that: UCAR-T cells can significantly increase the expression of endogenous NKG2A after being repeatedly stimulated by tumor cells (target cells) *in vitro;* the expression of endogenous NKG2A in UCAR-T cells with endogenous NKG2A knockout after repeated stimulation with tumor cells (target cells) is significantly inhibited, that also verifies the effective knocked out of endogenous NKG2A;
Figure 7: The tumor cell killing results of UCAR-T cells *in vitro* show that: both BCMA UCAR-T-NKG2A KO and BCMA-NKG2A-UCAR-T-NKG2A KO can effectively kill RPMI-8226 cells, and the killing ability is comparable to that of BCMA UCAR-T and BCMA -NKG2A UCAR-T, indicating that BCMA UCAR-T-NKG2A KO and BCMA-NKG2A UCAR-T-NKG2A KO can effectively kill myeloma cells;
Figure 8: The tumor cell killing results of UCAR-T cells *in vivo* show that: compared with the BCMA UCAR-T group, the BCMA UCAR-T-NKG2A KO group can significantly inhibit the growth of tumor volume; compared with the BCMA-NKG2A UCAR-T group, BCMA-NKG2A KO group can also significantly inhibit the growth of tumor volume;
Figure 9: Schematic diagram of BCMA-loop-NKG2A CAR and NKG2A-loop-BCMA CAR plasmids;

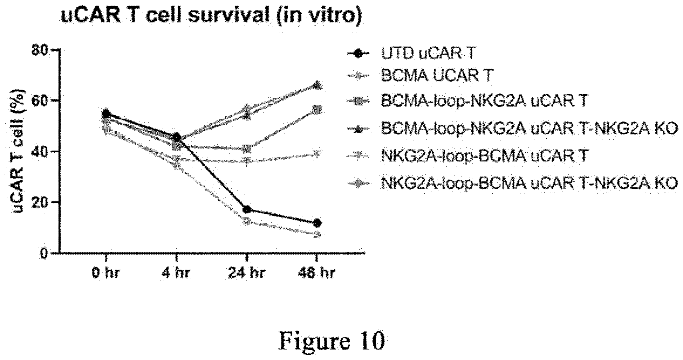

Figure 10: Detection of the resistance function of NKG2A knockout tandem UCAR-T cells to NK cells *in vitro* shows that: when co-incubated with activated and expanded NK cells, compared with UTD UCAR-T and BCMA UCAR-T cells, the proportion of BCMA-loop-NKG2A uCAR-T and NKG2A-loop-BCMA UCAR T cells is significantly increased at 24 hours and 48 hours; compared with BCMA-loop-NKG2A uCAR-T, the proportion of the BCMA-loop-NKG2A UCAR-T-NKG2A KO cells is significantly increased at 24 hours and 48 hours; compared with NKG2A-loop-BCMA UCAR T cells, the proportion of endogenous NKG2A knockout NKG2A-loop-BCMA UCAR T-NKG2A KO cells is significantly increased at 24 hours and 48 hours;
Figure 11: Detection of anti-tumor function of NKG2A knockout tandem UCAR-T cells *in vivo* shows: compared with NKG2A-loop-BCMA-UCAR-T cells, the growth rate of tumor volume in the NKG2A-loop-BCMA-UCAR-T-NKG2A KO cell group is significantly inhibited;
Figure 12: The resistance function of NKG2A knockout tandem UCAR-T to NK cells in immunodeficient mice shows: on D4 and D7, the number of CD4-positive (Figure 12A) and CD8-positive (Figure 12B) T cells in BCMA-loop-NKG2A UCAR-T-NKG2A KO cells is significantly bigger than that in the BCMA uCAR-T cell group;
Figure 13: The resistance function of NKG2A knockout tandem UCAR-T to NK cells in humanized recombinant mice shows: compared with UTD UCAR-T and BCMA uCAR-T cells, the expansion and survival of BCMA-loop-NKG2A UCAR-T-NKG2A KO cells *in vivo* are significantly improved (Figure 13A), and BCMA-loop-NKG2A UCAR-T-NKG2A KO cells significantly inhibit the number of NK cells (Figure 13B);
Figure 14: Detection of *in vitro* anti-tumor activity and anti NK activity of NKG2A knockout tandem UCAR-T in the presence of NK cells shows that: BCMA-loop-NKG2A UCAR-T-NKG2A KO cells and BCMA uCAR-T cells can both significantly kill tumor cells; the number of BCMA-loop-NKG2A UCAR-T-NKG2A KO cells is significantly bigger than that of BCMA uCAR-T cells, and the number of NK cells in the BCMA-loop-NKG2A UCAR-T-NKG2A KO cell group is smaller than that of the BCMA uCAR-T group;
Figure 15: The *in vitro* cytokine secretion of NKG2A knockout tandem UCAR-T cells shows that: BCMA-loop-NKG2A UCAR-T-NKG2A KO cells have similar secretion levels of INF-γ, IL2, and TNF cytokines to BCMA uCAR-T (Fig. 15A); BCMA-loop-NKG2A UCAR-T-NKG2A KO cells have similar secretion level of IL4, IL6 and IL10 cytokines to BCMA uCAR-T (Figure 15B);
Figure 16: The *in vivo* anti-tumor function test of different forms of tandem UCAR-T cells with NKG2A knockout shows that: compared with BCMA UCAR-T, NKG2A knockout NKG2A-loop-BCMA UCAR T-NKG2A KO and BCMA-loop-NKG2A UCAR-T-NKG2A KO cells show better antitumor activity.

### SPECIFIC EMBODIMENTS

Unless defined otherwise, all technical and scientific terms used herein have the same meaning as commonly understood by one of skilled in the fields of gene therapy, biochemistry, genetics, and molecular biology. Methods and/or materials similar or equivalent to those described herein can be used in the practice of the present application. All publications, patents, and other references mentioned herein are hereby incorporated by reference in their entirety. In case of conflict, the present specification, including definitions, will control. In addition, the materials, methods, and examples are illustrative only and not intended to be limiting unless otherwise specified.

Unless otherwise indicated, the practice of the present application will employ conventional techniques of cell biology, cell culture, molecular biology, transgenic biology, microbiology, recombinant DNA and immunology, which are all within the skill of the art. These techniques have been fully disclosed in the prior art, for example, Current Protocols in Molecular Biology (FrederickM. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M.J. Gaited., 1984); Mullis et al. U.S. Pat. No. 4,683,195; Nucleic Acid Hybridization (B.D. Harries & S.J. Higginseds. 1984); Transcription And Translation (B.D.Hames&S.J.Higginseds.1984); Culture Of Animal Cells (R.I. Freshney, Alan R.Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York); Gene Transfer Vectors For Mammalian Cells (J.H. Miller and M.P. Caloseds., 1987, Cold Spring Harbor Laboratory); Immunochemical Methods In Cell And Molecular Biology (Mayer and Walker, eds., Academic Press, London, 1987); Hand book Of Experimental Immunology, Volumes I-IV (D.M. Weir and C.C. Blackwell, eds., 1986); and Manipulating the Mouse Embryo (Cold Spring Harbor Laboratory Press, Cold Spring Harbor, N.Y, 1986).

This disclosure of claimed subject matter in range format should be understood that the description in range format is merely for convenience and brevity, and should not be construed as an inflexible limitation on the scope of the claimed subject matter. Accordingly, the description of a range should be considered to have specifically disclosed all the possible subranges as well as individual values within that range. For example, where a range is provided, every intervening value between the upper and lower limit of that range, as well as both upper and lower limits, are encompassed within the claimed subject matter. Smaller ranges between the upper and lower limits of that range may independently include the upper and lower limits of these smaller ranges and are also within the scope of the claimed subject matter, unless the upper and lower limits of the range are explicitly excluded. Where the stated range includes one or two limits, the claimed subject matter also includes ranges excluding either or both of those limits. This applies regardless of the width of the range.

As used herein, the term "about" refers to the usual error range for each value readily known to those skilled in the art. Reference herein to "about" a value or parameter includes embodiments referring to the value or parameter itself. For example, description of "about X" includes description of "X". Herein, "about" may be an acceptable error range in the technical field. For example, it can refer to the value or parameter within ± 10% of the "approximate" value or parameter, e.g., about 5 uM can include any number between 4.5 uM and 5.5 uM.

Unless otherwise indicated, any concentration range, percentage range, ratio range or integer range described herein should be understood to include any integer within the stated range, and, where appropriate, the numerical value of fractions thereof (e.g., one-tenth and one hundredth of an integer).

### TERMS

The term "cell" refers to a cell of human or non-human animal origin. In one embodiment, the engineered cells may also refer to endogenous NKG2A knockout cells. In one embodiment, the engineered cells may also refer to T cells whose endogenous NKG2A is knocked out and which express a CAR targeting immune cells and/or tumor cells.

The term "host" or "subject" refers to a recipient receiving a transplant, and in some specific embodiments, may be an individual (such as a human) receiving exogenous cells implantation. In some specific embodiments, a "subject" may be a clinical patient, a clinical trial volunteer, an experimental animal, and the like. The subject may be suspected of having a disease characterized by cell proliferation, or has a disease characterized by cell proliferation, or is diagnosed with a disease characterized by cell proliferation, or is a control subject who is confirmed not to have a disease characterized by cell proliferation. In some specific embodiments, the subject is or may be suffering from an immune disease, such as an autoimmune disease, or after receiving a transplant therapy.

The term "engineering" refers to a comprehensive science and technology that uses the principles and methods of cell biology and molecular biology to change the genetic material in cells or obtain cell products at the level of cells or organelles according to people's wishes through some engineering means. In one embodiment, the engineering refers to one or more alterations of a nucleic acid, such as a nucleic acid within the genome of an organism. In one embodiment, the engineering refers to changes, additions and/or deletions of genes. In one embodiment, the engineered cells may also refer to cells with added, deleted and/or altered genes. The term "gene disruption" refers to the artificial manipulation of genetic information in an individual organism, including but not limited to genes, RNA or proteins etc., to interfere with the transmission, regulation or expression of genetic information. Gene disruption can be modified by gene editing, such as mutation or deletion of genes by gene knockout technology; gene silencing can be achieved by inhibiting the expression of mRNA such as RNAi technology; It can also degrade proteins by expressing recombinant proteins such as PROTAC technology. In one embodiment, endogenous NKG2A is knocked out by CRISPR/Cas9 technology, or TRAC, endogenous NKG2A and B2M are knocked out.

The terms "genetic modification", "gene modification", "gene engineering" or "modified" refer to methods of modifying cells, including but not limited to change the protein expression level of genes to cause gene defects through gene editing in coding or non-coding regions or their expression regulatory regions, or through endonuclease and/or antisense RNA technology, or increase the introduction of exogenous proteins and/or complexes, small molecule inhibitors. In some specific embodiments, the modified cells are stem cells (e.g., hematopoietic stem cells (HSC) or progenitor cells, embryonic stem cells (ES), induced pluripotent stem (iPS) cells), lymphocytes (e.g., T cells), which can be obtained from a subject or a donor. Cells can be modified to express exogenous constructs, such as pre-TCRα protein, chimeric antigen receptor (CAR) or T cell receptor (TCR), which can be integrated into the cellular genome.

The term "pluripotent stem cell" has the potential to differentiate into any of the three germ layers: endoderm (e.g., gastric junction, gastrointestinal tract, lung, etc.), mesoderm (e.g., muscle, bone, blood, genitourinary tissue, etc.) or ectoderm (such as epidermal tissue and nerve system tissue). As used herein, the term "pluripotent stem cells" also encompasses "induced pluripotent stem cells" or "iPSCs", which are a type of pluripotent stem cells derived from non-pluripotent cells. In one embodiment, the pluripotent stem cells are derived from cells transformed by reprogramming somatic cells with characteristics of pluripotent stem cells. Such "iPS" or "iPSC" cells can be generated by inducing the expression of certain regulatory genes or by exogenously applying certain proteins.

"Pluripotent stem cell characteristic" refers to a cell characteristic that distinguishes a pluripotent stem cell from other cells. For example, human pluripotent stem cells express at least several of the following markers: SSEA-3, SSEA-4, TRA-1-60, TRA-1-81, TRA-2-49/6E, ALP, Sox2, E-cadherin protein, UTF-1, Oct4, Rex1 and Nanog. Having cell morphology associated with pluripotent stem cells is also characteristic of pluripotent stem cells.

The "T cells" described herein can be PBMC, bone marrow, lymph node tissue, umbilical cord blood, thymus tissue, and natural T cells obtained from infected sites, ascites, pleural effusion, spleen tissue, and tumor tissue, and can also be a cell population with specific phenotypic characteristics obtained by sorting etc., or a mixed cell population with different phenotypic characteristics, such as "T cells" can be cells containing at least one T cell subset: stem cell-like memory T cells (Tscm cells), central memory T cells (Tcm), effector T cells (Tef, Teff), regulatory T cells (tregs) and/or effector memory T cells (Tern). In some cases, "T cells" may be a specific subtype of T cells, such as γ δ T cells. In some cases, T cells can be obtained from blood collected from an individual using any number of techniques known to those of skilled in the art, such as FicollTM isolation and/or apheresis. In one embodiment, the T cells are derived from induced pluripotent stem cells. In one embodiment, the cells from the circulating blood of the individual are obtained by apheresis. Apheresis products usually contain lymphocytes, including T cells, monocytes, granulocytes, B cells, other nucleated white blood cells, red blood cells, and platelets. In one embodiment, cells collected by apheresis can be washed to remove plasma molecules and placed in a suitable buffer or culture medium for subsequent processing steps. The T cells may be derived from healthy donors, or from individuals diagnosed with cancer. The T cells can be autologous T cells or allogeneic T cells. The T cells can be primary T cells.

The "T cells" described herein can also be T cells carrying exogenous proteins recognizing target antigens, such as CAR T cells, TCR-T cells, T cell antigen coupler (TAC), T cell fusion proteins, etc.

NKG2A (OMIM 161555, the full disclosure of which is incorporated herein by reference) is a member of the NKG2 transcriptome that, together with CD94, forms a heterodimeric inhibitory receptor CD94/NKG2A found on the surface of subpopulation of NK cells, α/β T cells, γ/δ T cells, and NKT cells. As used herein, "NKG2A" refers to the NKG2A gene or encoded protein and any variants, derivatives or isoforms. Human NKG2A comprises 233 amino acids in three domains, a cytoplasmic domain comprising residues 1-70, a transmembrane region comprising residues 71-93, and an extracellular domain comprising residues 94-233, the human NKG2A has the following sequence:

MDNQGVIYSDLNLPPNPKRQQRKPKGNKSSILATEQEITYAELNLQKASQDFQ
GNDKTYHCKDLPSAPEKLIVGILGIICLILMASVVTIVVIPSTLIQRHNNSSLNTR
TQKARHCGHCPEEWITYSNSCYYIGKERRTWEESLLACTSKNSSLLSIDNEEE
MKFLSIISPSSWIGVFRNSSHHPWVTMNGLAFKHEIKDSDNAELNCAVLQVNR
LKSAQCGSSIIYHCKHKL (SEQ ID NO: 59).

The term "MHC" is histocompatibility complex, which is a general term for all gene groups encoding biocompatible complex antigens. MHC antigens are expressed in the tissues of all higher vertebrates, they are called HLA antigens in human cells and plays an important role in the transplant response, with rejection mediated by T cells that respond to histocompatibility antigens on the surface of the implanted tissue. MHC proteins play a crucial role in T cell stimulation, antigen presenting cells (usually dendritic cells) display peptides that are degradation products of exogenous proteins and belonging to MHC on the cell surface, in the presence of co-stimulatory signals, T cells are activated and act on target cells that also display the same peptide/MHC complex. For example, stimulated T helper cells would target macrophages displaying antigens bound to their MHC, or cytotoxic T cells (CTLs) would act on virus-infected cells displaying exogenous viral peptides. MHC antigens are divided into NHC class I antigens and MHC class II antigens.

NKG2A/CD94 is a heterodimer, which is the receptor of non-classic HLA-I molecule HLA-E, distributed on the surface of most NK cells, and plays an inhibitory role. After binding to HLA-E, NKG2A transmits inhibitory signals to inhibit the cytotoxic activity of these immune cells, thereby weakening the clearance of viruses (such as polyoma virus or human cytomegalovirus) by T cells, or inhibiting killing of immune cells to tumors cell. HLA-E is a non-classical HLA-I molecule. Its mRNA can be detected in all nucleated cells, but its protein expression is limited to a few organs and cells, and the expression intensity is weak. Different from the classic HLA-I molecules, the polymorphism of HLA-E is relatively weak, with only 26 alleles and only 8 kinds of encoded proteins. In China, only two types are more popular: HLA-E *01:01 and HLA-E*01:03, the distribution ratio is about 1:1, indicating that the HLA-E molecular sequence is relatively conservative. And the antigenic peptide presented by it is the leader sequence of HLA-I (A, B, C and G, etc.) class molecules, which is relatively highly conserved, and the effect of inhibiting NK cell killing is quite significant.

The term "Human leukocyte antigen" (HLA) is the coding gene of human major histocompatibility complex, which is located on chromosome 6 (6p21.31), and is closely related to the function of human immune system. HLA includes class I, class II and class III gene portions. The antigens expressed by HLA class I and class II genes are located on the cell membrane and are MHC-I (encoded by HLA-A, HLA-B, HLA-C sites) and MHC-II (encoded by HLA-D region), HLA Class I is distributed on the surface of almost all cells in the body and is a heterodimer composed of heavy chain (α chain) and β2 microglobulin (B2M); HLA Class II is mainly a glycoprotein located on the surface of macrophages and B lymphocytes.

The term "B2M" is for beta-2 microglobulin, also known as B2M, it is the light chain of MHC class I molecule. In humans, B2M is encoded by the b2m gene located on chromosome 15, and is opposite to other MHC genes located as a gene cluster on chromosome 6. Studies have shown that when the B2M gene is mutated, hematopoietic grafts from mice lacking normal cell surface MHC I expression are rejected by NK cells in normal mice, indicating that defective expression of MHC I molecules makes cells vulnerable to host immune system repulsion (Bix et al. 1991).

The term "exogenous" refers to a function of a nucleic acid molecule or polypeptide, cell, tissue, etc. that has not been expressed endogenously in the organism itself, or the expression level is insufficient to achieve overexpression.

The term "endogenous" means that a nucleic acid molecule or polypeptide etc. is derived from the organism itself.

In one embodiment, the exogenous protein may be an exogenously introduced protein that recognizes a target antigen, such as an exogenous receptor.

The term "exogenous receptor" refers to a fusion molecule formed by linking DNA fragments from different sources or corresponding cDNAs of proteins by genetic recombination technology, including extracellular domains, transmembrane domains and intracellular domains. Chimeric receptors include, but are not limited to: Chimeric Antigen Receptor (CAR), Chimeric T Cell Receptor (TCR), T Cell Antigen Coupler (TAC).

The term "chimeric antigen receptor" (CAR) refers to two or more polypeptides; when immune effector cells express the CAR, then the immune cells can specifically target the target cells (the present application includes tumor cells, NK cells), and generate intracellular signals. In certain embodiments, a CAR comprises at least one extracellular antigen binding domain, a transmembrane domain, and an intracellular signaling domain (also referred to as a "cytoplasmic signaling domain"). The intracellular signaling domain includes a functional signaling domain of a stimulatory molecule and/or a co-stimulatory molecule, in one aspect, the stimulatory molecule is a zeta chain bound to a T cell receptor complex; in one aspect, a cytoplasmic signaling domain further includes functional signaling domains of one or more co-stimulatory molecules, such as 4-1BB (i.e., CD137), CD27 and/or CD28. In certain embodiments, groups of polypeptides are contiguous with each other. Exemplarily, the CAR targeting NKG2A comprises the sequence of SEQ ID NO:9. The CAR targeting BCMA comprises the sequence as shown by SEQ ID NO:25. The CAR targeting both NKG2A and BCMA includes the antigen-binding domain as shown by SEQ ID NO:30 and/or 32; or includes the CAR as shown by SEQ ID NO:55 and/or 57. In a specific embodiment, the engineered T cell targeting both NKG2A and BCMA comprises the amino acid sequence as shown by SEQ ID NO:26 or the nucleotide sequence as shown by SEQ ID NO:27. In a specific embodiment, the engineered T cell targeting both NKG2A and BCMA comprises the amino acid sequence as shown by SEQ ID NO:55 and/or SEQ ID NO:57; or comprises the nucleotide sequence as shown by SEQ ID NO:56 and/or SEQ ID NO: 58. The term "T cell receptor (TCR)" mediates T cell recognition of specific major histocompatibility complex (MHC)-restricted peptide antigens, including classical TCR receptors and optimized TCR receptors. The classic TCR receptor consists of two peptide chains α and β, and each peptide chain can be divided into a variable region (V region), a constant region (C region), a transmembrane region and a cytoplasmic region, etc., and its antigen specificity exists in the V region, and the V region (Vα, Vβ) has three hypervariable regions CDR1, CDR2, and CDR3. In one aspect, T cells expressing classical TCR can induce specific response of TCR of T cells to target antigens by stimulating T cells such as with antigens.

The term "chimeric T cell receptor" includes recombinant polypeptides derived from the various polypeptides that make up the TCR, which are capable of binding to surface antigens on target cells, and interacting with other polypeptides of the complete TCR complex, usually colocalized at the surface of T cells. A chimeric T cell receptor consists of a TCR subunit and an antigen-binding domain composed of a human or humanized antibody domain, wherein the TCR subunit includes at least part of the TCR extracellular domain, transmembrane domain, and TCR intracellular domain; the TCR subunit is operatively linked to the antibody domain, wherein the extracellular, transmembrane, and intracellular signal domains of the TCR subunit are derived from CD3ε, CD3y, CD3z, the α chain of TCR, or the β chain of TCR, and the chimeric T cell receptor is integrated into the TCR/CD3 complex expressed on T cells.

The main function of the two polymorphic subunits (TCRα β or TCRγ δ) in the TCR/CD3 complex is to recognize antigens binding to MHC molecules, and the cytoplasmic region is very short; the main function of the CD3 molecule is to participate in the assembly, stability and signal transduction of the TCR/CD3 complex. The intracytoplasmic part of the CD3 molecular subunit comprises a common sequence, i.e. D/EX2YX2L/IX8YX2L/I, which comprises two YXXL/I structures. Because its sequence is closely related to lymphocyte activation and signal transduction after lymphocyte antigen recognition, this sequence is called antigen recognition activation motifs (antigen recognition activation motifs, ARAMS), which comprises two YXXL/I structures. Because its sequence is closely related to lymphocyte activation and signal transduction after lymphocyte antigen recognition, this sequence is called antigen recognition activation motifs (antigen recognition activation motifs, ARAMS). Among them, CD3y, ε, and δ each comprises one ARAM, the η chain comprises two, and the ζ chain comprises three.

The term "T cell antigen coupler (TAC)" includes three functional domains: 1. Antigen binding domain, including single-chain antibody, designed ankyrin repeat protein (DARPin) or other targeting groups; 2, the extracellular region domain, the single-chain antibody binding to CD3, so that the TAC receptor and the TCR receptor are close; 3, the transmembrane region and the intracellular region of the CD4 co-receptor, wherein, the intracellular domain links protein kinase LCK, catalyzes the phosphorylation of immunoreceptor tyrosine-activating motifs (ITAMs) of the TCR complex as the initial step of T cell activation.

The term "signaling domain" also known as "cytoplasmic signaling domain" refers to a functional part of a protein that functions by conveying information within a cell used to modulate the activity of cells via defined signal transduction pathways by producing secondary messengers or by acting as effectors in response to such messengers. The intracellular signaling domain may include the entire intracellular portion of the protein, or the entire native intracellular signaling domain, or a functional fragment or derivative thereof. Exemplary, the signaling domain of a CAR targeting BCMA, targeting NKG2A, and/or targeting both NKG2A and BCMA includes CD3ζ. In a specific embodiment, CD3ζ is a human CD3ζ molecule, comprising the sequence as shown by SEQ ID NO:51 or SEQ ID NO:52. The term "co-stimulatory molecule" is a cell surface molecule and its ligand that provides a co-stimulatory signal for full activation of T (or B) cells, and when combined with a cell-stimulatory signaling molecule, such as TCR/CD3, the combination results in T cell proliferation and/or signals of up- or down-regulation of key molecules and to mediate co-stimulatory responses of T cells. Costimulatory molecules include but are not limited to MHC class I molecules, BTLA and Toll ligand receptors, and OX40, CD2, CD27, CD28, CDS, ICAM-1, LFA-1 (CD11a/CD18) and 4-1BB (CD137). Exemplary, the signaling domain of a CAR targeting BCMA, targeting NKG2A, and/or targeting both NKG2A and BCMA includes 4-BB. In a specific embodiment, 4-1BB is a human 4-BB molecule, comprising the sequence as shown by SEQ ID NO:49 or SEQ ID NO:50.

The term "stimulation" or "activation" refers to the process by which a cell transitions from a quiescent state to an active state. The process can include responses to antigenic, phenotypic or genetic changes in migration and/or functional activity status. For example, the term "activation" may refer to the process by which T cells are gradually activated. The activation process is jointly regulated by the first stimulatory signal and co-stimulatory signal. The activation of T cells is a dynamic process, and both of its duration and degree of activation are affected by external stimuli. "T cell activation" refers to the state of a T cell that is stimulated to induce detectable cell proliferation, cytokine production, and/or detectable effector function.

Using CD3/CD28 magnetic beads, both of in vitro antigen stimulation or in vivo antigen stimulation will affect the degree and duration of T cell activation. In one embodiment, the engineered T cells are activated through co-incubated with tumor cells containing a specific target antigen or after virus infection.

The term "Genome editing" or "Gene editing" refers to the genetic engineering technology that uses site-specific nucleases to insert, knock out, modify or replace DNA at specific positions in the genome of an organism, which will change the DNA sequence. This technique is sometimes called "gene editing" or "genome engineering." Gene editing can be used to achieve precise and efficient gene knockout or gene knockin.

Nuclease-directed genome targeted modification technology usually consists of a DNA recognition domain and a non-specific endonuclease domain. The DNA recognition domain recognizes the target site and localizes the nuclease to the genome region that needs to be edited, then the DNA double-strand is cleaved by non-specific endonuclease, causing the self-repair mechanism of DNA breakage, thereby triggering the mutation of the gene sequence and promoting the occurrence of homologous recombination. The endonuclease may be Meganuclease, zinc finger nuclease, CRISPR/Cas9 nuclease, MBBBD-nuclease or TALEN-nuclease. In a preferred embodiment, the endonuclease is CRISPR/Cas9 nuclease, TALEN-nuclease. Gene knockout technologies using nucleases include CRISPR/Cas9 technology, ZFN technology, TALE technology and TALE-CRISPR/Cas9 technology. In some specific embodiments, the gene editing technology is selected from single Base Editor technology, Prime Editor technology and homing endonuclease (Meganuclease) technology.

The term "artificial zinc finger nuclease (ZFN)" technology is the first generation of nuclease site-directed modification technology, using zinc finger motifs that can specifically recognize triplet DNA fragments instead of bases as the basic unit of recognizing a specific DNA sequence. The most classic zinc finger nuclease is to fuse a non-specific endonuclease FokI with a domain containing zinc fingers, and obviously, its purpose is to cut specific sequences.

The term "transcription activator-like effector (TALE)" has DNA binding specificity, has a module that can specifically recognize bases, and is simple and convenient to operate. The TALE-DNA binding domain is composed of tandem repeating units, most of which contain 34 amino acids, and the 12th and 13th amino acids of the unit are designed as variable regions (repeat variable residues, RVD). The RVD of TALE recognizes 4 bases of the DNA sequence with high specificity, and the 13th amino acid directly binds to the base of DNA specifically. According to the DNA sequence, a specific TALEDN recognition binding domain can be constructed at any site, which can be widely used in gene sequence mutation modification and gene targeting. Set the DNA target sequence, assemble the TALE-DNA binding domain, fuse the non-specific DNA cleavage domain of Fok I endonuclease, and assemble into TALE nucleases (tanscription activator-like effector nucleases, TALENs). TALENs target DNA to generate DNA double-strand breaks (DSBs).

CRISPER/Cas9 is the third generation gene editing technology. "CRISPR system" collectively refers to transcripts and other elements involved in the expression of or directing the activity of CRISPR-associated ("Cas") genes, including sequences encoding Cas genes, tracr (transactivating CRISPR) sequences (e.g., tracrRNA or active moieties tracrRNA), tracr pairing sequences (covering "direct repeats" and partial direct repeats for tracrRNA processing in the context of endogenous CRISPR systems), guide sequences (also known as "spacers" in the context of endogenous CRISPR systems "), or other sequences and transcripts from CRISPR loci. CRISPR systems are characterized by elements that facilitate the formation of the CRISPR complex (also referred to as the prospacer in the context of endogenous CRISPR systems) at the site of the target sequence. In the context of CRISPR complex formation, "target sequence" refers to a sequence to which a guide sequence is designed to be complementary, wherein hybridization between the target sequence and the guide sequence facilitates the formation of the CRISPR complex. Full complementarity is not required, provided that there is sufficient complementarity to cause hybridization and promote the formation of a CRISPR complex. After the CRISPR complex is formed, under the action of the cas9 enzyme, it can cut specific sites in the genome and introduce gene mutations; it can also regulate gene expression, such as activation or inhibition. A target sequence can comprise any polynucleotide, such as DNA or RNA polynucleotides. In some specific embodiments, the target sequence is located in the nucleus or cytoplasm of the cell.

The term "gene silencing" refers to the phenomenon of non-expression or low expression of genes due to various reasons. Gene silencing can be gene silencing at the transcriptional level due to DNA methylation, heterochromatinization, and position effects, or it can be post-transcriptional gene silencing, that is, gene inactivation caused by specific inhibition of target RNA at the post-transcriptional level of genes, including translational inhibition mediated by antisense RNA, RNA interference, and microRNA.

"TCR silencing" refers to no or low expression of endogenous TCR.

"MHC silencing" refers to no or low expression of endogenous MHC.

The term "CRISPR (Clustered regularly interspaced short palindromic repeats)" refers to regularly clustered interspaced short palindromic repeats.

The term "Cas9 (CRISPR-associated nuclease)" is a CRISPR-associated nuclease, which is an RNA-guided technology that uses Cas9 nuclease to edit targeted genes.

In general, a guide sequence (gRNA) is any polynucleotide sequence that is sufficiently complementary to a target polynucleotide sequence to hybridize to the target sequence and direct sequence-specific binding of the CRISPR complex to the target sequence. gRNA is used to guide, bind or recognize the Cas enzyme. In some specific embodiments, the degree of complementarity between the guide sequence and its corresponding target sequence is about or more than about 50%, 60%, 75%, 80%, 85%, 90%, 95%, 97.5%, 99%, or more when optimally aligned using a suitable alignment algorithm. Optimal alignment can be determined using any suitable algorithm for aligning sequences, non-limiting examples of which include the Smith-Waterman algorithm, Needleman-Wunsch algorithm, Algorithms based on Burrows-Wheeler Transformation (such as Burrows-Wheeler Aligner), ClustalW, Clustal X, BLAT, Novoalign (Novocraft Technology Company), ELAND (Illumina, San Diego, CA), SOAP (available at soap.genomics.org.cn), and Maq (available at maq.sourceforge.net). In some specific embodiments, the CRISPR enzyme is part of a fusion protein comprising one or more heterologous protein domains (e.g., about or more than about 1, 2, 3, 4, 5, 6, 7, 8, 9, 10 or more domains in addition to the CRISPR enzyme). A CRISPR enzyme fusion protein may comprise any other protein, and optionally a linker sequence between any two domains. Examples of protein domains that can be fused to CRISPR enzymes include, but are not limited to, epitope tags, reporter gene sequences, and one or more protein domains that have the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tag, V5 tag, FLAG tag, influenza virus hemagglutinin (HA) tag, Myc tag, VSV-G tag, and thioredoxin (Trx) tag.

The term "Cas9 enzyme" may be wild-type Cas9 or artificially modified Cas9.

The term "sgRNA" refers to a short or small gRNA.

When performing gene editing, the gRNA, tracr paired sequence, and tracr sequence can be administered alone or as a whole RNA sequence.

The combination of Cas9 protein and gRNA can cleave DNA at a specific site. The recognition sequence of CRISPR/Cas system derived from Streptococcus pyogenes is 23bp, and can target 20bp. The last three bases of NGG sequence of the recognition site are called PAM (protospacer adjacent motif) sequence.

CRISPR/Cas9 transgenes can be delivered by vectors (e.g., AAV, adenovirus, lentivirus) and/or particles and/or nanoparticles, and/or electroporation.

In one embodiment, CRISPER/Cas technology is used to knock out the exons of the corresponding coding genes of the constant regions of one or two chains of the α and β chains of NKG2A, B2M, and TCR, to make endogenous NKG2A, B2M, TCR inactive; in one embodiment, the first exon of the constant region of the endogenous TCRα chain is knocked out, and the gRNA used is selected from the sequence as shown by SEQ ID NO:53. In a specific embodiment, CRISPR/Cas9 technology is used to knocked out the endogenous NKG2A gene of the engineered T cells, and the gRNA used is selected from the sequence as shown by SEQ ID NO:12, SEQ ID NO:13, SEQ ID NO:14, SEQ ID NO:15, SEQ ID NO:60, SEQ ID NO:61, SEQ ID NO:62, SEQ ID NO:63, SEQ ID NO:64, SEQ ID NO:65, SEQ ID NO:66, SEQ ID NO: 67 and SEQ ID NO:68. CRISPR/Cas9 technology is used to knocked out the endogenous B2M gene of the engineered T cells, and the gRNA used is selected from the sequence as shown by SEQ ID NO:54.

"Inhibiting" or "repressing" the expression of B2M or TCR or NKG2A refers to reducing the expression of B2M or TCR or NKG2A in cells by at least 1%, at least 5%, at least 10%, at least 20%, at least 30%, at least 40%, at least 50%, at least 60%, at least 70%, at least 80%, at least 90%, at least 95%, at least 99%, or 100%. More specifically, "inhibiting" or "repressing" the expression of B2M or TCR or NKG2A means that the content of B2M or TCR or NKG2A in cells is reduced by at least 1%, at least 5%, at least 10%, at least 20%, at least 30%, at least 40%, at least 50%, at least 60%, at least 70%, at least 80%, at least 90%, at least 95%, at least 99%, or 100%. Specific antibodies to NKG2A, B2M, TCR can be used to determine the copy number of the corresponding gene in the cell, mRNA expression or the expression or content of protein by any suitable method known in the art, such as gene sequencing, PCR, blot hybridization, ELISA, immunohistochemistry, Western Blotting or flow cytometry.

"RNA interfering agent" as used herein is defined as any agent that interferes with or inhibits the expression of a target gene by RNA interference (RNAi). Such RNA interfering agents include, but are not limited to, nucleic acid molecules of RNA molecules homologous to a target gene or a fragment thereof, short interfering RNA (siRNA), shRNA or miRNA, and small molecules that interfere with or inhibit the expression of target genes by RNA interference (RNAi).

In one embodiment, the target gene may be a gene encoding NKG2A, or a gene encoding a protein regulating the expression or activity of NKG2A, or a gene encoding a protein involved in NKG2A-dependent signaling.

The "antibody" described herein may be a full-length antibody or an antibody fragment that retains the ability to bind to an antigen. Antibody fragment refers to any molecule comprising the antigen-binding portion (e.g., CDR) of the antibody from which the molecule is derived. Examples of antibody fragments include, but are not limited to, Fab, Fab', F(ab')2 and Fv fragments, dAbs, linear antibodies, scFv antibodies and multispecific antibodies formed from antigen binding molecules. In some specific embodiments, an antibody refers to an antibody fragment that specifically binds an antigen, including one or more complementarity determining regions (CDRs) thereof, and in a further embodiment, the antibody may be a single-chain antibody (scFv). In some specific embodiments, the antibody comprises or consists of an avimer. Exemplarily, the antibody recognizing NKG2A or the functional fragment thereof comprises HCDR1 as shown by SEQ ID NO:3, and/or HCDR2 as shown by SEQ ID NO:4, and/or HCDR3 as shown by SEQ ID NO:5, and /or LCDR1 as shown by SEQ ID NO:6, and/or LCDR2 as shown by SEQ ID NO:7, and/or LCDR3 as shown by SEQ ID NO:8. Exemplarily, the antibody recognizing NKG2A or the functional fragment thereof comprises the heavy chain variable region as shown by SEQ ID NO:1 and/or the light chain variable region as shown by SEQ ID NO:2. Exemplarily, the antibody recognizing NKG2A or the functional fragment thereof comprises the SCFV sequence as shown by SEQ ID NO:10. Exemplarily, the antibody recognizing BCMA or a functional fragment thereof comprises HCDR1 as shown by SEQ ID NO: 16, and/or HCDR2 as shown by SEQ ID NO: 17, and/or HCDR3 as shown by SEQ ID NO: 18, and/or LCDR1 as shown by SEQ ID NO:19, and/or LCDR2 as shown by SEQ ID NO:20, and/or LCDR3 as shown by SEQ ID NO:21. Exemplarily, the antibody recognizing BCMA or a functional fragment thereof comprises the heavy chain variable region as shown by SEQ ID NO:22 and/or the light chain variable region as shown by SEQ ID NO:23. Exemplarily, the antibody recognizing BCMA or a functional fragment thereof comprises the SCFV sequence as shown by SEQ ID NO:24.

The term "transfection" refers to the introduction of exogenous nucleic acid into a eukaryotic cell. Transfection can be achieved by various means known in the art, including calcium phosphate-DNA co-precipitation, DEAE-dextran-mediated transfection, polybrene-mediated transfection, electroporation, microinjection, Liposome fusion, lipofection, protoplast fusion, retroviral infection and biolistics.

The terms "nucleic acid molecule code", "coding DNA sequence" and "coding DNA" refer to the sequence or order of deoxyribonucleotides along a deoxyribonucleic acid chain. The order of these deoxyribonucleotides determines the order of amino acids along the polypeptide (protein) chain. Thus, a nucleic acid sequence encodes an amino acid sequence.

The term "individual" refers to any animal, such as a mammal or a marsupial. Subjects of the application include, but are not limited to, humans, non-human primates (such as rhesus or other types of macaques), mice, pigs, horses, donkeys, cows, sheep, rats, and poultry of any kind.

The term "peripheral blood mononuclear cell" (PBMC) refers to cells with a single nucleus in peripheral blood, including lymphocytes, monocytes and the like.

As used herein, the term "sequence" when used to refer to a nucleotide sequence may include DNA or RNA, and may be single or double stranded.

As used herein, the term "effective amount" refers to an amount that provides a therapeutic or prophylactic benefit.

The term "expression vector" as used herein refers to a vector comprising a recombinant polynucleotide, which includes an expression control sequence operably linked to a nucleotide sequence to be expressed. Expression vectors contain sufficient cis-acting elements for expression; other elements for expression may be provided by the host cell or by an in vitro expression system. Expression vectors include all those known in the art, such as plasmids, viruses (e.g., lentiviruses, retroviruses, adenoviruses, and adeno-associated viruses).

The term "vector" as used herein is a composition comprising an isolated nucleic acid and which can be used to deliver the isolated nucleic acid to the interior of a cell. Many vectors are known in the art, including but not limited to linear polynucleotides, polynucleotides associated with ionic or amphiphilic compounds, plasmids and viruses. Thus, the term "vector" includes autonomously replicating plasmids or viruses. Non-plasmid and non-viral compounds that facilitate the transfer of nucleic acids into cells may also be included, such as polylysine compounds, liposomes, and the like.

The term sequence "identity" as used herein determines percent identity by comparing two best-matched sequences over a comparison window (e.g., at least 20 positions), wherein the portion of the polynucleotide or polypeptide sequence within the comparison window may include additions or deletions (i.e. gaps), e.g., for the two sequences that best match, there's a gap of 20% or less (e.g., 5 to 15%, or 10 to 12%) compared to the reference sequence (which does not contain additions or deletions). Percentages are generally calculated by determining the number of positions where the same nucleic acid base or amino acid residue occurs in the two sequences to yield the number of correctly matched positions, dividing the number of correctly matched positions by the total number of positions in the reference sequence (i.e., window size), and multiply the result by 100 to yield the percent of sequence identity.

In some specific embodiments, the chimeric receptors of the application are chimeric antigen receptors.

Chimeric antigen receptors typically comprise an extracellular antigen binding domain. In some specific embodiments, the extracellular antigen binding region can be fully human, humanized, murine, or the chimera in the extracellular antigen binding region is composed of amino acid sequences from at least two different animals.

Examples of extracellular antigen binding domains may be scFv, Fv, Fab, Fab', Fab'-SH, F(ab')2, single domain fragments, or natural ligands that engage their cognate receptors, and any derivative thereof.

In some aspects, the extracellular antigen binding region (e.g., scFv), may comprise light chain CDRs specific for the antigen. In some cases, light chain CDRs may comprise two or more light chain CDRs, which may be referred to as light chain CDR-1, CDR-2, etc. In some cases, light chain CDRs may comprise three light chain CDRs, which may be referred to as light chain CDR-1, light chain CDR-2, and light chain CDR-3, respectively. In one embodiment, a set of CDRs present on a common light chain may be collectively referred to as light chain CDRs.

In some aspects, the extracellular antigen binding region (e.g., scFv) may comprise heavy chain CDRs specific for the antigen. The heavy chain CDRs may be the heavy chain complementarity determining regions of an antigen binding unit such as scFv. In some cases, a heavy chain CDR may comprise two or more heavy chain CDRs, which may be referred to as heavy chain CDR-1, CDR-2, etc. In some cases, the heavy chain CDRs may comprise three heavy chain CDRs, which may be referred to as heavy chain CDR-1, heavy chain CDR-2, and heavy chain CDR-3, respectively. In one embodiment, a group of CDRs present on a common heavy chain may be collectively referred to as heavy chain CDRs.

By using genetic engineering, the extracellular antigen-binding region can be modified in various ways. In some cases, the extracellular antigen binding region can be mutated such that the extracellular antigen binding region can be selected to have a higher affinity for its target antigen. In some cases, the affinity of an extracellular antigen binding domain for its target antigen can be optimized for a target antigen that can be expressed at low levels on normal tissues. In other cases, clones of extracellular antigen-binding domains with higher affinity for the membrane-bound form of the target antigen may be preferred over their soluble counterparts.

In some instances, the extracellular antigen binding region also includes a hinge or spacer, the terms hinge and spacer can be used interchangeably. The hinge can be considered as part of the CAR used to provide flexibility to the extracellular antigen-binding region. For example, the hinge can be the native hinge region of the CD8α molecule.

The term "transmembrane domain" may anchor the chimeric protein at the plasma membrane of the cell. For example, the transmembrane domain of CD28, CD8α (also known as CD8 transmembrane domain) can be employed. Exemplarily, the CD8 transmembrane domain includes the amino acid sequence as shown by SEQ ID NO:45 or the nucleotide sequence as shown by SEQ ID NO:46. Exemplarily, the CD28 transmembrane domain includes the amino acid sequence as shown by SEQ ID NO:47 or the nucleotide sequence as shown by SEQ ID NO:48.

The term "modulate" refers to positive or negative changes. Examples of adjustments include changes of 1%, 2%, 10%, 25%, 50%, 75%, or 100%. In a specific embodiment, it refers to a negative change.

The term "treatment" refers to interventions in an attempt to alter the disease process, either prophylaxis or intervention in the clinicopathological process. Therapeutic effects include, but are not limited to, preventing the occurrence or recurrence of the disease, relieving symptoms, reducing any direct or indirect pathological consequences of the disease, preventing metastasis, slowing down the progression of the disease, improving or mitigating the condition, mitigating or improving the prognosis, etc. In a specific embodiment, the engineered T cells provided by the present application can inhibit tumor cell proliferation, and/or inhibit tumor cell proliferation and tumor volume increase in vivo.

The term "prevention" refers to interventions that are attempted in advance of a disease such as rejection of cell transplant.

The term "transplantation immune rejection" means that after the host has been transplanted with allogeneic tissues, organs, or cells etc., the foreign graft is recognized by the host's immune system as a "foreign component" and initiates immunological responses to attack, destroy and clear against the graft. The application provides a cell for resisting transplant immune rejection and a method for resisting and suppressing rejection.

The engineered T cells provided by the present application can be used to treat, prevent or improve autoimmune diseases or inflammatory diseases, especially inflammatory diseases related to autoimmune diseases, such as arthritis (such as rheumatoid arthritis, arthritis chronica progrediente and osteoarthritis) and rheumatic diseases, including inflammatory conditions and rheumatic diseases involving bone loss and inflammatory pain, spondyloarthropathy (including ankylosing spondylitis), Reiter Syndrome, reactive arthritis, psoriatic arthritis, juvenile idiopathic arthritis and enteropathic arthritis, enthesitis, hypersensitivity (including airway hypersensitivity and skin hypersensitivity), and anaphylaxis. The engineered T cells provided by the present application are used to treat and prevent diseases including autoimmune hematological disorders (including, for example, hemolytic anemia, aplastic anemia, pure red blood cell anemia and idiopathic thrombocytopenia), systemic lupus erythematosus (SLE), lupus nephritis, inflammatory muscle disease (dermatomyositis), periodontitis, polychondritis, scleroderma, Wegener's granulomatosis, dermatomyositis, chronic active hepatitis, myasthenia gravis, psoriasis, Steven Johnson syndrome, spontaneous sprue, autoimmune inflammatory bowel disease (including, for example, ulcerative colitis, Crohn's disease, and irritable bowel syndrome), endocrine eye disease, Graves' disease, tuberculosis, multiple sclerosis, systemic sclerosis, fibrotic disease, primary biliary cirrhosis, juvenile diabetes mellitus (type I diabetes), uveitis, keratoconjunctivitis sicca and vernal keratoconjunctivitis, interstitial pulmonary fibrosis, periprosthetic osteolysis, glomerulonephritis (with and without nephrotic syndrome including, for example, idiopathic nephrotic syndrome or minimal change nephropathy), multiple myeloma, other types of tumors, inflammatory diseases of the skin and cornea, myositis, loosening of bone implants, metabolic disorders (such as obesity, atherosclerosis, and other cardiovascular diseases including dilated cardiomyopathy, myocarditis, type II diabetes, and dyslipidemia), and autoimmunity thyroid disease (including Hashimoto's thyroiditis), primary vasculitis of small and medium vessels, large vessel vasculitis including giant cell arteritis, hidradenitis suppurativa, neuromyelitis optica, Sjogren's syndrome, Behcet's disease, atopic and contact dermatitis, bronchiolitis, inflammatory muscle disease, autoimmune peripheral neuropathy, immune renal, liver and thyroid disease, inflammation and atherosclerosis, autoinflammatory febrile syndrome, immunohematology disorders and bullous diseases of the skin and mucous membranes.

The engineered T cells provided by the present application can be used to treat, prevent or improve asthma, bronchitis, bronchiolitis, idiopathic interstitial pneumonia, pneumoconiosis, emphysema and other obstructive or inflammatory diseases of the airway.

The engineered T cells of the application may be administered as the sole active ingredient or in combination with other drugs such as immunosuppressants or immunomodulators or other anti-inflammatory or other cytotoxic or anti-cancer agents (e.g. as an adjuvant thereof or in combination with), for example to treat or prevent diseases related to immune disorders. For example, the antibodies of the application can be used in combination with the following drugs: DMARDs, such as gold salts, sulfasalazine, antimalarials, methotrexate, D-penicillamine, azathioprine, mycophenolic acid, tacrolimus, sirolimus, minocycline, leflunomide, glucocorticoids; calcineurin inhibitors such as cyclosporin A or FK 506; modulators of lymphocyte recirculation such as FTY720 and FTY720 analogs; mTOR inhibitors such as rapamycin, 40-O-(2-hydroxyethyl)-rapamycin, CCI779, ABT578, AP23573, or TAFA-93; ascomycins with immunosuppressive properties such as ABT-281, ASM981 etc.; corticosteroids; cyclophosphamide; azathioprine; leflunomide; mizoribine; mycophenolate mofetil; 15-deoxyspergualin or its immunosuppressive homologs, analogs or derivatives; immunosuppressive monoclonal antibodies, e.g., monoclonal antibodies to leukocyte receptors, e.g., MHC, CD2, CD3, CD4, CD7, CD8, CD25, CD28, CD40, CD45, CD58, CD80, CD86 or ligands thereof; other immunomodulatory compounds.

"Tumor antigen" refers to an antigen that emerges or is overexpressed during the onset, progression of a hyperproliferative disease. In certain aspects, the hyperproliferative disorder of the application refers to cancer.

The tumor antigens described in the present application may be solid tumor antigens or blood tumor antigens.

The tumor antigens of the present application include, but are not limited to: thyroid stimulating hormone receptor (TSHR); CD171; CS-1; C-type lectin-like molecule-1; ganglioside GD3; Tn antigen; CD19; CD20; CD 22; CD 30; CD 70; CD 123; CD 138; CD33; CD44; CD44v7/8; CD38; CD44v6; B7H3(CD276), B7H6; KIT(CD117); α subunit of interleukin-13 receptor (IL-13Rα), interleukin-11 receptor alpha (IL-11Rα); prostate stem cell antigen (PSCA); prostate-specific membrane antigen (PSMA); carcinoembryonic antigen (CEA); NY-ESO-1; HIV-1 Gag; MART-1; gp100; tyrosinase; Mesothelin; EpCAM; protease serine 21 (PRSS21); vascular endothelial growth factor receptor, vascular endothelial growth factor receptor 2 (VEGFR2); Lewis (Y) antigen; CD24; platelet-derived growth factor receptor Beta (PDGFR-β); stage-specific embryonic antigen-4 (SSEA-4); cell surface-associated mucin 1 (MUC1), MUC6; epidermal growth factor receptor family and mutants thereof (EGFR, EGFR2, ERBB3, ERBB4, EGFRvIII); neural cell adhesion molecule (NCAM); carbonic anhydrase IX (CAIX); LMP2; ephrin type A receptor 2 (EphA2); fucosyl-GM1; sialyl Lewis adhesion molecule (sLe); Ganglioside GM3; TGS5; high molecular weight melanoma-associated antigen (HMWMAA); ortho-acetyl GD2 ganglioside (OAcGD2); folate receptor; tumor vascular endothelial marker 1 (TEM1/CD248); tumor vascular endothelial marker 7-related (TEM7R); Claudin 6, Claudin18.2, Claudin18.1; ASGPR1; CDH16; 5T4; 8H9; α vβ 6 integrin; B cell maturation antigen (BCMA); CA9; kappa light chain ; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM 1; fibronectin; tenascin; carcinoembryonic variant of tumor necrosis region; G protein-coupled receptors class C group 5-member D (GPRC5D); X chromosome open reading frame 61 (CXORF61); CD97; CD179a; anaplastic lymphoma kinase (ALK); polysialic acid; placenta-specific 1 (PLAC1); Hexose part of globoH glycoceramide (GloboH); mammary gland differentiation antigen (NY-BR-1); uroplakin 2 (UPK2); hepatitis A virus cell receptor 1 (HAVCR1); adrenergic receptor beta 3 (ADRB3); pannexin 3 (PANX3); G protein coupled receptor 20 (GPR20); lymphocyte antigen 6 complex locus K9 (LY6K); olfactory receptor 51E2 (OR51E2); TCRγ alternate reading frame protein (TARP); Wilms tumor protein (WT1); ETS translocation variant gene 6 (ETV6-AML); spermatozoa protein 17 (SPA17); X antigen family member 1A (XAGE1); angiopoietin binding cell surface receptor 2 (Tie2); melanoma cancer testicular antigen-1 (MAD -CT-1); melanoma cancer testicular antigen-2 (MAD-CT-2); Fos-associated antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); paired box protein Pax-3 (PAX3); androgen receptor; cyclin B1; homologues derived from neuroblastoma of V-myc avian myelomatosis virus oncogene (MYCN); Ras homolog family member C (RhoC); cytochrome P450 1B1 (CYP1B1); CCCTC-binding factor (zinc finger protein)-like (BORIS); squamous cell carcinoma antigen 3 recognized by T cells (SART3); paired box protein Pax-5 (PAX5); proacrosin-binding protein sp32 (OYTES 1); Lymphocyte-specific protein tyrosine kinase (LCK); A kinase-anchored protein 4 (AKAP-4); synovial sarcoma X breakpoint 2 (SSX2); CD79a; CD79b; CD72; leukocyte-associated immunoglobulin-like receptor 1(LAIR1); Fc fragment of IgA receptor (FCAR); Leukocyte immunoglobulin-like receptor subfamily member 2 (LILRA2); CD300 molecule-like family member f (CD300LF); C-type lectin domain family 12 member A(CLEC12A); Bone marrow stromal cell antigen 2 (BST2); EGF-like module containing mucin-like hormone receptor-like 2 (EMR2); lymphocyte antigen 75 (LY75); Glypican-3 (GPC3) ; Fc receptor-like 5 (FCRL5); immunoglobulin lambda-like polypeptide 1 (IGLL1). Preferably, the tumor antigen is BCMA or CD 19.

Pathogen antigens are selected from: antigens of viruses, bacteria, fungi, protozoa, or parasites; virus antigens are selected from: cytomegalovirus antigens, Epstein-Barr virus antigens, human immunodeficiency virus antigens, or influenza virus antigens.

An engineered cell is provided in some embodiments, wherein the engineered T cell is genetically engineered to reduce the expression, activity and/or signaling of NKG2A in the cell.

In one embodiment, wherein the engineered T cell is engineered to reduce the expression, activity and/or signaling of endogenous NKG2A.

In some embodiments, after exogenous stimulation to the engineered T cell, the expression, activity of the endogenous NKG2A and/or the expression, activity of signaling related proteins are not up-regulated or slightly up-regulated.

In one embodiment, the engineered T cells comprise:
gene disruption of the gene encoding NKG2A and/or gene disruption resulting in reduced expression of NKG2A in the engineered T cell;
gene disruption of a gene encoding a protein regulating the expression or activity of NKG2A and/or gene disruption resulting in reduced expression of a protein regulating the expression or activity of NKG2A; and/or
gene disruption of a gene encoding a protein involved in NKG2A-dependent signaling and/or gene disruption resulting in reduced expression of a protein involved in NKG2A-dependent signaling.

In one embodiment, the use of gene editing techniques and/or gene silencing techniques results in reduced expression of NKG2A, reduced expression of proteins that regulate the expression or activity of NKG2A, and/or reduced expression of proteins involved in NKG2A-dependent signaling.

In one embodiment, the endogenous NKG2A gene/gene encoding proteins regulating the expression or activity NKG2A/gene encoding proteins involved in NKG2A-dependent signaling in the engineered T cell is knocked out by gene editing technology;
preferably, the gene editing technology is selected from CRISPR/Cas9 technology, ZFN technology, TALE technology, TALE-CRISPR/Cas9 technology, Base Editor technology, prime editing technology and/or homing endonuclease technology;
more preferably, the gene editing technology is CRISPR/Cas9 technology.

In one embodiment, the endogenous NKG2A gene of the engineered T cell is knocked out using CRISPR/Cas9 technology.

In one embodiment, the gRNA used in CRISPR/Cas9 technology is as shown by the sequence selected from the group consisting of: SEQ ID NO: 12, SEQ ID NO: 13, SEQ ID NO:14, SEQ ID NO:15, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and/or SEQ ID NO:68.

In one embodiment, the engineered T cell is a T cell derived from a natural T cell and/or induced from pluripotent stem cells;
preferably, the T cells are autologous/allogeneic T cells;
preferably, the T cells are primary T cells;
preferably, the T cells are derived from human autologous T cells.

In one embodiment, the T cells comprise memory stem cell-like T cells (Tscm cells), central memory T cells (Tcm), effector T cells (Tef), regulatory T cells (tregs), effector memory T cells (Tern), or γ δ T cells, or a combination thereof.

In one embodiment, the engineered T cells express exogenous protein 1 targeting one or more autologous or allogeneic immune cells or immune regulatory cells;
preferably, the immune cells or immunoregulatory cells include NK cells, activated T cells, Treg cells, myeloid-derived immunosuppressive cells, macrophages or a combination thereof.

In one embodiment, the exogenous protein 1 targets NK cells.

In one embodiment, the exogenous protein 1 targets NKG2A.

In one embodiment, the exogenous protein 1 comprises an antibody recognizing NKG2A or a functional fragment thereof;
preferably, the antibody recognizing NKG2A or the functional fragment thereof comprises HCDR1 as shown by SEQ ID NO:3, HCDR2 as shown by SEQ ID NO:4, HCDR3 as shown by SEQ ID NO:5, and LCDR1 as shown by SEQ ID NO:6, LCDR2 as shown by SEQ ID NO: 7, LCDR3 as shown by SEQ ID NO: 8; or
the antibody recognizing NKG2A or the functional fragment thereof comprises the heavy chain variable region as shown by SEQ ID NO:1 and/or the light chain variable region as shown by SEQ ID NO.1; or
the antibody recognizing NKG2A or the functional fragment thereof comprises the SCFV sequence as shown by SEQ ID NO:10.

In one embodiment, the engineered T cells also express exogenous protein 2, and the exogenous protein 2 recognizes tumor antigens or pathogen antigens;
preferably, the tumor antigens include BCMA, CD19, GPC3, Claudin18.2, EGFR or a combination thereof.

In one embodiment, the exogenous protein 2 comprises an antibody recognizing BCMA or a functional fragment thereof;
preferably, the antibody recognizing BCMA or a functional fragment thereof comprises HCDR1 as shown by SEQ ID NO: 16, HCDR2 as shown by SEQ ID NO: 17, HCDR3 as shown by SEQ ID NO: 18, LCDR1 as shown by SEQ ID NO: 19, LCDR2 as shown by SEQ ID NO:20, and/or LCDR3 as shown by SEQ ID NO:21; or
the antibody recognizing BCMA or a functional fragment thereof comprises the heavy chain variable region as shown by SEQ ID NO:22 and/or the light chain variable region as shown by SEQ ID NO: 23; or
the antibody recognizing BCMA or a functional fragment thereof comprises the SCFV sequence as shown by SEQ ID NO:24.

In one embodiment, the engineered T cells simultaneously express exogenous protein 1 and exogenous protein 2, the exogenous protein 1 targets NKG2A, and the exogenous protein 2 targets BCMA; preferably, the engineered T cells comprise the sequence as shown by SEQ ID NO:26 or SEQ ID NO:27.

In one embodiment, the exogenous protein 1 also targets tumors or pathogens; preferably, the tumors express proteins including BCMA, CD19, GPC3, Claudin18.2, EGFR or a combination thereof.

In one embodiment, the exogenous protein 1 further comprises an antibody recognizing BCMA or a functional fragment thereof;
preferably, the antibody recognizing BCMA or a functional fragment thereof comprises HCDR1 as shown by SEQ ID NO: 16, HCDR2 as shown by SEQ ID NO: 17, HCDR3 as shown by SEQ ID NO: 18, LCDR1 as shown by SEQ ID NO: 19, LCDR2 as shown by SEQ ID NO:20, and/or LCDR3 as shown by SEQ ID NO:21; or
the antibody recognizing BCMA or a functional fragment thereof comprises the heavy chain variable region as shown by SEQ ID NO:22 and/or the light chain variable region as shown by SEQ ID NO: 23; or
the antibody recognizing BCMA or a functional fragment thereof comprises the SCFV sequence as shown by SEQ ID NO:24.

In one embodiment, the exogenous protein 1 comprises an antibody recognizing NKG2A or a functional fragment thereof and an antibody recognizing a tumor antigen or a functional fragment thereof, and their linkage modes are:
(1) light chain/heavy chain (or light chain variable region/heavy chain variable region) of the antibody recognizing NKG2A-heavy chain/light chain (or heavy chain variable region/light chain variable region) of the antibody recognizing NKG2A—the heavy chain/light chain (or heavy chain variable region/light chain variable region) of the antibody recognizing a tumor antigen-the light chain/heavy chain (or the light chain variable region/heavy chain variable region) of the antibody recognizing a tumor antigen;
(2) the light chain (or light chain variable region) of the antibody recognizing a tumor antigen-the heavy chain (or heavy chain variable region) of the antibody recognizing NKG2A—the light chain (or light chain variable region) of the antibody recognizing NKG2A—the heavy chain (or heavy chain variable region) of the antibody recognizing a tumor antigen; or
(3) the light chain (or light chain variable region) of the antibody recognizing NKG2A—the heavy chain (or heavy chain variable region) of the antibody recognizing a tumor antigen-the light chain (or light chain variable region) of the antibody recognizing a tumor antigen-the heavy chain (or heavy chain variable region) of the antibody recognizing NKG2A;
preferably, the exogenous protein 1 comprises the amino acid sequence as shown by SEQ ID NO:30 and/or SEQ ID NO:32.

In one embodiment, the exogenous protein 1 and/or exogenous protein 2 is a chimeric antigen receptor (CAR), a chimeric T cell receptor, a T cell antigen coupler (TAC) or a combination thereof; preferably, the CAR comprises:
(i) an antibody specifically binding to the target antigen or a functional fragment thereof, the transmembrane region of CD28 or CD8, and CD3ζ;
(ii) an antibody specifically binding to the target antigen or a functional fragment thereof, the transmembrane region of CD28 or CD8, the co-stimulatory signal domain of CD28 and CD3ζ;
(iii) an antibody specifically binding to a target antigen or a functional fragment thereof, the transmembrane region of CD28 or CD8, the co-stimulatory signal domain of CD137 and CD3ζ; and/or
(iv) an antibody specifically binding to a target antigen or a functional fragment thereof, the transmembrane region of CD28 or CD8, the co-stimulatory signal domain of CD28, the co-stimulatory signal domain of CD 137, and CD3ζ.

In one embodiment, the exogenous protein 1 is a chimeric antigen receptor 1, and the target antigen recognized by the chimeric antigen receptor 1 includes T cells, NK cells, Treg cells, myeloid-derived immunosuppressive cells (MDSC), macrophage molecular markers or a combination thereof; or the target antigen recognized by the chimeric antigen receptor 1 also includes tumor antigens and/pathogen antigens; the exogenous protein 2 is a chimeric antigen receptor 2, and the target antigens recognized by the chimeric antigen receptor 2 include tumor antigens and/ pathogen antigens.

In one embodiment, the chimeric antigen receptor 1 targets NKG2A or targets NKG2A and BCMA; the chimeric antigen receptor 2 targets BCMA; preferably, the chimeric antigen receptor 1 comprises the sequence as shown by SEQ ID NO: 9, SEQ ID NO: 30, SEQ ID NO: 32, SEQ ID NO: 55, and/or SEQ ID NO: 57; the chimeric antigen receptor 2 comprises the sequence as shown by SEQ ID NO: 25.

In one embodiment, the engineered T cells also comprise:
knockout of genes encoding TCR proteins and/or low or no expression of endogenous TCR molecules;
knockout of genes encoding MHC proteins and/or low or no expression of endogenous MHC;
preferably, the TCR is a TRAC molecule, and the MHC is a B2M molecule.

In one embodiment, endogenous MHC and endogenous TCR are knocked out using CRISPR/Cas9 technology; preferably, in CRISPR/Cas9 technology, the gRNA used to knock out B2M is selected from the sequence as shown by SEQ ID NO: 54, the gRNA used to knock out TRAC is selected from the sequence as shown by SEQ ID NO:53.

In one embodiment, compared with a T cell that has not been engineered, the engineered T cell has an increase in immune activity of about 10%, 20%, 30%, 40%, 50%, 60%, 70%, 80%, 90%, or 100%.

In one embodiment, compared with a T cell that has not been engineered, the engineered T cell has an increased resistance to NK cells and/or an increase in the killing ability of NK cells by about 10%, 20%, 30%, 40%, 50%, 60%, 70%, 80%, 90% or 100%.

In one embodiment, compared with a T cell that has not been engineered, the engineered T cell inhibits tumor cell proliferation, and/or inhibit tumor cell proliferation in vivo, and increase tumor volume by about 10%, 20%, 30% 40%, 50%, 60%, 70%, 80%, 90%, or 100%.

In one embodiment, compared with a T cell that has not been engineered, the expression, activity and/or signaling of NKG2A in the activated engineered T cell is reduced by greater than about 10%, 20%, 30%, 40%, 50%, 60%, 70%, 80%, 90%, 95%, or 100%.

In one embodiment, the engineered T cells can improve the kiling of tumor cells by T cells and/or CAR-T cells carrying a CAR for target antigens that were introduced into the subject earlier, simultaneously, and later, and enhance both survival and proliferation of the T cells and/or CAR-T cells.

Some embodiments provide nucleic acids, expression vectors, viruses for use in preparing any of the engineered T cells mentioned herein.

Some embodiments provide methods for preparing any of the engineered T cells mentioned herein, or the nucleic acids, expression vectors, or viruses described above.

Some embodiments provide a method for producing engineered T cells, comprising genetically engineering T cells to reduce the expression, activity and/or signaling of NKG2A in the cells; preferably, reducing expression comprises:
disrupting or inhibiting the gene encoding NKG2A and/or disrupting or inhibiting a gene in the T cells, resulting in reduced expression of NKG2A;
disrupting or inhibiting a gene encoding a protein regulating the expression or activity of NKG2A and/or disrupting or inhibiting a gene in the T cell, resulting in reduced expression of a protein regulating the expression or activity of NKG2A; and/or
disrupting or inhibiting a gene encoding a protein involved in NKG2A-dependent signaling and/or disrupting or inhibiting a gene in the T cell, resulting in a decrease in the expression of a protein involved in NKG2A-dependent signaling.

In one embodiment, gene disruption is achieved by introducing into the T cell an endonuclease targeting the gene;
preferably, the endonuclease is selected from TAL nuclease, meganuclease, zinc finger nuclease, Cas9 and Argonaute;
preferably, the endogenous NKG2A gene of the T cell is knocked out using CRISPR/Cas9 technology.

In one embodiment, compared with the expression in T cells that have not been engineered, the expression, activity and/or signaling of NKG2A in the engineered T cells is reduced by greater than or greater than about 10%, 20%, 30%, 40%, 50%, 60%, 70%, 80%, 90%, 95% or 100%.

In one embodiment, the engineered T cells are derived from T cells isolated from humans;
preferably, comprising isolated T cells derived from peripheral blood mononuclear cells (PBMC);
preferably, the T cells are primary T cells.

In one embodiment, the cells are genetically engineered to express chimeric antigen receptor (CAR), modified T cell (antigen) receptor (TCR), T cell fusion protein (TFP), T cell antigen coupler (TAC), aTCR-T or a combination thereof; preferably, the CAR comprises:
(i) an antibody specifically binding the target antigen or a functional fragment thereof, the transmembrane region of CD28 or CD8, the co-stimulatory signal domain of CD28 and CD3ζ; and/or
(ii) an antibody specifically binding to a target antigen or a functional fragment thereof, the transmembrane region of CD28 or CD8, the co-stimulatory signal domain of CD28 and CD3ζ; and/or
(iii) an antibody specifically binding to a target antigen or a functional fragment thereof, the transmembrane region of CD28 or CD8, the co-stimulatory signal domain of CD137 and CD3ζ; and/or
(vi) an antibody specifically binding to a target antigen or a functional fragment thereof, the transmembrane region of CD28 or CD8, the costimulatory signal domain of CD28, the costimulatory signal domain of CD137 and CD3ζ.

In one embodiment, the CAR comprises an antibody capable of specifically recognizing BCMA:
the antibody specifically recognizing BCMA comprises HCDR1 as shown by SEQ ID NO:16, HCDR2 as shown by SEQ ID NO:17, HCDR3 as shown by SEQ ID NO:18, LCDR1 as shown by SEQ ID NO:19, LCDR2 as shown by SEQ ID NO:20, and/or LCDR3 as shown by SEQ ID NO:21; or
comprises the heavy chain variable region as shown by SEQ ID NO:22 and/or the light chain variable region as shown by SEQ ID NO:23; or
comprises the sequence as shown by SEQ ID NO:24.

In one embodiment, also included is to genetically engineer the T cells to reduce the expression, activity and/or signaling of TCR and MHC in the cells; preferably, the TCR includes TRAC, and the MHC includes B2M.

In one embodiment, gene disruption is achieved by introducing an endonuclease targeting the gene into the T cells; preferably, the endonuclease is selected from TAL nuclease, meganuclease, zinc finger nuclease, Cas9 and Argonaute; preferably, CRISPR/Cas9 technology is used to knock out TRAC and B2M genes in the T cell.

In one embodiment, compared with the expression in T cells that have not been engineered, the TCR and MHC expression, activity and/or signaling of the genetically engineered T cells are reduced by greater than or greater than about 50%, 60%, 70%, 80%, 90% or 95%.

In one embodiment, the T cells are genetically engineered to express an antibody recognizing NKG2A or a functional fragment thereof, and/or express a chimeric antigen receptor (CAR), a modified T cell (antigen) receptor (TCR), T cell fusion protein (TFP), T cell antigen coupler (TAC), or aTCR-T specifically binding to NKG2A or a combination thereof; preferably, the CAR comprises:
(i) an antibody specifically binding to NKG2A or a functional fragment thereof, the transmembrane region of CD28 or CD8, the co-stimulatory signal domain of CD28 and CD3ζ; and/or
(ii) an antibody specifically binding to NKG2A or a functional fragment thereof, the transmembrane region of CD28 or CD8, the co-stimulatory signal domain of CD28 and CD3ζ; and/or
(iii) an antibody specifically binding to NKG2A or a functional fragment thereof, the transmembrane region of CD28 or CD8, the co-stimulatory signal domain of CD137 and CD3ζ; and/or
(vi) an antibody specifically binding to NKG2A or a functional fragment thereof, the transmembrane region of CD28 or CD8, the costimulatory signal domain of CD28, the costimulatory signal domain of CD137 and CD3ζ.

In one embodiment, the antibody specifically binding to NKG2A comprises HCDR1 as shown by SEQ ID NO:3, HCDR2 as shown by SEQ ID NO:4, HCDR3 as shown by SEQ ID NO:5, LCDR1 as shown by SEQ ID NO:6, LCDR2 as shown by SEQ ID NO:7, and/or LCDR3 as shown by SEQ ID NOG; or
the antibody specifically binding to NKG2A comprises the heavy chain variable region as shown by SEQ ID NO:1 and/or the light chain variable region as shown by SEQ ID NO:2; or
the antibody specifically binding to NKG2A comprises the sequence as shown by SEQ ID NO:10.

In some specific embodiments, provided is an engineered T cell produced by any of the methods mentioned herein.

In some embodiments, provided is a composition comprising an effective amount of any of the engineered T cells mentioned herein, and/or an effective amount of the nucleic acid, expression vector, virus or combination thereof used to prepare any of the mentioned engineered T cells herein;
preferably, a pharmaceutically acceptable carrier is also included;
preferably, the carrier is saline solution, dextrose solution or 5% human serum albumin;
preferably, a cryoprotectant is also included.

A kit is provided in some specific embodiments, wherein it comprises any of the engineered T cells mentioned herein, the nucleic acid, expression vector, or virus described herein, and/or the composition described herein as well as additional agents for treatment of diseases.

A method for treating a disease is provided in some embodiments, comprising administering to a subject in need any of the engineered T cells mentioned herein, the nucleic acid, expression vector, virus described herein, or the composition described herein, and/or the kit described herein;
preferably, before administering the engineered T cells, the engineered T cells are produced according to any of the methods mentioned herein;
preferably, the subject is a human;
preferably, wherein the engineered T cells are autologous or allogeneic T cells.

In one embodiment, the disease includes immune disease; preferably, the immune disease includes autoimmune disease and/or anti-graft immune rejection.

In one embodiment, the disease includes tumor; preferably, the tumor includes hematologic tumor and/or solid tumor.

In one embodiment, *in vivo* introduction of engineered T cells with reduced expression, activity and/or signaling of endogenous NKG2A can improve the kiling of target cells by CAR-T cells carrying targeted antigen that is introduced into the subject earlier, simultaneously and later, and enhance the survival and proliferation of the CAR-T cells.

Some embodiments provide engineered T cells in which endogenous NKG2A is knocked out or silenced. These embodiments provide engineered T cells that are genetically engineered to have reduced NKG2A expression, activity and/or signaling in the cells. Methods of engineering the cells are also provided. In some embodiments, the method includes introducing gene knockout or inhibitory nucleic acid, resulting in knockout or silencing of NKG2A gene, and/or a gene of upstream or downstream signal molecule related to NKG2A signal pathway, resulting in reduced expression, activity and/or signaling of NKG2A in the T cells.

In some embodiments, the engineered T cells are genetically engineered to directly reduce or eliminate the expression or activity of NKG2A.

In some specific embodiments, the engineered T cells are genetically engineered to indirectly reduce or eliminate the expression or activity of NKG2A, such as by reducing or eliminating the expression of a protein regulating the expression or activity of NKG2A (e.g., a transcription factor that controls the expression of NKG2A). In some specific embodiments, the engineered T cells are genetically engineered to reduce or eliminate the expression or activity of molecules involved in downstream signaling of NKG2A.

In some specific embodiments, the CRISPR/Cas9 system is used for gene editing of NKG2A gene, and/or molecules interacted with NKG2A, and/or the upstream or downstream signal molecules related to the NKG2A signal pathway.

In some embodiments, the sequence of gRNA or sgRNA in the CRISPR/Cas9 system is as shown by SEQ ID NO:12, SEQ ID NO:13, SEQ ID NO:14, or SEQ ID NO:15.

In some embodiments, the endogenous NKG2A in the T cell is not induced to be expressed or only weakly expressed under the repeated stimulation of target cells *in vivo* or *in vitro,* wherein the NKG2A gene in the engineered T cells is edited using the CRISPR/Cas9 system.

In some specific embodiments, the *in vivo* or *in vitro* anti-tumor activity of the T cells with NKG2A gene edited using the CRISPR/Cas9 system is enhanced.

In some specific embodiments, the *in vivo* or *in vitro* anti-NK-cell activity of the T cells with NKG2A gene edited using the CRISPR/Cas9 system is enhanced, and the cell survival and proliferation capabilities of the T cells in the presence of NK cells are enhanced.

In one embodiment, the CRISPR/Cas9 system is used to knock out NKG2A, TCR, and B2M genes, which can be used to prepare allogeneic T cells.

Some embodiments provide engineered T cells expressing chimeric receptors targeting non-tumor antigens and with endogenous NKG2A knockout or silenced. In these embodiments, the engineered T cells are genetically engineered to directly reduce or eliminate the expression or activity of NKG2A, the engineered cells are also genetically engineered to express chimeric receptors (CAR, modified TCR, TFP, TAC, aTCR or a combination thereof).

In a specific embodiment, the targeted non-tumor antigen may be one or more *in vivo* molecules expressed on immune and immune regulatory cells (also known as exogenous protein 1). Such molecules may be molecular markers of T cells or NK cells; molecular markers of Treg cells, myeloid-derived immunosuppressive cells (MDSC), or macrophages.

In specific embodiments, the above-mentioned engineered endogenous NKG2A knockout or silenced T cells express a chimeric molecule targeting NK cells to protect the engineered T cells from being eliminated in the host individual. The embodiments also include, for example, methods for avoiding an allogeneic immune response in an individual receiving a tissue or organ transplant.

In one embodiment, the resistance to NK cells *in vitro* is significantly improved in the T cells by the combination of the knockout or silencing of endogenous NKG2A and the expression of NKG2A-CAR, thereby maintains a high survival rate of the T cells. In one embodiment, the knockout or silencing of endogenous NKG2A and the expression of NKG2A-CAR can significantly improve the killing of NK cells by T cells in immune-competent animals, and further improve the survival and proliferation of the T cells in immune-competent animals. In one embodiment, T cells or CAR-T cells with endogenous NKG2A knockout or silenced and NKG2A-CAR expressed can still effectively secrete cytotoxic factors and kill tumor cells.

In one embodiment, provided are engineered allogeneic T cells expressing NKG2A-CAR and with endogenous NKG2A, TCR, B2M knockout or silenced.

Some embodiments provide engineered T cells expressing chimeric antigen receptors targeting to tumors or viruses and with endogenous NKG2A knockout or silenced. In one embodiment the chimeric antigen receptor targets BCMA, GPC3, or Claudin18.2.

In one embodiment, the *in vivo* or *in vitro* anti-tumor activity of the engineered T cells expressing BCMA-CAR and with endogenous NKG2A knocked out or silenced is enhanced; the killing of NK cells in immune-competent animals is improved, further, survival and proliferation of the T cells in immune-competent animals are improved.

In one embodiment, the resistance to NK cells *in vitro* of the T cells expressing BCMA-CAR, and with endogenous NKG2A knockout or silenced in combination with the expression of NKG2A-CAR is significantly increased, thereby maintaining a high survival rate of T cells; significantly improving the killing effect of T cells on NK cells in immune-competent animals, and further improving the survival and proliferation of T cells in immune-competent animals.

In one embodiment, T cells or CAR-T cells expressing BCMA-CAR, and with endogenous NKG2A knocked out or silenced and NKG2A-CAR expression can still effectively secrete cytotoxic factors and kill tumor cells.

In one embodiment, provided are engineered allogeneic T cells expressing BCMA-CAR and NKG2A-CAR, and with endogenous NKG2A, TCR, B2M knockout or silenced.

Some embodiments provide engineered T cells in which antibodies targeting tumor or viral antigens and antibodies targeting NK cells are expressed in tandem on the same chimeric antigen receptor, and endogenous NKG2A is knocked out or silenced.

In one embodiment the chimeric antigen receptor targets BCMA and NKG2A.

In one embodiment, the *in vivo* or *in vitro* anti-tumor activity of the engineered T cells with tandem CAR targeting BCMA and NKG2A and with endogenous NKG2A knockout or silenced is enhanced; the killing of NK cells in immune-competent animals is enhanced, further improving survival and proliferation of the T cells in immune-competent animals.

In one embodiment, the *in vitro* resistance to NK cells of the T cells with tandem CAR targeting BCMA and NKG2A, and with endogenous NKG2A knockout or silenced is significantly improved, thereby maintaining a high survival rate of T cells; significantly improving the killing effect of T cell on NK cells in immune-competent animals, further improving the survival and proliferation of the T cells in immune-competent animals; two tandem expressing CAR-T cells, BCMA-loop-NKG2A UCAR-T and NKG2A-loop-BCMA UCAR-T can both significantly inhibit the growth of tumor cells *in vivo,* and have similar levels of anti-tumor activity.

In one embodiment, T cells or CAR-T cells with tandem CAR targeting BCMA and NKG2A, and endogenous NKG2A knocked out or silenced can still effectively secrete cytotoxic factors and kill tumor cells.

In one embodiment, provided are engineered allogeneic T cells with tandem CAR targeting BCMA and NKG2A, and with endogenous NKG2A, TCR, B2M knocked out or silenced.

In some embodiments, the engineered T cells are genetically engineered to directly reduce or eliminate the expression or activity of NKG2A and TCR, the cells are also genetically engineered to express at least two chimeric receptors (CAR, modified TCR, TFP, TAC, aTCR or a combination thereof), wherein at least one of the chimeric receptors specifically binds to a tumor antigen or a viral antigen, and at least one of the chimeric receptors specifically binds to NKG2A. In some specific embodiments, the engineered T cells are genetically engineered to directly reduce or eliminate NKG2A and TCR expression or activity, the cells are also genetically engineered to express an NKG2A antibody or a functional fragment thereof and at least one chimeric receptor (CAR, modified TCR, TFP, TAC, aTCR, or a combination thereof), wherein at least one of the chimeric receptors specifically binds a tumor antigen or a viral antigen.

CAR-T cells and preparation methods thereof disclosed in, for example, Chinese patent application publication No. CN107058354A, CN107460201A, CN105194661A, CN105315375A, CN105713881A, CN106146666A, CN106519037A, CN106554414A, CN105331585A, CN106397593A, CN106467573A, CN104140974A, CN108884459A, CN107893052A, CN108866003A, CN108853144A, CN109385403A, CN109385400A, CN109468279A, CN109503715A, CN109908176A, CN109880803A, CN110055275A, CN110123837A, CN110438082A, CN110468105A, and international patent application publication numbers WO2017186121A1, WO2018006882A1, WO2015172339A8, WO2018/018958A1, WO2014180306A1, WO2015197016A1, WO2016008405A1, WO2016086813A1, WO2016150400A1, WO2017032293A1, WO2017080377A1, WO2017186121A1, WO2018045811A1, WO2018108106A1, WO2018/219299, WO2018/210279, WO2019/024933, WO2019/114751, WO2019/114762, WO2019/141270, WO2019/149279, WO2019/170147A1, WO2019/210863, WO2019/219029 are incorporated in this patent application.

The present application is further described in combination with specific embodiments. It should be understood that these examples are only used to illustrate the present application and are not intended to limit the scope of the present application. The experimental method that does not indicate specific condition in the following examples, are usually according to conventional conditions described in such as J. Sambrook et al., eds., A Laboratory Guide to Molecular Cloning, Third Edition, Science Press, 2002, or as recommended by the manufacturer.

### Example 1: Preparation of T cells overexpressing CAR targeting NKG2A and BCMA

In order to verify the effect of NKG2A knockout, we respectively constructed engineered T cells expressing chimeric antigen receptor (CAR) targeting BCMA and CAR targeting both BCMA and NKG2A to test the effect of NKG2A knockout on anti-tumor activity (targeting tumor antigens) and the effect on NK cell clearance (targeting non-tumor antigens).

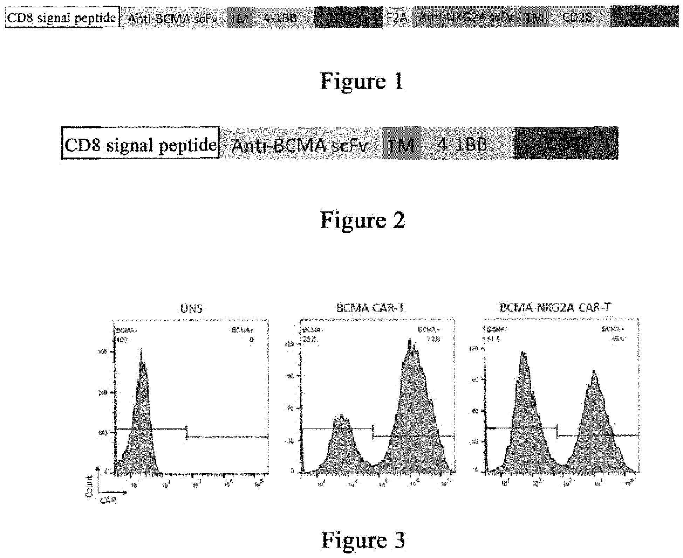

Using conventional molecular biology methods of the field, with PRRLsin as the vector, a lentiviral vector PRRLsin-BCMA-NKG2A-CAR (abbreviated as BCMA-NKG2A-CAR) expressing NKG2A and BCMA chimeric antigen receptors was constructed (Figure 1). Sequence of BCMA-NKG2A-CAR (amino acid sequence as shown by SEQ ID NO: 26) consists of CD8 signal peptide (SEQ ID NO: 41, SEQ ID NO: 42), single-chain antibody of BCMA (the amino acid sequence of VH is as shown by SEQ ID NO : 22, the amino acid sequence of VL is as shown by SEQ ID NO: 23), CD8 hinge (SEQ ID NO: 43, SEQ ID NO: 44), CD8 transmembrane domain (SEQ ID NO: 45, SEQ ID NO: 46), 4-1BB co-stimulatory factor (SEQ ID NO:49, SEQ ID NO:50), T cell activating factor CD3ζ (SEQ ID NO:51, SEQ ID NO:52), F2A (SEQ ID NO:40), the single-chain antibody of NKG2A (the amino acid sequence of VH is as shown by SEQ ID NO: 1, the amino acid sequence of VL is as shown by SEQ ID NO: 2), CD8 hinge (SEQ ID NO: 43, SEQ ID NO: 44), transmembrane and intracellular domain of CD28 (SEQ ID NO:28, SEQ ID NO:29), and T cell activating factor CD3ζ (SEQ ID NO:51, SEQ ID NO:52).

Construction of the lentiviral plasmid PRRLsin-BCMA-CAR (Fig. 2) expressing the chimeric antigen receptor of BCMA, and comprising CD8 signal peptide (SEQ ID NO:41, SEQ ID NO:42), the single-chain antibody of BCMA (the amino acid sequence of VH is as shown by SEQ ID NO:22, the amino acid sequence of VL is as shown by SEQ ID NO:23), CD8 hinge (SEQ ID NO:43, SEQ ID NO:44), transmembrane domain of CD8 (SEQ ID NO:45, SEQ ID NO:46), 4-1BB costimulatory factor (SEQ ID NO:49, SEQ ID NO:50), T cell activating factor CD3ζ (SEQ ID NO:51, SEQ ID NO:52).

The above two vectors were transfected into 293T cells, and the lentiviruses were packaged to obtain the corresponding lentiviruses BCMA-NKG2A-CAR and BCMA-CAR respectively. The infection method is a conventional infection method in the field of preparing T cells expressing chimeric antigen receptors.

Ficoll-Paque (GE bioscience) was used for density gradient centrifugation to separate peripheral blood mononuclear cells (PBMC), and anti-CD3/CD28 magnetic beads were added to obtain T cells after *in vitro* activation and expansion. The T cells infected and activated by lentivirus BCMA-CAR and BCMA-NKG2A-CAR were cultured and expanded to the required number to obtain BCMA CAR-T cells and BCMA-NKG2A CAR-T cells, respectively. Cells not transfected with virus were named UTD cells. The antibody anti-BCMA single-chain antibody was used as the primary antibody, and the anti-biotin PE antibody was used as the secondary antibody for flow cytometry detection. The results are shown in Figure 3. The positive rate of BCMA CAR-T cells was about 50%, and the positive rate of BCMA-NKG2A CAR-T cells was about 40%.

### Example 2: Preparation of UCAR-T cells and NKG2A knockout UCAR-T cells

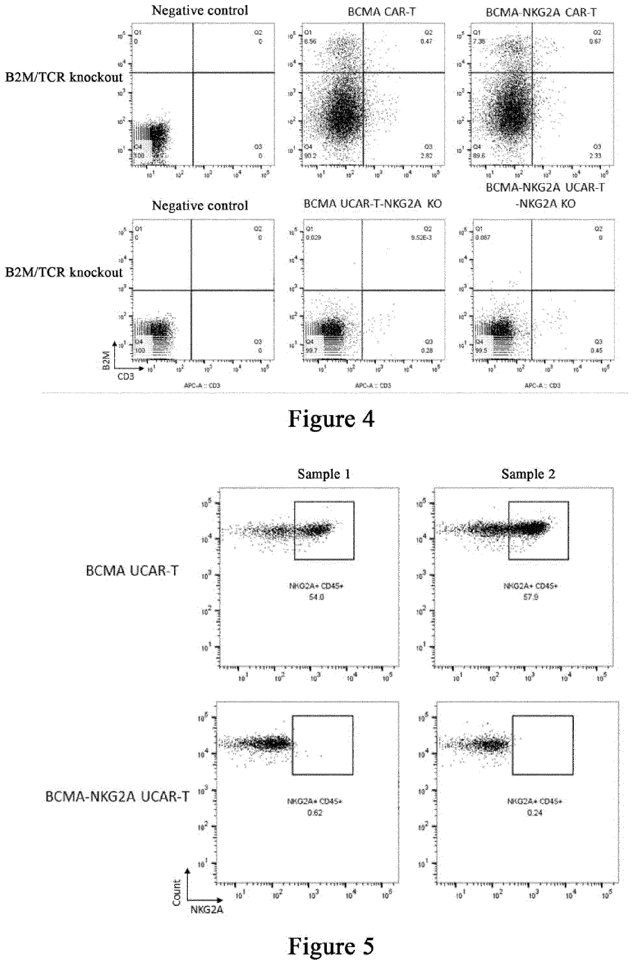

The sgRNA sequences targeting TRAC, B2M and NKG2A (SEQ ID NO:53, SEQ ID NO:54, SEQ ID NO:13) were synthesized *in vitro* according to the reagent instructions (GeneArt^{™} Precision gRNA Synthesis Kit, thermo Tisher). On the fifth day after virus transfection, the CAR-T cells in Example 1 were subjected to double knockout of TRAC and B2M genes or triple knockout of TRAC/B2M/NKG2A, respectively. The Cas9 enzyme and gRNA were incubated at room temperature at a ratio of 1:4, and the cells were mixed with the Cas9 enzyme and sgRNA complex (RNP) (the final concentration of the Cas 9 enzyme was 2uM for TRAC/B2M double-knockout, the final concentration of Cas 9 enzyme was 3uM for TRAC/B2M/NKG2A triple knockout), and the RNP complex was introduced into CAR-T cells by maxcyte electroporation instrument. Gene-knocked out CAR-T cells were expanded *in vitro,* the cells were labeled with anti-B2M and CD3 antibodies, and then labeled with a secondary antibody conjugated with phycoerythrin (PE). The labeled cells were treated with anti-PE magnetic beads and sorted by the sorting column, collecting the CD3 and B2M double-negative cells (the sorting kit was purchased from Miltenyi), and obtained universal UCAR-T cells with TCR and B2M deletion (BCMA UCAR-T, BCMA-NKG2A UCAR-T) or universal UCAR-T cells with NKG2A knockout and TCR and B2M deletion (BCMA UCAR-T-NKG2A KO, BCMA-NKG2A UCAR-T-NKG2A KO). The removal efficiencies of TCR and B2M in UCAR-T cells were detected by flow cytometry. The results showed that efficiency of B2M/TCR knockout in the B2M/TCR double knockout and B2M//TCR triple knockout groups was about 90%, and the B2M/TCR double-negative ratio after sorting was about 99% (Figure 4). Correspondingly, after TCR and B2M knockout and sorting (B2M/TCR removal), T cells that were not transfected with virus were named UTD UCAR-T.

### Example 3: NKG2A expression and knockout detection

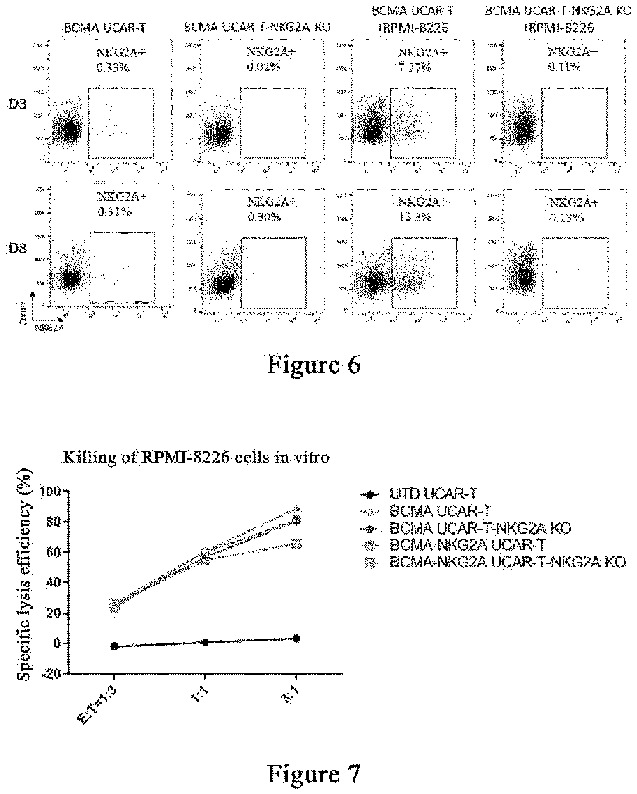

Since NKG2A is hardly expressed in normal cultured T cells (Figure 6), we injected T cells into mice bearing target cells to detect the expression and knockout of NKG2A in T cells, so as to judge the knockout status of NKG2A in UCAR-T cells with triple knockout of TRAC/B2M /NKG2A.

The multiple myeloma cell line RPMI-8226 was cultured *in vitro,* and 5×10⁶ cells were inoculated subcutaneously in NPG immunodeficient mice. The tumor volume was measured 12 to 14 days after inoculation for screening and grouping. The average tumor volume was about 350mm³, they were divided into 2 groups (BCMA UCAR-T, BCMA-NKG2A UCAR-T), 2 rats in each group. 1.5×10⁶ BCMA UCAR-T and BCMA-NKG2A UCAR-T cells were injected through the tail vein respectively (denoted as day 0 D0). After day 12, the peripheral blood of the mice was taken for flow cytometric detection, as shown in figure 5, anti-NKG2A flow antibody was used for staining, on day 12, the expression of NKG2A in BCMA UCAR-T cells in two RPMI-8226 tumor-bearing NPG mice was significantly up-regulated, up to 50-60% of BCMA UCAR-T cells expressed NKG2A; and on day 12, the expression level of NKG2A in BCMA-NKG2A UCAR-T cells in two RPMI-8226 tumor-bearing NPG mice was very low, presumably because that after BCMA-NKG2A UCAR-T cells were stimulated by tumor cells, positive cells induced to express NKG2A will be self-eliminated by BCMA-NKG2A UCAR-T cells, thus the detected expression level of NKG2A was very low (Figure 5).

Further, *in vivo* conditions were simulated by repeatedly stimulating tumor cells, and BCMA UCAR-T-NKG2A KO with NKG2A knockout and BCMA UCAR-T are used for detection. The above cells were co-incubated with RPMI-8226 tumor cells expressing BCMA antigen at a ratio of 1:1 (denoted as D0 day), and detected on day 3, and at the same time, they were co-incubated for the second time at a ratio of 1:1. On day 8, they were detected for the second time. Stained with anti-NKG2A antibody for flow cytometry, the detection results in Figure 6 show that when RPMI-8226 tumor cells were not added, the expression of NKG2A in BCMA UCAR-T and BCMA UCAR-T-NKG2A KO cells was very weak on day 3 and day 8; when co-incubated with tumor cells for 8 days, the expression of NKG2A in BCMA UCAR-T cells was significantly up-regulated; while the expression of NKG2A in BCMA UCAR-T-NKG2A KO cells was still weak after co-incubating with tumor cells for 8 days, indicating that endogenous NKG2A was efficiently knocked out.

The results in Figure 5 and Figure 6 also show that co-incubation of CAR-T cells with target cells or repeated stimulation by target cells can significantly increase the expression of endogenous NKG2A in CAR-T cells; knockout of endogenous NKG2A from CAR-T cells significantly inhibited the expression of NKG2A in CAR-T cells incubated with target cells (figure 6). It should be noted that it can be speculated that after T cells are exposed to pathogenic microorganisms such as viruses and bacteria, NKG2A is also likely to be up-regulated after being exposed to similar pathogenic microorganisms again, which is similar to previous reports that the expression of NKG2A was significantly upregulated in T cells treated with tumor vaccines (Cell 175, 1744-1755, December 13, 2018). The above results indicate that under the repeated stimulation by target cells *in vivo* or *in vitro,* endogenous NKG2A will be significantly up-regulated in T cells or CAR-T; and when the NKG2A gene is knocked out, under repeated stimulation of target cells *in vivo* or *in vitro,* endogenous NKG2A will not be induced to express or only express weakly in T cells or CAR-T.

### Example 4: detection of in vitro tumor killing function

The multiple myeloma cell line RPMI-8226 was cultured *in vitro* as tumor target cells. Cell density was adjusted, a certain amount of cells were inoculated into a 96-well plate, and the corresponding UCAR-T cells were inoculated according to three ratios of effector T cells: target cells: 1:3, 1:1 and 3:1. RPMI-1640+10% FBS was used as the medium, and were co-incubated in 37°C, 5% CO₂ incubator for 18 hours. According to the kit (CytoTox 96^{®} Non-Radioactive Cytotoxicity Assay, promega company) instruction, the supernatant was taken for lactate dehydrogenase (LDH) content determination, and the lysis efficiency of RPMI-8226 cells in each group of UTD UCAR-T, BCMA UCAR-T, BCMA-NKG2A UCAR-T, BCMA UCAR-T-NKG2A KO, and BCMA-NKG2A UCAR-T-NKG2A KO were calculated. The test results are shown in Figure 7. Both BCMA UCAR-T-NKG2A KO and BCMA-NKG2A-UCAR-T-NKG2A KO can effectively kill RPMI-8226 cells, and the killing ability is comparable to that of BCMA UCAR-T and BCMA-NKG2A UCAR-T, indicating that BCMA UCAR-T-NKG2A KO and BCMA-NKG2A UCAR-T-NKG2A KO can effectively kill myeloma cells. Therefore, endogenous NKG2A knockout CAR-T cells can still effectively kill target cells.

### Example 5: detection of in vivo anti-tumor function

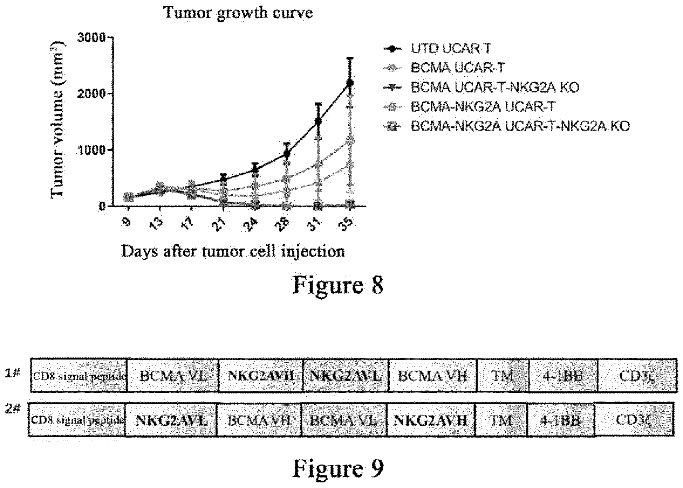

The multiple myeloma cell line RPMI-8226 was cultured *in vitro,* and 5×10⁶ cells were inoculated subcutaneously in NPG immunodeficient mice. 12-14 days after inoculation, the average tumor volume was about 150-200mm³, and they were divided into 5 groups (UTD UCAR-T, BCMA UCAR-T, BCMA-NKG2A UCAR-T, BCMA UCAR-T-NKG2A KO, BCMA-NKG2A UCAR-T-NKG2A KO), 5 mice in each group. 1×10⁶ above-mentioned UTD UCAR-T, BCMA CAR-T, BCMA UCAR-T, BCMA-NKG2A UCAR-T, BCMA UCAR-T-NKG2A KO, and BCMA-NKG2A UCAR-T-NKG2A KO cells were injected into the tail vein respectively; after injection, the body weight was measured twice a week (including the day of grouping and administration and the day for euthanasia), the long diameter and short diameter of the tumors were measured and recorded with a vernier caliper, the tumor volume was calculated, the tumor growth curve was drawn according to the tumor volume, and the difference of tumor growth curves among groups was compared (tumor volume: V=1/2×long diameter × short diameter²). The test results are shown in Figure 8. Compared with UTD UCAR-T, each group can significantly inhibit the growth of tumor volume; among them, the BCMA UCAR-T-NKG2A KO group can significantly inhibit the growth of tumor volume compared with the BCMA UCAR-T group; compared with the BCMA-NKG2A UCAR-T group, the BCMA-NKG2A UCAR-T-NKG2A KO cell group can also significantly inhibit the growth of tumor volume.

The above results indicate that the knockout of endogenous NKG2A can significantly improve the anti-tumor activity of T cells or CAR-T cells *in vivo.*

### Example 6: preparation of CAR-T cells targeting tandem BCMA and NKG2A

In the above examples, the CARs targeting BCMA and NKG2A are expressed separately and regulated by different co-stimulatory factors. Further, we verified the effect of NKG2A knockout on anti-tumor activity and clearance of NK cells in UCAR-T cells expressing BCMA and NKG2A in tandem.

Using the methods of conventional molecular biology in the field, BCMA-loop-NKG2A (amino acid sequence as shown by SEQ ID NO: 30), NKG2A-loop-BCMA (amino acid sequence shown as SEQ ID NO: 32) (Table 1) were respectively linked with CD8 signal peptide (SEQ ID NO:41, SEQ ID NO:42), CD8hinge, transmembrane domain of CD8, 4-1BB co-stimulatory factor and T cell activating factor CD3ζ in tandem, and inserted into the vector PRRLsin to construct the vector PRRLsin-BCMA-loop-NKG2A (#1) and PRRLsin-NKG2A-loop-BCMA (#2) (FIG. 9). Exemplarily, the single-chain antibody of BCMA (the amino acid sequence of VH is as shown by SEQ ID NO: 22, the amino acid sequence of VL is as shown by SEQ ID NO: 23), the single-chain antibody of NKG2A (the amino acid sequence of VH is as shown by SEQ ID NO : 1, the amino acid sequence of VL is as shown by SEQ ID NO: 2), G4S (the amino acid sequence is as shown by SEQ ID NO: 34, the nucleotide sequence is as shown by SEQ ID NO: 35), (G4S) 3 (the amino acid sequence is as shown by SEQ ID NO:36, the nucleotide sequence is as shown by SEQ ID NO:37), linker1 (the amino acid sequence is as shown by SEQ ID NO:38, the nucleotide sequence is as shown by SEQ ID NO:39), CD8hinge (the amino acid sequence is as shown by SEQ ID NO:43, the nucleotide sequence is as shown by SEQ ID NO:44), the transmembrane domain of CD8 (the amino acid sequence is as shown by SEQ ID NO:45, the nucleotide sequence is as shown by SEQ ID NO:46), 4-1BB co-stimulatory factor (the amino acid sequence is as shown by SEQ ID NO:49, the nucleotide sequence is as shown by SEQ ID NO:50), T cell activating factor CD3ζ (the amino acid sequence is as shown by SEQ ID NO: 51, the nucleotide sequence is as shown by SEQ ID NO: 52).

**Table 1.**

| Fragment | Component |
|---|---|
| BCMA-loop-NKG2A | BCMAVL-G4S-NKG2AVH-(G4S)3-NKG2AVL-G4S-BCMAVH |
| NKG2A-loop-BCMA | NKG2AVL-G4S-BCMAVH-linker1-BCMAVL-G4S-NKG2AVH |

The above two vectors PRRLsin-BCMA-loop-NKG2A and PRRLsin-NKG2A-loop-BCMA were used to prepare BCMA-loop-NKG2A-CAR T cells and NKG2A-loop-BCMA-CAR T cells respectively according to the method described in Example 1.

According to the method described in Example 2, the above-mentioned BCMA-loop-NKG2A CAR T cells and NKG2A-loop-BCMA CAR T cells were respectively subjected to double knockout of TRAC and B2M genes or triple knockout of TRAC/B2M/NKG2A to prepare BCMA-loop - NKG2A-UCAR T cells, BCMA-loop-NKG2A UCAR-T-NKG2A KO cells, NKG2A-loop-BCMA UCAR T cells, and BCMA-loop-NKG2A UCAR-T-NKG2A KO cells.

### Example 7: detection of resistance function of tandem UCAR-T cells against NK cells in vitro

Took 5×10⁴ prepared UTD UCAR-T, BCMA UCAR-T, BCMA-loop-NKG2A uCAR-T, BCMA-loop-NKG2A UCAR-T-NKG2A KO cells, NKG2A-loop-BCMA-UCAR T cells, NKG2A-loop -BCMA UCAR-T-NKG2A KO cells and co-incubated them with activated and expanded NK cells (aNK) at a ratio of 1:1 *in vitro,* and staining for flow cytometry was performed at 0hr, 4hr, 24hr and 48hr to detect the ratio of uCAR-T cells and NK cells in the co-culture system. The results are shown in Figure 10, when co-incubating with activated and expanded NK cells, compared with UTD UCAR-T and BCMA UCAR-T cells, the proportion of BCMA-loop-NKG2A uCAR-T and NKG2A-loop-BCMA UCAR T cells increased significantly at 24 hours and 48 hours, indicating that the T cells expressing CAR targeting NKG2A can significantly improve the resistance of T cells or CAR-T cells to NK cells.

Furthermore, when co-incubating with activated and expanded NK cells, compared with BCMA-loop-NKG2A uCAR-T, the proportion of endogenous NKG2A knockout BCMA-loop-NKG2A UCAR-T-NKG2A KO cells were significantly improved at 24 hours and 48 hours. Compared with NKG2A-loop-BCMA UCAR T cells, the proportion of endogenous NKG2A knockout NKG2A-loop-BCMA UCAR T-NKG2A KO cells was significantly increased at 24 hours and 48 hours. The above results show that the knockout of endogenous NKG2A can significantly improve the resistance function of T cells or CAR-T cells to NK cells, thereby maintaining a high survival rate of T cells or CAR-T cells.

### Example 8: detection of in vivo anti-tumor function of tandem UCAR-T cells

Next, we compared the effect of NKG2A knockout on the *in vivo* antitumor activity of tandem UCAR-T. UTD UCAR-T and BCMA UCAR-T were selected as negative and positive controls respectively, NKG2A-loop-BCMA UCAR-T and NKG2A knockout NKG2A-loop-BCMA UCAR-T-NKG2A KO were selected as experimental groups.

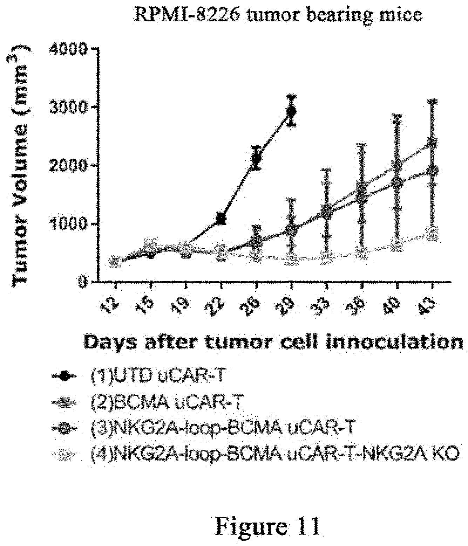

The multiple myeloma cell line RPMI-8226 was cultured *in vitro,* and 5×10⁶ cells were inoculated subcutaneously in NPG immunodeficient mice. The tumor volume was measured 12 to 14 days after inoculation for screening and grouping. The average tumor volume was about 350mm³. The mice were divided into 4 groups (UTD UCAR-T, BCMA UCAR-T, NKG2A-loop-BCMA UCAR T, NKG2A-loop-BCMA UCAR-T-NKG2A KO), with 5 mice in each group. 1.5×10⁶ UTD, BCMA UCAR-T, NKG2A-loop-BCMA UCAR T, NKG2A-loop-BCMA UCAR-T-NKG2A KO cells were injected into the tail vein respectively. After injection, body weight was measured twice a week (including the day for grouping and administration and the day for euthanasia), the long diameter and short diameter of the tumor were measured and recorded with a vernier caliper, the tumor volume was calculated, and the tumor growth curve was drawn according to the tumor volume, and the difference of the tumor growth curves were compared among the groups (tumor volume: V=1/2×long diameter× short diameter²). The test results are shown in Figure 11, compared with NKG2A-loop-BCMA-UCAR-T cells, the growth rate of tumor volume in the NKG2A-loop-BCMA-UCAR-T-NKG2A KO cell group was significantly inhibited, indicating that the knockout of endogenous NKG2A can significantly improve the *in vivo* anti-tumor activity of T cells or CAR-T cells with tandemly expressed CARs.

As described in Examples 7 and 8, in tandemly expressed CAR-T cells, the knockout of endogenous NKG2A can not only improve the anti-tumor activity of CAR-T cells (targeting tumor BCMA-CAR), but also improve the resistance/clearance ability of CAR-T cells to NK cells (targeting non-tumor antigen NKG2A-CAR). It shows that the knockout of endogenous NKG2A can significantly enhance the CAR activity targeting tumor antigens or non-tumor antigens.

### Example 9: detection of resistance function of UCAR-T to NK cells in vivo

UTD UCAR-T and BCMA UCAR-T were selected as controls, and NKG2A knockout BCMA-loop-NKG2A UCAR-T-NKG2A KO was selected as the experimental group to detect the in vivo resistance of NKG2A knockout to NK cells.

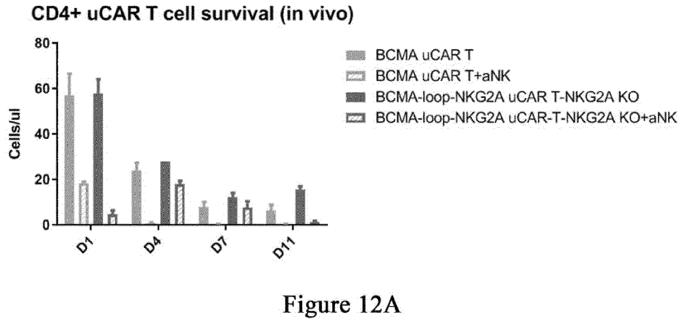

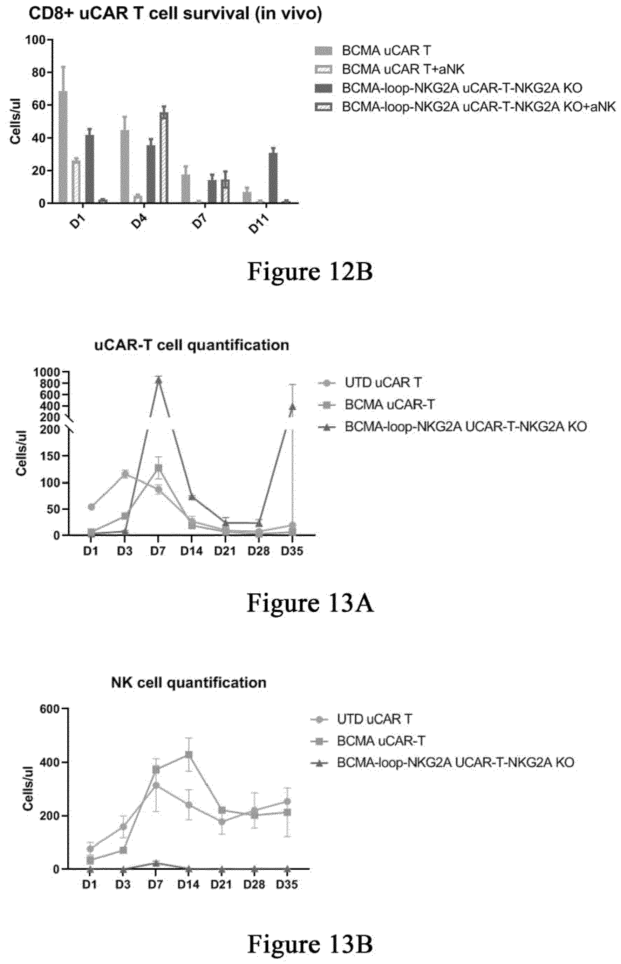

NPG immunodeficient mice were divided into 4 groups, 3 in each group: each group was respectively given BCMA UCAR-T (marked as BCMA UCAR-T), BCMA UCAR-T and aNK cell group (marked as BCMA UCAR-T + aNK), BCMA-loop-NKG2A UCAR-T-NKG2A KO cells (marked as BCMA-loop-NKG2A UCAR-T-NKG2A KO), BCMA-loop-NKG2A UCAR-T-NKG2A KO cells and aNK cell group (marked as BCMA -loop-NKG2A UCAR-T-NKG2A KO + aNK). Activated NK cells (aNK) were expanded *in vitro,* and injected into the tail vein of NPG immunodeficient mice (purchased from Beijing Vitalstar Biotechnology Co.,Ltd.) at a dose of 8×10⁶/mouse, then 8×10⁶ prepared BCMA -loop-NKG2A-UCAR-T-NKG2A KO cells and BCMA uCAR-T cells were respectively injected into the tail vein (as D0), followed by injection of 2×10⁶ aNK cells on D4 and D8 respectively. On D1, D4, D7 and D11, the peripheral blood of the mice was collected for flow cytometric detection, and CD4 and CD8 positive T cells were quantified. The results are shown in Figures 12A and 12B, on D4 and D7, the number of CD4-positive and CD8-positive T cells in BCMA-loop-NKG2A UCAR-T-NKG2A KO cells was significantly higher than that in the BCMA uCAR-T cell group, indicating that BCMA-loop-NKG2A-UCAR-T-NKG2A KO cells still had a higher survival rate with the presence of *in vivo* aNK cells. It shows that the knockout of endogenous NKG2A combined with T cells or CAR-T cells expressing NKG2A-CAR significantly improves the resistance to NK cells *in vivo.*

NPG mice overexpressing human IL-15, humanized recombinant mice (purchased from Jiangsu Jicui Yaokang Co., Ltd.), in which human immune system is reconstructed by injecting hematopoietic stem cells in order to simulate the *in vivo* immune environment. 1×10⁷ BCMA-loop-NKG2A UCAR-T-NKG2A KO, BCMA UCAR-T and UTD UAR-T cells were injected into the humanized recombinant mice through the tail vein (as D0), and on D1, D3, D7, D14, D21, D28 and D35, the peripheral blood of the mice was taken for hCD45/hHLA-ABC/hCD16 staining for flow cytometry, and the cells were quantified by TruCount tubes. As shown in Figure 13A and 13B, compared with UTD UCAR-T and BCMA uCAR-T cells, the expansion and survival of BCMA-loop-NKG2A UCAR-T-NKG2A KO cells *in vivo* were significantly improved, and BCMA-loop-NKG2A UCAR-T-NKG2A KO cells significantly suppressed the number of NK cells. The above results indicate that BCMA-loop-NKG2A UCAR-T-NKG2A KO cells can survive and expand better in immune-competent mice, which indicates that knockout of endogenous NKG2A and expression of NKG2A-CAR can significantly increase the killing of NK cells by T cells or CAR-T cells in immune-competent animals, and further improves the survival and proliferation of T cells or CAR-T cells in immune-competent animals.

### Example 10: detection of in vitro anti-tumor activity and anti-NK activity of tandem UCAR-T in the presence of NK cells

UTD UCAR-T and BCMA UCAR-T were selected as controls, and NKG2A knockout NKG2A-loop-BCMA-UCAR-T-NKG2A KO was selected as the experimental group to detect the anti-tumor activity and resistance to NK cells of tandem UCAR-T after NKG2A knockout in the presence of tumor cells and NK cells *in vitro.*

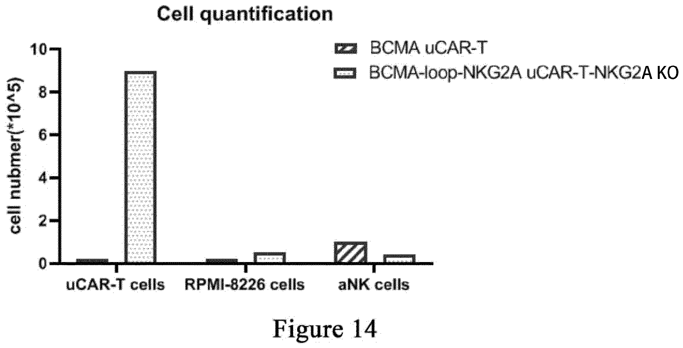

The RPMI-8226 tumor cells were stained and labeled with CFSE, took 2×10⁵ RPMI-8226 cells and inoculated them into 96-well plates according to the ratio of tumor cells: T cells: aNK cells = 1:2:2, co-culturing for 5 days, and then performed cell counts and flow cytometry staining. As shown in Figure 14, both BCMA-loop-NKG2A UCAR-T-NKG2A KO cells and BCMA uCAR-T cells can significantly kill tumor cells; the number of BCMA-loop-NKG2A UCAR-T-NKG2A KO cells is significantly bigger than that of BCMA uCAR-T cells, and the number of NK cells in the BCMA-loop-NKG2A UCAR-T-NKG2A KO cell group is smaller than that in the BCMA uCAR-T group, indicating that BCMA-loop-NKG2A UCAR-T-NKG2A KO cells can not only significantly inhibit the survival of NK cells, but also have better expansion and survival. This shows that the knockout of endogenous NKG2A and the expression of NKG2A-CAR can significantly improve the ability of T cells or CAR-T cells to inhibit the survival of NK cells, and can significantly improve the expansion and survival ability of T cells or CAR-T cells and resulting in significant ability to kill tumor cells.

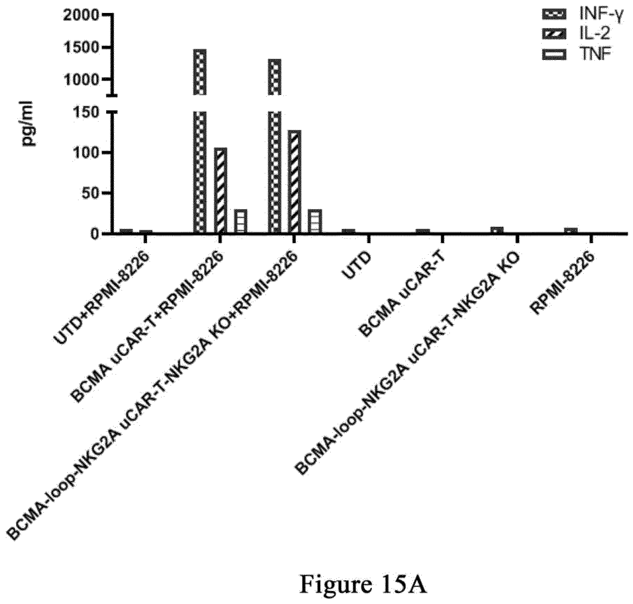

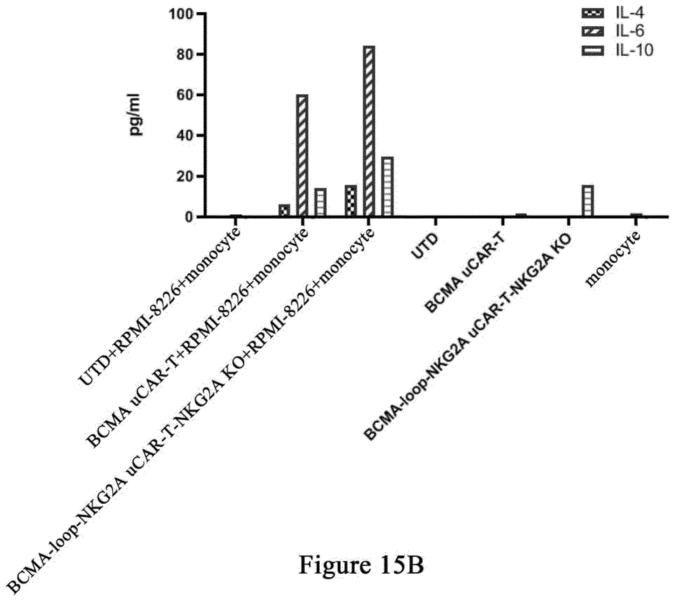

The *in vitro* cytokine secretion of BCMA-loop-NKG2A UCAR-T-NKG2A KO cells was further detected. BCMA uCAR-T and UTD UCAR-T were used as positive and negative controls respectively. 7500 RPMI-8226 cells were taken as tumor target cells, co-incubation was carried out according to T cells: tumor cells: autologous derived monocytes = 1: 1: 1, and the supernatant was taken after 24 hours for cytometric bead array detection technology (CBA) to detect the expression of INF-γ, IL2, TNF, IL4, IL6 and IL10. The results are shown in Figure 15A and 15B, BCMA-loop-NKG2A UCAR-T-NKG2A KO cells and BCMA uCAR-T have similar secretion levels of INF-γ, IL2, and TNF cytokines, indicating that BCMA-loop-NKG2A UCAR-T -NKG2A KO cells can effectively secrete cytotoxic factors and kill tumor cells; BCMA-loop-NKG2A UCAR-T-NKG2A KO cells and BCMA uCAR-T have similar secretion levels of IL4, IL6 and IL10 cytokines, indicating that BCMA-loop-NKG2A UCAR-T-NKG2A KO cells can not cause significant cytokine inflammation. The above results indicate that T cells or CAR-T cells with endogenous NKG2A knockout and NKG2A-CAR expressed can still effectively secrete cytotoxic factors and kill tumor cells.

### Example 11: detection of in vivo anti-tumor function of different forms of tandem UCAR-T cells after NKG2A knockout

Since different forms of tandem-expressing CAR-T cells may have different anti-tumor activities, we compared the *in vivo* antitumor effect of two tendem-expressed UCAR-T (BCMA-loop-NKG2A UCAR-T and NKG2A-loop-BCMA UCAR-T) after NKG2A knockdout. UTD UCAR-T was used as the negative control, and BCMA UCAR-T was used as the positive control.

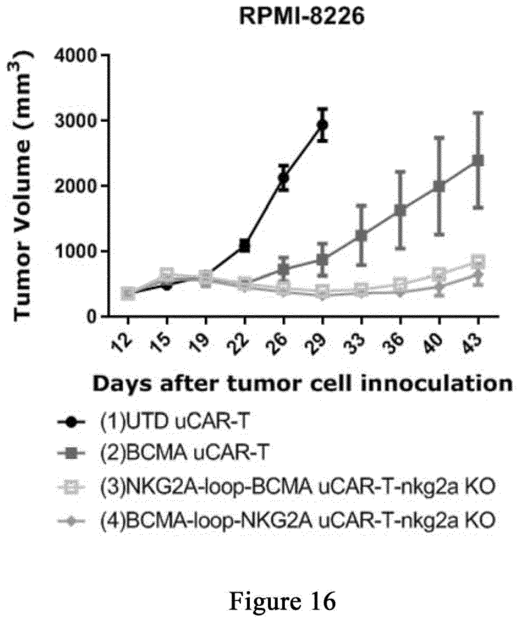

The multiple myeloma cell line RPMI-8226 was cultured *in vitro,* and 5×10⁶ cells were inoculated subcutaneously in NPG immunodeficient mice. The tumor volume was measured 12 to 14 days after inoculation for screening and grouping. The average tumor volume was about 350 mm³, the mice were divided into 4 groups (UTD UCAR-T, BCMA UCAR-T, NKG2A-loop-BCMA UCAR T-NKG2A KO, BCMA-loop-NKG2A UCAR-T-NKG2A KO), 5 mice in each group. 1.5×10⁶ UTD, BCMA UCAR-T, NKG2A-loop-BCMA UCAR T-NKG2A KO, BCMA-loop-NKG2A UCAR-T-NKG2A KO cells were injected through the tail vein respectively. After injection, the body weight was measured twice a week (including the day for grouping and administration and the day for euthanasia), the long diameter and short diameter of the tumor were measured and recorded with a vernier caliper, the tumor volume was calculated, the tumor growth curve was drawn according to the tumor volume, and the difference of tumor growth curves among groups was compared (tumor volume: V=1/2×long diameter × short diameter²). The test results are shown in Figure 16. Compared with BCMA UCAR-T, NKG2A-loop-BCMA UCAR T-NKG2A KO and BCMA-loop-NKG2A UCAR-T-NKG2A KO cells with NKG2A knockout show better anti-tumor activity, and there is no significant difference between NKG2A-loop-BCMA UCAR T-NKG2A KO and BCMA-loop-NKG2A UCAR-T-NKG2A KO cells. The above results indicate that both tandem-expressing CAR-T cells of BCMA-loop-NKG2A UCAR-T and NKG2A-loop-BCMA UCAR-T with NKG2A knockout can significantly inhibit the growth of tumor cells *in vivo,* and they have similar levels of anti-tumor activity.

In summary, NKG2 knockout can significantly improve the anti-tumor activity of CAR-T cells targeting tumor antigen BCMA (as shown in Figure 8 and Figure 11), at the same time it can also significantly improve the resistance of CAR-T targeting non-tumor antigen NKG2A to NK cells (as shown in Figure 10), indicating that NKG2A knockout can significantly improve the functional activity of T cells or CAR-T. At the same time, NKG2A knockout can enhance the functional activity of T cells, not only for single-target CAR-T cells (BCMA CAR), dual-target CAR-T cells (BCMA-NKG2A CAR), but also for different tandem-expressing CAR-T cells (BCMA-loop-NKG2A CAR, NKG2A-loop-NKG2A CAR).

All documents mentioned in this application are incorporated by reference in this application as if each were individually incorporated by reference. In addition, it should be understood that after reading the above teaching content of the present application, those skilled in the art can make various changes or modifications to the present application, and these equivalent versions also fall within the scope defined by the appended claims of the present application.

The sequences used in this application are shown in the table below:

| SEQ ID NO: | name | sequence |
|---|---|---|
| 1 | VH of anti-NKG2A | QVQLVQSGAEVKKPGASVKVSCKASGYTFTSYWMNWV |
| | antibody (amino acid sequence) | RQAPGQGLEWMGRIDPYDSETHYAQKLQGRVTMTTDTS TSTAYMELRSLRSDDTAVYYCARGGYDFDVGTLYWFFDV WGQGTTVTVSS |
| 2 | VL of anti-NKG2A antibody (amino acid sequence) | DIQMTQSPSSLSASVGDRVTITCRASENIYSYLAWYQQKP GKAPKLLIYNAKTLAEGVPSRFSGSGSGTDFTLTISSLQPE DFATYYCQHHYGTPRTFGGGTKVEIK |
| 3 | HCDR1 of anti-NKG2A antibody (amino acid sequence) | SYWMN |
| 4 | HCDR2 of anti-NKG2A antibody (amino acid sequence) | RIDPYDSETHYAQKLQG |
| 5 | HCDR3 of anti-NKG2A antibody (amino acid sequence) | GGYDFDVGTLYWFFDV |
| 6 | LCDR1 of anti-NKG2A antibody (amino acid sequence) | RASENIYSYLA |
| 7 | LCDR2 of anti-NKG2A antibody (amino acid sequence) | NAKTLAE |
| 8 | LCDR3 of anti-NKG2A antibody (amino acid sequence) | QHHYGTPRT |
| 9 | amino acid sequence of NKG2A CAR | MALPVTALLLPLALLLHAARPQVQLVQSGAEVKKPGASV KVSCKASGYTFTSYWMNWVRQAPGQGLEWMGRIDPYD SETHYAQKLQGRVTMTTDTSTSTAYMELRSLRSDDTAVY YCARGGYDFDVGTLYWFFDVWGQGTTVTVSSGGGGSG GGGSGGGGSDIQMTQSPSSLSASVGDRVTITCRASENIYS YLAWYQQKPGKAPKLLIYNAKTLAEGVPSRFSGSGSGTD FTLTISSLQPEDFATYYCQHHYGTPRTFGGGTKVEIKTTTP APRPPTPAPTIASQPLSLRPEACRPAAGGAVHTRGLDFACD FWVLVVVGGVLACYSLLVTVAFIIFWVRSKRSRLLHSDY MNMTPRRPGPTRKHYQPYAPPRDFAAYRSRVKFSRSADA PAYQQGQNQLYNELNLGRREEYDVLDKRRGRDPEMGGK PQRRKNPQEGLYNELQKDKMAEAYSEIGMKGERRRGKG HDGLYQGLSTATKDTYDALHMQALPPR |
| 10 | amino acid sequence | QVQLVQSGAEVKKPGASVKVSCKASGYTFTSYWMNWV |
| | of NKG2A scfv | RQAPGQGLEWMGRIDPYDSETHYAQKLQGRVTMTTDTS TSTAYMELRSLRSDDTAVYYCARGGYDFDVGTLYWFFDV WGQGTTVTVSSGGGGSGGGGSGGGGSDIQMTQSPSSLSA SVGDRVTITCRASENIYSYLAWYQQKPGKAPKLLIYNAK TLAEGVPSRFSGSGSGTDFTLTISSLQPEDFATYYCQHHYG TPRTFGGGTKVEIK |
| 11 | mRNA sequence of NKG2A NM_002259.5 | CCACTCTTGACTCACTCTGAGCCTTCACAGGGCAGTCT GCGAAGATTGCAGGCATTGTTTGTTCTTGTCTTGGATTT ATGCCTTTAAATTTCACCTTTTATTACACAGCTATAGCAG GCCTTTTTATGAGACTAACCTGGCCTCTCCACTAAAGGA TGTGTGACTTTCTGGGGACAGAAGAGTACAGTCCCTGA CATCACACACTGCAGAGATGGATAACCAAGGAGTAATC TACTCAGACCTGAATCTGCCCCCAAACCCAAAGAGGCA GCAACGAAAACCTAAAGGCAATAAAAACTCCATTTTAG CAACTGAACAGGAAATAACCTATGCGGAATTAAACCTT CAAAAAGCTTCTCAGGATTTTCAAGGGAATGACAAAAC CTATCACTGCAAAGATTTACCATCAGCTCCAGAGAAGC TCATTGTTGGGATCCTGGGAATTATCTGTCTTATCTTAAT GGCCTCTGTGGTAACGATAGTTGTTATTCCCTCTACATTA ATACAGAGGCACAACAATTCTTCCCTGAATACAAGAAC TCAGAAAGCACGTCATTGTGGCCATTGTCCTGAGGAGT GGATTACATATTCCAACAGTTGTTACTACATTGGTAAGG AAAGAAGAACTTGGGAAGAGAGTTTGCTGGCCTGTAC TTCGAAGAACTCCAGTCTGCTTTCTATAGATAATGAAGA AGAAATGAAATTTCTGTCCATCATTTCACCATCCTCATG GATTGGTGTGTTTCGTAACAGCAGTCATCATCCATGGGT GACAATGAATGGTTTGGCTTTCAAACATGAGATAAAAG ACTCAGATAATGCTGAACTTAACTGTGCAGTGCTACAA GTAAATCGACTTAAATCAGCCCAGTGTGGATCTTCAATA ATATATCATTGTAAGCATAAGCTTTAGAGGTAAAGCGTT TGCATTTGCAGTGCATCAGATAAATTGTATATTTCTTAAA ATAGAAATATATTATGATTGCATAAATCTTAAAATGAATT ATGTTATTTGCTCTAATAAGAAAATTCTAAATCAATTATT GAAACAGGATACACACAATTACTAAAGTACAGACATCC TAGCATTTGTGTCGGGCTCATTTTGCTCAACATGGTATT TGTGGTTTTCAGCCTTTCTAAAAGTTGCATGTTATGTGA GTCAGCTTATAGGAAGTACCAAGAACAGTCAAACCCAT GGAGACAGAAAGTAGAATAGTGGTTGCCAATGTCTGAG GGAGGTTGAAATAGGAGATGACCTCTAACTGATAGAAC GTTACTTTGTGTCGTGATGAAAACTTTCTAAATTTCAGT AGTGGTGATGGTTGTAACTCTGCGAATATACTAAACATC ATTGATTTTTAATCATTTTAAGTGCATGAAATGTATGCTT TGTACACGACACTTCAATAAAGCTATCCAGAAAAAAAA AAAAA |
| 12 | sgRNA1 targeting NKG2A (nucleotide sequence) | GGTCTGAGTAGATTACTCCT |
| 13 | sgRNA2 targeting NKG2A (nucleotide sequence) | GTAACAGCAGTCATCATCCA |
| 14 | sgRNA3 targeting NKG2A (nucleotide sequence) | GGAGCTGATGGTAAATCTGC |
| 15 | sgRNA4 targeting NKG2A (nucleotide sequence) | TGAACAGGAAATAACCTATG |
| 16 | HCDR1 of anti-BCMA antibody (amino acid sequence) | GNAMS |
| 17 | HCDR2 of anti-BCMA antibody (amino acid sequence) | AISGNGGSTFYADSVKG |
| 18 | HCDR3 of anti-BCMA antibody (amino acid sequence) | VRPFWGTFDY |
| 19 | LCDR1 of anti-BCMA antibody (amino acid sequence) | RASQSVSSSYLA |
| 20 | LCDR2 of anti-BCMA antibody (amino acid sequence) | GASSRAT |
| 21 | LCDR3 of anti-BCMA antibody (amino acid sequence) | QQYFNPPEYT |
| 22 | VH of anti-BCMA antibody (amino acid sequence) | EVQLLESGGGLVQPGGSLRLSCAASGFTFGGNAMSWVR QAPGKGLEWVSAISGNGGSTFYADSVKGRFTISRDNSKN TLYLQMNSLRAEDTAVYYCAKVRPFWGTFDYWGQGTLV TVSS |
| 23 | VL of anti-BCMA antibody (amino acid sequence) | EIVLTQSPGTLSLSPGERATLSCRASQSVSSSYLAWYQQKP GQAPRLLIYGASSRATGIPDRFSGSGSGTDFTLTISRLEPED FAVYYCQQYFNPPEYTFGQGTKVEIKR |
| 24 | amino acid sequence | EVQLLESGGGLVQPGGSLRLSCAASGFTFGGNAMSWVR |
| | of BCMA SCFV | QAPGKGLEWVSAISGNGGSTFYADSVKGRFTISRDNSKN TLYLQMNSLRAEDTAVYYCAKVRPFWGTFDYWGQGTLV TVSSGGGGSGGGGSGGGGSEIVLTQSPGTLSLSPGERATL SCRASQSVSSSYLAWYQQKPGQAPRLLIYGASSRATGIPD RFSGSGSGTDFTLTISRLEPEDFAVYYCQQYFNPPEYTFGQ GTKVEIKR |
| 25 | amino acid sequence of BCMA-CAR | MALPVTALLLPLALLLHAARPEVQLLESGGGLVQPGGSL RLSCAASGFTFGGNAMSWVRQAPGKGLEWVSAISGNGG STFYADSVKGRFTISRDNSKNTLYLQMNSLRAEDTAVYYC AKVRPFWGTFDYWGQGTLVTVSSGGGGSGGGGSGGGGS EIVLTQSPGTLSLSPGERATLSCRASQSVSSSYLAWYQQKP GQAPRLLIYGASSRATGIPDRFSGSGSGTDFTLTISRLEPED FAVYYCQQYFNPPEYTFGQGTKVEIKRTTTPAPRPPTPAPT IASQPLSLRPEACRPAAGGAVHTRGLDFACDIYIWAPLAG TCGVLLLSLVITLYCKRGRKKLLYIFKQPFMRPVQTTQEE DGCSCRFPEEEEGGCELRVKFSRSADAPAYQQGQNQLYN ELNLGRREEYDVLDKRRGRDPEMGGKPQRRKNPQEGLY NELQKDKMAEAYSEIGMKGERRRGKGHDGLYQGLSTAT KDTYDALHMQALPPR |
| 26 | amino acid sequence of BCMA-NKG2A CAR | MALPVTALLLPLALLLHAARPEVQLLESGGGLVQPGGSL RLSCAASGFTFGGNAMSWVRQAPGKGLEWVSAISGNGG STFYADSVKGRFTISRDNSKNTLYLQMNSLRAEDTAVYYC AKVRPFWGTFDYWGQGTLVTVSSGGGGSGGGGSGGGGS EIVLTQSPGTLSLSPGERATLSCRASQSVSSSYLAWYQQKP GQAPRLLIYGASSRATGIPDRFSGSGSGTDFTLTISRLEPED FAVYYCQQYFNPPEYTFGQGTKVEIKRTTTPAPRPPTPAPT IASQPLSLRPEACRPAAGGAVHTRGLDFACDIYIWAPLAG TCGVLLLSLVITLYCKRGRKKLLYIFKQPFMRPVQTTQEE DGCSCRFPEEEEGGCELRVKFSRSADAPAYQQGQNQLYN ELNLGRREEYDVLDKRRGRDPEMGGKPQRRKNPQEGLY NELQKDKMAEAYSEIGMKGERRRGKGHDGLYQGLSTAT KDTYDALHMQALPPRVKQTLNFDLLKLAGDVESNPGPM ALPVTALLLPLALLLHAARPQVQLVQSGAEVKKPGASVK VSCKASGYTFTSYWMNWVRQAPGQGLEWMGRIDPYDS ETHYAQKLQGRVTMTTDTSTSTAYMELRSLRSDDTAVYY CARGGYDFDVGTLYWFFDVWGQGTTVTVSSGGGGSGG GGSGGGGSDIQMTQSPSSLSASVGDRVTITCRASENIYSY LAWYQQKPGKAPKLLIYNAKTLAEGVPSRFSGSGSGTDF TLTISSLQPEDFATYYCQHHYGTPRTFGGGTKVEIKTTTPA PRPPTPAPTIASQPLSLRPEACRPAAGGAVHTRGLDFACDF WVLVVVGGVLACYSLLVTVAFIIFWVRSKRSRLLHSDYM NMTPRRPGPTRKHYQPYAPPRDFAAYRSRVKFSRSADAPA YQQGQNQLYNELNLGRREEYDVLDKRRGRDPEMGGKP QRRKNPQEGLYNELQKDKMAEAYSEIGMKGERRRGKGH |
| | | DGLYQGLSTATKDTYDALHMQALPPR |
| 27 | BCMA-NKG2A CAR (nucleotide sequence) | ATGGCCTTACCAGTGACCGCCTTGCTCCTGCCGCTGGC CTTGCTGCTCCACGCCGCCAGGCCGGAGGTGCAATTGC TGGAGTCTGGGGGAGGCTTGGTACAGCCTGGGGGGTC CCTGAGACTCTCCTGTGCAGCCTCCGGATTCACCTTTG GCGGTAATGCCATGTCCTGGGTCCGCCAGGCTCCAGGG AAGGGGCTGGAGTGGGTCTCAGCAATTAGTGGTAATGG TGGTAGTACATTCTACGCAGACTCCGTGAAGGGCCGGT TCACCATCTCCAGAGACAATTCCAAGAACACGCTGTAT CTGCAGATGAACAGCCTGAGAGCCGAGGACACGGCCG TATATTACTGTGCGAAAGTTCGTCCATTCTGGGGTACTT TCGACTACTGGGGCCAAGGAACCCTGGTCACCGTCTCG AGTGGTGGAGGCGGTTCAGGCGGAGGTGGTTCTGGCG GTGGCGGATCGGAAATCGTGTTAACGCAGTCTCCAGGC ACCCTGTCTTTGTCTCCAGGGGAAAGAGCCACCCTCTC TTGCAGGGCCAGTCAGAGTGTTAGCAGCAGCTACTTAG CCTGGTACCAGCAGAAACCTGGCCAGGCTCCCAGGCTC CTCATCTATGGAGCATCCAGCAGGGCCACTGGCATCCC AGACAGGTTCAGTGGCAGTGGATCCGGGACAGACTTC ACTCTCACCATCAGCAGACTGGAGCCTGAAGATTTTGC AGTGTATTACTGTCAGCAGTACTTCAACCCACCAGAATA CACGTTCGGCCAGGGGACCAAAGTGGAAATCAAACGT ACCACGACGCCAGCGCCGCGACCACCAACACCGGCGC CCACCATCGCGTCGCAGCCCCTGTCCCTGCGCCCAGAG GCGTGCCGGCCAGCGGCGGGGGGCGCAGTGCACACGA GGGGGCTGGACTTCGCCTGTGATATCTACATCTGGGCG CCCTTGGCCGGGACTTGTGGGGTCCTTCTCCTGTCACT GGTTATCACCCTTTACTGCAAACGGGGCAGAAAGAAAC TCCTGTATATATTCAAACAACCATTTATGAGACCAGTAC AAACTACTCAAGAGGAAGATGGCTGTAGCTGCCGATTT CCAGAAGAAGAAGAAGGAGGATGTGAACTGAGAGTG AAGTTCAGCAGGAGCGCAGACGCCCCCGCGTACCAGC AGGGCCAGAACCAGCTCTATAACGAGCTCAATCTAGGA CGAAGAGAGGAGTACGATGTTTTGGACAAGAGACGTG GCCGGGACCCTGAGATGGGGGGAAAGCCGCAGAGAAG GAAGAACCCTCAGGAAGGCCTGTACAATGAACTGCAG AAAGATAAGATGGCGGAGGCCTACAGTGAGATTGGGAT GAAAGGCGAGCGCCGGAGGGGCAAGGGGCACGATGG CCTTTACCAGGGTCTCAGTACAGCCACCAAGGACACCT ACGACGCCCTTCACATGCAGGCCCTGCCCCCTCGCGTG AAACAGACTTTGAATTTTGACCTTCTGAAGTTGGCAGG AGACGTTGAGTCCAACCCTGGGCCCATGGCCTTACCAG TGACCGCCTTGCTCCTGCCGCTGGCCTTGCTGCTCCAC GCCGCCAGGCCGCAGGTGCAGCTGGTGCAGAGCGGCG |
| | | CCGAGGTGAAGAAGCCCGGCGCCAGCGTGAAGGTGAG CTGCAAGGCCAGCGGCTACACCTTCACCAGCTACTGGA TGAACTGGGTGCGGCAGGCCCCCGGCCAGGGCCTGGA GTGGATGGGCCGGATCGACCCCTACGACAGCGAGACCC ACTACGCCCAGAAGCTGCAGGGCCGGGTGACCATGAC CACCGACACCAGCACCAGCACCGCCTACATGGAGCTGC GGAGCCTGCGGAGCGACGACACCGCCGTGTACTACTG CGCCCGGGGCGGCTACGACTTCGACGTGGGCACCCTGT ACTGGTTCTTCGACGTGTGGGGCCAGGGCACCACCGTG ACCGTGAGCAGCGGTGGAGGCGGTTCAGGCGGAGGTG GTTCTGGCGGTGGCGGATCGGACATCCAGATGACCCAG AGCCCCAGCAGCCTGAGCGCCAGCGTGGGCGACCGGG TGACCATCACCTGCCGGGCCAGCGAGAACATCTACAGC TACCTGGCCTGGTACCAGCAGAAGCCCGGCAAGGCCC CCAAGCTGCTGATCTACAACGCCAAGACCCTGGCCGAG GGCGTGCCCAGCCGGTTCAGCGGCAGCGGCAGCGGCA CCGACTTCACCCTGACCATCAGCAGCCTGCAGCCCGAG GACTTCGCCACCTACTACTGCCAGCACCACTACGGCAC CCCCCGGACCTTCGGCGGCGGCACCAAGGTGGAGATC AAGACCACGACGCCAGCGCCGCGACCACCAACACCGG CGCCCACCATCGCGTCGCAGCCCCTGTCCCTGCGCCCA GAGGCGTGCCGGCCAGCGGCGGGGGGCGCAGTGCACA CGAGGGGGCTGGACTTCGCCTGTGATTTTTGGGTGCTG GTGGTGGTTGGTGGAGTCCTGGCTTGCTATAGCTTGCTA GTAACAGTGGCCTTTATTATTTTCTGGGTGAGGAGTAAG AGGAGCAGGCTCCTGCACAGTGACTACATGAACATGAC TCCCCGCCGCCCCGGGCCAACCCGCAAGCATTACCAGC CCTATGCCCCACCACGCGACTTCGCAGCCTATCGCTCCA GAGTGAAGTTCAGCAGGAGCGCAGACGCCCCCGCGTA CCAGCAGGGCCAGAACCAGCTCTATAACGAGCTCAATC TAGGACGAAGAGAGGAGTACGATGTTTTGGACAAGAG ACGTGGCCGGGACCCTGAGATGGGGGGAAAGCCGCAG AGAAGGAAGAACCCTCAGGAAGGCCTGTACAATGAAC TGCAGAAAGATAAGATGGCGGAGGCCTACAGTGAGATT GGGATGAAAGGCGAGCGCCGGAGGGGCAAGGGGCAC GATGGCCTTTACCAGGGTCTCAGTACAGCCACCAAGGA CACCTACGACGCCCTTCACATGCAGGCCCTGCCCCCTC GC |
| 28 | transmembrane and intracellular domain of CD28 (amino acid sequence) | FWVLVVVGGVLACYSLLVTVAFIIFWVRSKRSRLLHSDY MNMTPRRPGPTRKHYQPYAPPRDFAAYRS |
| 29 | transmembrane and intracellular domain | TTTTGGGTGCTGGTGGTGGTTGGTGGAGTCCTGGCTTG CTATAGCTTGCTAGTAACAGTGGCCTTTATTATTTTCTGG |
| | of CD28 (nucleotide sequence) | GTGAGGAGTAAGAGGAGCAGGCTCCTGCACAGTGACT ACATGAACATGACTCCCCGCCGCCCCGGGCCAACCCGC AAGCATTACCAGCCCTATGCCCCACCACGCGACTTCGC AGCCTATCGCTCC |
| 30 | fragment of BCMA-loop-NKG2A (amino acid sequence) | EIVLTQSPGTLSLSPGERATLSCRASQSVSSSYLAWYQQKP GQAPRLLIYGASSRATGIPDRFSGSGSGTDFTLTISRLEPED FAVYYCQQYFNPPEYTFGQGTKVEIKRGGGGSQVQLVQS GAEVKKPGASVKVSCKASGYTFTSYWMNWVRQAPGQG LEWMGRIDPYDSETHYAQKLQGRVTMTTDTSTSTAYMEL RSLRSDDTAVYYCARGGYDFDVGTLYWFFDVWGQGTTV TVSSGGGGSGGGGSGGGGSDIQMTQSPSSLSASVGDRVTI TCRASENIYSYLAWYQQKPGKAPKLLIYNAKTLAEGVPS RFSGSGSGTDFTLTISSLQPEDFATYYCQHHYGTPRTFGGG TKVEIKGGGGSEVQLLESGGGLVQPGGSLRLSCAASGFTF GGNAMSWVRQAPGKGLEWVSAISGNGGSTFYADSVKGR FTISRDNSKNTLYLQMNSLRAEDTAVYYCAKVRPFWGTF DYWGQGTLVTVSS |
| 31 | fragment of BCMA-loop-NKG2A (nucleotide sequence) | GAAATCGTGTTAACGCAGTCTCCAGGCACCCTGTCTTT GTCTCCAGGGGAAAGAGCCACCCTCTCTTGCAGGGCC AGTCAGAGTGTTAGCAGCAGCTACTTAGCCTGGTACCA GCAGAAACCTGGCCAGGCTCCCAGGCTCCTCATCTATG GAGCATCCAGCAGGGCCACTGGCATCCCAGACAGGTTC AGTGGCAGTGGATCCGGGACAGACTTCACTCTCACCAT CAGCAGACTGGAGCCTGAAGATTTTGCAGTGTATTACT GTCAGCAGTACTTCAACCCACCAGAATACACGTTCGGC CAGGGGACCAAAGTGGAAATCAAACGTGGTGGAGGCG GTTCACAGGTGCAGCTGGTGCAGAGCGGCGCCGAGGT GAAGAAGCCCGGCGCCAGCGTGAAGGTGAGCTGCAAG GCCAGCGGCTACACCTTCACCAGCTACTGGATGAACTG GGTGCGGCAGGCCCCCGGCCAGGGCCTGGAGTGGATG GGCCGGATCGACCCCTACGACAGCGAGACCCACTACGC CCAGAAGCTGCAGGGCCGGGTGACCATGACCACCGAC ACCAGCACCAGCACCGCCTACATGGAGCTGCGGAGCCT GCGGAGCGACGACACCGCCGTGTACTACTGCGCCCGG GGCGGCTACGACTTCGACGTGGGCACCCTGTACTGGTT CTTCGACGTGTGGGGCCAGGGCACCACCGTGACCGTG AGCAGCGGTGGAGGCGGTTCAGGCGGAGGTGGTTCTG GCGGTGGCGGATCGGACATCCAGATGACCCAGAGCCCC AGCAGCCTGAGCGCCAGCGTGGGCGACCGGGTGACCA TCACCTGCCGGGCCAGCGAGAACATCTACAGCTACCTG GCCTGGTACCAGCAGAAGCCCGGCAAGGCCCCCAAGC TGCTGATCTACAACGCCAAGACCCTGGCCGAGGGCGTG CCCAGCCGGTTCAGCGGCAGCGGCAGCGGCACCGACT TCACCCTGACCATCAGCAGCCTGCAGCCCGAGGACTTC |
| | | GCCACCTACTACTGCCAGCACCACTACGGCACCCCCCG GACCTTCGGCGGCGGCACCAAGGTGGAGATCAAGGGC GGTGGCGGATCGGAGGTGCAATTGCTGGAGTCTGGGG GAGGCTTGGTACAGCCTGGGGGGTCCCTGAGACTCTCC TGTGCAGCCTCCGGATTCACCTTTGGCGGTAATGCCATG TCCTGGGTCCGCCAGGCTCCAGGGAAGGGGCTGGAGT GGGTCTCAGCAATTAGTGGTAATGGTGGTAGTACATTCT ACGCAGACTCCGTGAAGGGCCGGTTCACCATCTCCAGA GACAATTCCAAGAACACGCTGTATCTGCAGATGAACAG CCTGAGAGCCGAGGACACGGCCGTATATTACTGTGCGA AAGTTCGTCCATTCTGGGGTACTTTCGACTACTGGGGC CAAGGAACCCTGGTCACCGTCTCGAGT |
| 32 | fragment of NKG2A-loop-BCMA (amino acid sequence) | DIQMTQSPSSLSASVGDRVTITCRASENIYSYLAWYQQKP GKAPKLLIYNAKTLAEGVPSRFSGSGSGTDFTLTISSLQPE DFATYYCQHHYGTPRTFGGGTKVEIKGGGGSEVQLLESG GGLVQPGGSLRLSCAASGFTFGGNAMSWVRQAPGKGLE WVSAISGNGGSTFYADSVKGRFTISRDNSKNTLYLQMNS LRAEDTAVYYCAKVRPFWGTFDYWGQGTLVTVSSGSTS GSGKPGSGEGSTKGEIVLTQSPGTLSLSPGERATLSCRASQ SVSSSYLAWYQQKPGQAPRLLIYGASSRATGIPDRFSGSG SGTDFTLTISRLEPEDFAVYYCQQYFNPPEYTFGQGTKVEI KRGGGGSQVQLVQSGAEVKKPGASVKVSCKASGYTFTS YWMNWVRQAPGQGLEWMGRIDPYDSETHYAQKLQGRV TMTTDTSTSTAYMELRSLRSDDTAVYYCARGGYDFDVGT LYWFFDVWGQGTTVTVSS |
| 33 | fragment of NKG2A-loop-BCMA (nucleotide sequence) | GACATCCAGATGACCCAGAGCCCCAGCAGCCTGAGCG CCAGCGTGGGCGACCGGGTGACCATCACCTGCCGGGC CAGCGAGAACATCTACAGCTACCTGGCCTGGTACCAGC AGAAGCCCGGCAAGGCCCCCAAGCTGCTGATCTACAA CGCCAAGACCCTGGCCGAGGGCGTGCCCAGCCGGTTC AGCGGCAGCGGCAGCGGCACCGACTTCACCCTGACCA TCAGCAGCCTGCAGCCCGAGGACTTCGCCACCTACTAC TGCCAGCACCACTACGGCACCCCCCGGACCTTCGGCGG CGGCACCAAGGTGGAGATCAAGGGTGGAGGCGGTTCA GAGGTGCAATTGCTGGAGTCTGGGGGAGGCTTGGTACA GCCTGGGGGGTCCCTGAGACTCTCCTGTGCAGCCTCCG GATTCACCTTTGGCGGTAATGCCATGTCCTGGGTCCGCC AGGCTCCAGGGAAGGGGCTGGAGTGGGTCTCAGCAAT TAGTGGTAATGGTGGTAGTACATTCTACGCAGACTCCGT GAAGGGCCGGTTCACCATCTCCAGAGACAATTCCAAGA ACACGCTGTATCTGCAGATGAACAGCCTGAGAGCCGAG GACACGGCCGTATATTACTGTGCGAAAGTTCGTCCATTC TGGGGTACTTTCGACTACTGGGGCCAAGGAACCCTGGT CACCGTCTCGAGTGGTAGTACTTCTGGTTCTGGCAAAC |
| | | CGGGTTCAGGCGAAGGAAGTACAAAGGGGGAAATCGT GTTAACGCAGTCTCCAGGCACCCTGTCTTTGTCTCCAG GGGAAAGAGCCACCCTCTCTTGCAGGGCCAGTCAGAG TGTTAGCAGCAGCTACTTAGCCTGGTACCAGCAGAAAC CTGGCCAGGCTCCCAGGCTCCTCATCTATGGAGCATCC AGCAGGGCCACTGGCATCCCAGACAGGTTCAGTGGCA GTGGATCCGGGACAGACTTCACTCTCACCATCAGCAGA CTGGAGCCTGAAGATTTTGCAGTGTATTACTGTCAGCA GTACTTCAACCCACCAGAATACACGTTCGGCCAGGGGA CCAAAGTGGAAATCAAACGTGGCGGTGGCGGATCGCA GGTGCAGCTGGTGCAGAGCGGCGCCGAGGTGAAGAAG CCCGGCGCCAGCGTGAAGGTGAGCTGCAAGGCCAGCG GCTACACCTTCACCAGCTACTGGATGAACTGGGTGCGG CAGGCCCCCGGCCAGGGCCTGGAGTGGATGGGCCGGA TCGACCCCTACGACAGCGAGACCCACTACGCCCAGAA GCTGCAGGGCCGGGTGACCATGACCACCGACACCAGC ACCAGCACCGCCTACATGGAGCTGCGGAGCCTGCGGA GCGACGACACCGCCGTGTACTACTGCGCCCGGGGCGG CTACGACTTCGACGTGGGCACCCTGTACTGGTTCTTCG ACGTGTGGGGCCAGGGCACCACCGTGACCGTGAGCAG C |
| 34 | amino acid sequence of G4S | GGGGS |
| 35 | nucleotide sequence of G4S | GGTGGAGGCGGTTCA |
| 36 | amino acid sequence of (G4S)3 | GGGGSGGGGSGGGGS |
| 37 | nucleotide sequence of (G4S)3 | GGTGGAGGCGGTTCAGGCGGAGGTGGTTCTGGCGGTG GCGGATCG |
| 38 | amino acid sequence of linker1 | GSTSGSGKPGSGEGSTKG |
| 39 | nucleotide sequence of linker1 | GGTAGTACTTCTGGTTCTGGCAAACCGGGTTCAGGCGA AGGAAGTACAAAGGGG |
| 40 | F2A polypeptide fragment | VKQTLNFDLLKLAGDVESNPGP |
| 41 | amino acid sequence of CD8 signal | MALPVTALLLPLALLLHAARP |
| 42 | nucleotide sequence of CD8 signal | ATGGCCTTACCAGTGACCGCCTTGCTCCTGCCGCTGGC CTTGCTGCTCCACGCCGCCAGGCCG |
| 43 | amino acid sequence of CD8 hinge | TTTPAPRPPTPAPTIASQPLSLRPEACRPAAGGAVHTRGLD FACD |
| 44 | nucleotide sequence of CD8 hinge | ACCACGACGCCAGCGCCGCGACCACCAACACCGGCGC CCACCATCGCGTCGCAGCCCCTGTCCCTGCGCCCAGAG GCGTGCCGGCCAGCGGCGGGGGCGCAGTGCACACGA |
| | | GGGGGCTGGACTTCGCCTGTGAT |
| 45 | amino acid sequence of CD8 transmembrane region | IYIWAPLAGTCGVLLLSLVITLYC |
| 46 | nucleotide sequence of CD8 transmembrane region | ATCTACATCTGGGCGCCCTTGGCCGGGACTTGTGGGGT CCTTCTCCTGTCACTGGTTATCACCCTTTACTGC |
| 47 | amino acid sequence of CD28 transmembrane region | FWVLVVVGGVLACYSLLVTVAFIIFWV |
| 48 | nucleotide sequence of CD28 transmembrane region | TTTTGGGTGCTGGTGGTGGTTGGTGGAGTCCTGGCTTG CTATAGCTTGCTAGTAACAGTGGCCTTTATTATTTTCTGG GTG |
| 49 | amino acid sequence of 4-1BB costimulatory factor | KRGRKKLLYIFKQPFMRPVQTTQEEDGCSCRFPEEEEGGC EL |
| 50 | nucleotide sequence of 4-1BB costimulatory factor | AAACGGGGCAGAAAGAAACTCCTGTATATATTCAAACA ACCATTTATGAGACCAGTACAAACTACTCAAGAGGAAG ATGGCTGTAGCTGCCGATTTCCAGAAGAAGAAGAAGG AGGATGTGAACTG |
| 51 | amino acid sequence of T cell activating factor CD3ζ | RVKFSRSADAPAYQQGQNQLYNELNLGRREEYDVLDKR RGRDPEMGGKPQRRKNPQEGLYNELQKDKMAEAYSEIG MKGERRRGKGHDGLYQGLSTATKDTYDALHMQALPPR |
| 52 | nucleotide sequence of T cell activating factor CD3ζ | AGAGTGAAGTTCAGCAGGAGCGCAGACGCCCCCGCGT ACCAGCAGGGCCAGAACCAGCTCTATAACGAGCTCAAT CTAGGACGAAGAGAGGAGTACGATGTTTTGGACAAGA GACGTGGCCGGGACCCTGAGATGGGGGGAAAGCCGCA GAGAAGGAAGAACCCTCAGGAAGGCCTGTACAATGAA CTGCAGAAAGATAAGATGGCGGAGGCCTACAGTGAGAT TGGGATGAAAGGCGAGCGCCGGAGGGGCAAGGGGCA CGATGGCCTTTACCAGGGTCTCAGTACAGCCACCAAGG ACACCTACGACGCCCTTCACATGCAGGCCCTGCCCCCT CGC |
| 53 | nucleotide sequence of TRAC-sgRNA | AGAGTCTCTCAGCTGGTACA |
| 54 | nucleotide sequence of B2M-sgRNA | GAGTAGCGCGAGCACAGCTA |
| 55 | amino acid sequence of BCMA-loop-NKG2A | MALPVTALLLPLALLLHAARPEIVLTQSPGTLSLSPGERAT LSCRASQSVSSSYLAWYQQKPGQAPRLLIYGASSRATGIP DRFSGSGSGTDFTLTISRLEPEDFAVYYCQQYFNPPEYTFG |
| | CAR | QGTKVEIKRGGGGSQVQLVQSGAEVKKPGASVKVSCKA SGYTFTSYWMNWVRQAPGQGLEWMGRIDPYDSETHYA QKLQGRVTMTTDTSTSTAYMELRSLRSDDTAVYYCARGG YDFDVGTLYWFFDVWGQGTTVTVSSGGGGSGGGGSGG GGSDIQMTQSPSSLSASVGDRVTITCRASENIYSYLAWYQ QKPGKAPKLLIYNAKTLAEGVPSRFSGSGSGTDFTLTISSL QPEDFATYYCQHHYGTPRTFGGGTKVEIKGGGGSEVQLL ESGGGLVQPGGSLRLSCAASGFTFGGNAMSWVRQAPGK GLEWVSAISGNGGSTFYADSVKGRFTISRDNSKNTLYLQ MNSLRAEDTAVYYCAKVRPFWGTFDYWGQGTLVTVSST TTPAPRPPTPAPTIASQPLSLRPEACRPAAGGAVHTRGLDF ACDIYIWAPLAGTCGVLLLSLVITLYCKRGRKKLLYIFKQP FMRPVQTTQEEDGCSCRFPEEEEGGCELRVKFSRSADAPA YQQGQNQLYNELNLGRREEYDVLDKRRGRDPEMGGKP QRRKNPQEGLYNELQKDKMAEAYSEIGMKGERRRGKGH DGLYQGLSTATKDTYDALHMQALPPR |
| 56 | nucleotide sequence of BCMA-loop-NKG2A CAR | ATGGCCTTACCAGTGACCGCCTTGCTCCTGCCGCTGGC CTTGCTGCTCCACGCCGCCAGGCCGGAAATCGTGTTAA CGCAGTCTCCAGGCACCCTGTCTTTGTCTCCAGGGGAA AGAGCCACCCTCTCTTGCAGGGCCAGTCAGAGTGTTAG CAGCAGCTACTTAGCCTGGTACCAGCAGAAACCTGGCC AGGCTCCCAGGCTCCTCATCTATGGAGCATCCAGCAGG GCCACTGGCATCCCAGACAGGTTCAGTGGCAGTGGATC CGGGACAGACTTCACTCTCACCATCAGCAGACTGGAGC CTGAAGATTTTGCAGTGTATTACTGTCAGCAGTACTTCA ACCCACCAGAATACACGTTCGGCCAGGGGACCAAAGT GGAAATCAAACGTGGTGGAGGCGGTTCACAGGTGCAG CTGGTGCAGAGCGGCGCCGAGGTGAAGAAGCCCGGCG CCAGCGTGAAGGTGAGCTGCAAGGCCAGCGGCTACAC CTTCACCAGCTACTGGATGAACTGGGTGCGGCAGGCCC CCGGCCAGGGCCTGGAGTGGATGGGCCGGATCGACCC CTACGACAGCGAGACCCACTACGCCCAGAAGCTGCAG GGCCGGGTGACCATGACCACCGACACCAGCACCAGCA CCGCCTACATGGAGCTGCGGAGCCTGCGGAGCGACGA CACCGCCGTGTACTACTGCGCCCGGGGCGGCTACGACT TCGACGTGGGCACCCTGTACTGGTTCTTCGACGTGTGG GGCCAGGGCACCACCGTGACCGTGAGCAGCGGTGGAG GCGGTTCAGGCGGAGGTGGTTCTGGCGGTGGCGGATC GGACATCCAGATGACCCAGAGCCCCAGCAGCCTGAGC GCCAGCGTGGGCGACCGGGTGACCATCACCTGCCGGG CCAGCGAGAACATCTACAGCTACCTGGCCTGGTACCAG CAGAAGCCCGGCAAGGCCCCCAAGCTGCTGATCTACA ACGCCAAGACCCTGGCCGAGGGCGTGCCCAGCCGGTT CAGCGGCAGCGGCAGCGGCACCGACTTCACCCTGACC |
| | | ATCAGCAGCCTGCAGCCCGAGGACTTCGCCACCTACTA CTGCCAGCACCACTACGGCACCCCCCGGACCTTCGGCG GCGGCACCAAGGTGGAGATCAAGGGCGGTGGCGGATC GGAGGTGCAATTGCTGGAGTCTGGGGGAGGCTTGGTAC AGCCTGGGGGGTCCCTGAGACTCTCCTGTGCAGCCTCC GGATTCACCTTTGGCGGTAATGCCATGTCCTGGGTCCGC CAGGCTCCAGGGAAGGGGCTGGAGTGGGTCTCAGCAA TTAGTGGTAATGGTGGTAGTACATTCTACGCAGACTCCG TGAAGGGCCGGTTCACCATCTCCAGAGACAATTCCAAG AACACGCTGTATCTGCAGATGAACAGCCTGAGAGCCGA GGACACGGCCGTATATTACTGTGCGAAAGTTCGTCCATT CTGGGGTACTTTCGACTACTGGGGCCAAGGAACCCTGG TCACCGTCTCGAGTACCACGACGCCAGCGCCGCGACCA CCAACACCGGCGCCCACCATCGCGTCGCAGCCCCTGTC CCTGCGCCCAGAGGCGTGCCGGCCAGCGGCGGGGGGC GCAGTGCACACGAGGGGGCTGGACTTCGCCTGTGATAT CTACATCTGGGCGCCCTTGGCCGGGACTTGTGGGGTCC TTCTCCTGTCACTGGTTATCACCCTTTACTGCAAACGGG GCAGAAAGAAACTCCTGTATATATTCAAACAACCATTTA TGAGACCAGTACAAACTACTCAAGAGGAAGATGGCTGT AGCTGCCGATTTCCAGAAGAAGAAGAAGGAGGATGTG AACTGAGAGTGAAGTTCAGCAGGAGCGCAGACGCCCC CGCGTACCAGCAGGGCCAGAACCAGCTCTATAACGAGC TCAATCTAGGACGAAGAGAGGAGTACGATGTTTTGGAC AAGAGACGTGGCCGGGACCCTGAGATGGGGGGAAAGC CGCAGAGAAGGAAGAACCCTCAGGAAGGCCTGTACAA TGAACTGCAGAAAGATAAGATGGCGGAGGCCTACAGT GAGATTGGGATGAAAGGCGAGCGCCGGAGGGGCAAGG GGCACGATGGCCTTTACCAGGGTCTCAGTACAGCCACC AAGGACACCTACGACGCCCTTCACATGCAGGCCCTGCC CCCTCGC |
| 57 | amino acid sequence of NKG2A-loop-BCMA CAR | MALPVTALLLPLALLLHAARPDIQMTQSPSSLSASVGDRV TITCRASENIYSYLAWYQQKPGKAPKLLIYNAKTLAEGVP SRFSGSGSGTDFTLTISSLQPEDFATYYCQHHYGTPRTFGG GTKVEIKGGGGSEVQLLESGGGLVQPGGSLRLSCAASGF TFGGNAMSWVRQAPGKGLEWVSAISGNGGSTFYADSVK GRFTISRDNSKNTLYLQMNSLRAEDTAVYYCAKVRPFWG TFDYWGQGTLVTVSSGSTSGSGKPGSGEGSTKGEIVLTQS PGTLSLSPGERATLSCRASQSVSSSYLAWYQQKPGQAPRL LIYGASSRATGIPDRFSGSGSGTDFTLTISRLEPEDFAVYYC QQYFNPPEYTFGQGTKVEIKRGGGGSQVQLVQSGAEVKK PGASVKVSCKASGYTFTSYWMNWVRQAPGQGLEWMGR IDPYDSETHYAQKLQGRVTMTTDTSTSTAYMELRSLRSDD TAVYYCARGGYDFDVGTLYWFFDVWGQGTTVTVSSTTT |
| | | PAPRPPTPAPTIASQPLSLRPEACRPAAGGAVHTRGLDFAC DIYIWAPLAGTCGVLLLSLVITLYCKRGRKKLLYIFKQPFM RPVQTTQEEDGCSCRFPEEEEGGCELRVKFSRSADAPAYQ QGQNQLYNELNLGRREEYDVLDKRRGRDPEMGGKPQRR KNPQEGLYNELQKDKMAEAYSEIGMKGERRRGKGHDGL YQGLSTATKDTYDALHMQALPPR |
| 58 | nucleotide sequence of NKG2A-loop-BCMA CAR | ATGGCCTTACCAGTGACCGCCTTGCTCCTGCCGCTGGC CTTGCTGCTCCACGCCGCCAGGCCGGACATCCAGATGA CCCAGAGCCCCAGCAGCCTGAGCGCCAGCGTGGGCGA CCGGGTGACCATCACCTGCCGGGCCAGCGAGAACATCT ACAGCTACCTGGCCTGGTACCAGCAGAAGCCCGGCAA GGCCCCCAAGCTGCTGATCTACAACGCCAAGACCCTGG CCGAGGGCGTGCCCAGCCGGTTCAGCGGCAGCGGCAG CGGCACCGACTTCACCCTGACCATCAGCAGCCTGCAGC CCGAGGACTTCGCCACCTACTACTGCCAGCACCACTAC GGCACCCCCCGGACCTTCGGCGGCGGCACCAAGGTGG AGATCAAGGGTGGAGGCGGTTCAGAGGTGCAATTGCT GGAGTCTGGGGGAGGCTTGGTACAGCCTGGGGGGTCC CTGAGACTCTCCTGTGCAGCCTCCGGATTCACCTTTGG CGGTAATGCCATGTCCTGGGTCCGCCAGGCTCCAGGGA AGGGGCTGGAGTGGGTCTCAGCAATTAGTGGTAATGGT GGTAGTACATTCTACGCAGACTCCGTGAAGGGCCGGTT CACCATCTCCAGAGACAATTCCAAGAACACGCTGTATC TGCAGATGAACAGCCTGAGAGCCGAGGACACGGCCGT ATATTACTGTGCGAAAGTTCGTCCATTCTGGGGTACTTT CGACTACTGGGGCCAAGGAACCCTGGTCACCGTCTCGA GTGGTAGTACTTCTGGTTCTGGCAAACCGGGTTCAGGC GAAGGAAGTACAAAGGGGGAAATCGTGTTAACGCAGT CTCCAGGCACCCTGTCTTTGTCTCCAGGGGAAAGAGCC ACCCTCTCTTGCAGGGCCAGTCAGAGTGTTAGCAGCAG CTACTTAGCCTGGTACCAGCAGAAACCTGGCCAGGCTC CCAGGCTCCTCATCTATGGAGCATCCAGCAGGGCCACT GGCATCCCAGACAGGTTCAGTGGCAGTGGATCCGGGA CAGACTTCACTCTCACCATCAGCAGACTGGAGCCTGAA GATTTTGCAGTGTATTACTGTCAGCAGTACTTCAACCCA CCAGAATACACGTTCGGCCAGGGGACCAAAGTGGAAA TCAAACGTGGCGGTGGCGGATCGCAGGTGCAGCTGGT GCAGAGCGGCGCCGAGGTGAAGAAGCCCGGCGCCAGC GTGAAGGTGAGCTGCAAGGCCAGCGGCTACACCTTCA CCAGCTACTGGATGAACTGGGTGCGGCAGGCCCCCGGC CAGGGCCTGGAGTGGATGGGCCGGATCGACCCCTACGA CAGCGAGACCCACTACGCCCAGAAGCTGCAGGGCCGG GTGACCATGACCACCGACACCAGCACCAGCACCGCCTA CATGGAGCTGCGGAGCCTGCGGAGCGACGACACCGCC |
| | | GTGTACTACTGCGCCCGGGGCGGCTACGACTTCGACGT GGGCACCCTGTACTGGTTCTTCGACGTGTGGGGCCAGG GCACCACCGTGACCGTGAGCAGCACCACGACGCCAGC GCCGCGACCACCAACACCGGCGCCCACCATCGCGTCG CAGCCCCTGTCCCTGCGCCCAGAGGCGTGCCGGCCAG CGGCGGGGGGCGCAGTGCACACGAGGGGGCTGGACTT CGCCTGTGATATCTACATCTGGGCGCCCTTGGCCGGGAC TTGTGGGGTCCTTCTCCTGTCACTGGTTATCACCCTTTA CTGCAAACGGGGCAGAAAGAAACTCCTGTATATATTCA AACAACCATTTATGAGACCAGTACAAACTACTCAAGAG GAAGATGGCTGTAGCTGCCGATTTCCAGAAGAAGAAG AAGGAGGATGTGAACTGAGAGTGAAGTTCAGCAGGAG CGCAGACGCCCCCGCGTACCAGCAGGGCCAGAACCAG CTCTATAACGAGCTCAATCTAGGACGAAGAGAGGAGTA CGATGTTTTGGACAAGAGACGTGGCCGGGACCCTGAG ATGGGGGGAAAGCCGCAGAGAAGGAAGAACCCTCAG GAAGGCCTGTACAATGAACTGCAGAAAGATAAGATGGC GGAGGCCTACAGTGAGATTGGGATGAAAGGCGAGCGC CGGAGGGGCAAGGGGCACGATGGCCTTTACCAGGGTC TCAGTACAGCCACCAAGGACACCTACGACGCCCTTCAC ATGCAGGCCCTGCCCCCTCGC |
| 59 | amino acid sequence of NKG2A | MDNQGVIYSDLNLPPNPKRQQRKPKGNKSSILATEQEITY AELNLQKASQDFQGND-KTYHCKDLPSAPEKLIVGILGIIC LILMASVVTIVVIPSTLIQRHNNSSLNTRTQKARHCGHCPE EWITYSNSCYYIGKERRTWEESLLACTSKNSSLLSIDNEEE MKFLSIISPSSWIGVFRNSSHHPWVTMNGLAFKHEIKDSD NAELNCAVLQVNRLKSAQCGSSIIYHCKHKL |
| 60 | NKG2A-targeting sgRNA6 (nucleotide sequence) | ACTGCAGAGATGGATAACCA |
| 61 | NKG2A-targeting sgRNA10 (nucleotide sequence) | GCTCCAGAGAAGCTCATTGT |
| 62 | NKG2A-targeting sgRNA11 (nucleotide sequence) | TTGAAGGTTTAATTCCGCAT |
| 63 | NKG2A-targeting sgRNA12 (nucleotide sequence) | AACAACTATCGTTACCACAG |
| 64 | NKG2A-targeting sgRNA13 (nucleotide sequence) | AAGCTCATTGTTGGGATCCT |
| 65 | NKG2A-targeting sgRNA14 (nucleotide | GACATCACACACTGCAGAGA |
| | sequence) | |
| 66 | NKG2A-targeting sgRNA15 (nucleotide sequence) | GTGGCCATTGTCCTGAGGAG |
| 67 | NKG2A-targeting sgRNA16 (nucleotide sequence) | TCTTCGAAAATAGACTTACA |
| 68 | NKG2A-targeting sgRNA17 (nucleotide sequence) | ACTGGAGTTCTTCGAAGTAC |

## Claims

1. An engineered cell, wherein the engineered T cell is genetically engineered to reduce the expression, activity and/or signaling of NKG2A in the cell.

2. The engineered T cell according to claim 1, wherein the engineered T cell comprises:
a gene disruption of a gene encoding NKG2A, and/or a gene disruption resulting in reduced expression of NKG2A in the engineered T cell;
a gene disruption of a gene encoding a protein regulating the expression or activity of NKG2A, and/or a gene disruption resulting in reduced expression of the protein regulating the expression or activity of NKG2A; and/or
a gene disruption of a gene encoding a protein involved in NKG2A-dependent signaling, and/or a gene disruption resulting in reduced expression of a protein involved in NKG2A-dependent signaling.

3. The engineered T cell according to claim 2, wherein gene editing technology and/or gene silencing technology is used to result in reduced expression of NKG2A, reduced expression of the protein regulating the expression or activity of NKG2A, and/or reduced expression of the protein involved in NKG2A-dependent signaling.

4. The engineered T cell according to claim 3, wherein the endogenous NKG2A gene/a gene encoding the protein regulating the expression or activity of NKG2A/a gene encoding the protein involved in NKG2A-dependent signaling in the engineered T cell is knocked out by gene editing technology;
preferably, the gene editing technology is selected from the group consisting of: CRISPR/Cas9 technology, ZFN technology, TALE technology, TALE-CRISPR/Cas9 technology, Base Editor technology, prime editing technology, and/or homing endonuclease technology;
more preferably, the gene editing technology is CRISPR/Cas9 technology.

5. The engineered T cell according to claim 4, wherein CRISPR/Cas9 technology is used to knock out the endogenous NKG2A gene in the engineered T cell.

6. The engineered T cell according to claim 5, wherein the gRNA used in CRISPR/Cas9 technology is as shown by a sequence selected from the group consisting of: SEQ ID NO:12, SEQ ID NO:13, SEQ ID NO:14, SEQ ID NO:15, SEQ ID NO:60, SEQ ID NO:61, SEQ ID NO:62, SEQ ID NO:63, SEQ ID NO:64, SEQ ID NO:65, SEQ ID NO:66, SEQ ID NO:67, and/or SEQ ID NO:68.

7. The engineered T cell according to any one of claims 1 to 6, wherein the engineered T cell is a T cell derived from a natural T cell, and/or a T cell generated by inducing a pluripotent stem cell;
preferably, the T cell is an autologous/allogeneic T cell;
preferably, the T cell is a primary T cell;
preferably, the T cell is derived from a human autologous T cell.

8. The engineered T cell according to claim 7, wherein the T cell comprises a memory stem cell-like T cell (Tscm cell), a central memory T cell (Tcm), an effector T cell (Tef), a regulatory T cell (treg), an effector memory T cell (Tern), a γδ T cell, or a combination thereof.

9. The engineered T cell according to any one of claims 1 to 8, wherein the engineered T cell expresses an exogenous protein-1 targeting one or more autologous or allogeneic immune cells or immunoregulatory cells;
preferably, the immune cells or immunoregulatory cells comprise NK cells, activated T cells, Treg cells, myeloid-derived immunosuppressive cells, macrophages, or a combination thereof.

10. The engineered T cell according to claim 9, wherein the exogenous protein-1 targets NK cells.

11. The engineered T cell according to claim 10, wherein the exogenous protein-1 targets NKG2A.

12. The engineered T cell according to claim 11, wherein the exogenous protein-1 comprises an antibody recognizing NKG2A or a functional fragment thereof;
preferably, the antibody recognizing NKG2A or a functional fragment thereof comprises HCDR1 as shown by SEQ ID NO:3, HCDR2 as shown by SEQ ID NO:4, HCDR3 as shown by SEQ ID NO:5, LCDR1 as shown by SEQ ID NO:6, LCDR2 as shown by SEQ ID NO: 7, and/or LCDR3 as shown by SEQ ID NO: 8; alternatively, the antibody recognizing NKG2A or a functional fragment thereof comprises the heavy chain variable region as shown by SEQ ID NO:1 and/or the light chain variable region as shown by SEQ ID NO:2; alternatively, the antibody recognizing NKG2A or a functional fragment thereof comprises the sequence as shown by SEQ ID NO:10.

13. The engineered T cell according to any one of claims 1 to 12, wherein the engineered T cell further expresses an exogenous protein-2, and the exogenous protein-2 recognizes a tumor antigen or a pathogen antigen;
preferably, the tumor antigen comprises BCMA, CD19, GPC3, Claudin18.2, EGFR, or a combination thereof.

14. The engineered T cell according to claim 13, wherein the exogenous protein-2 comprises an antibody recognizing BCMA or a functional fragment thereof;
preferably, the antibody recognizing BCMA or a functional fragment thereof comprises HCDR1 as shown by SEQ ID NO: 16, HCDR2 as shown by SEQ ID NO: 17, HCDR3 as shown by SEQ ID NO: 18, LCDR1 as shown by SEQ ID NO: 19, LCDR2 as shown by SEQ ID NO:20, and/or LCDR3 as shown by SEQ ID NO:21; alternatively, the antibody recognizing BCMA or a functional fragment thereof comprises the heavy chain variable region as shown by SEQ ID NO:22 and/or the light chain variable region as shown by SEQ ID NO: 23; alternatively, the antibody recognizing BCMA or a functional fragment thereof comprises the sequence as shown by SEQ ID NO:24.

15. The engineered T cell according to claim 13 or 14, wherein the engineered T cell expresses exogenous protein-1 and exogenous protein-2, the exogenous protein-1 targets NKG2A, and the exogenous protein-2 targets BCMA;
preferably, the engineered T cell comprises the sequence as shown by SEQ ID NO:26 or SEQ ID NO:27.

16. The engineered T cell according to any one of claims 9 to 12, wherein the exogenous protein-1 further targets a tumor or a pathogen;
preferably, the tumor expresses BCMA, CD19, GPC3, Claudin18.2, EGFR or a combination thereof.

17. The engineered T cell according to claim 16, wherein the exogenous protein-1 further comprises an antibody recognizing BCMA or a functional fragment thereof;
preferably, the antibody recognizing BCMA or a functional fragment thereof comprises HCDR1 as shown by SEQ ID NO: 16, HCDR2 as shown by SEQ ID NO: 17, HCDR3 as shown by SEQ ID NO: 18, LCDR1 as shown by SEQ ID NO: 19, LCDR2 as shown by SEQ ID NO:20, and/or LCDR3 as shown by SEQ ID NO:21; alternatively, the antibody recognizing BCMA or a functional fragment thereof comprises the heavy chain variable region as shown by SEQ ID NO:22 and/or the light chain variable region as shown by SEQ ID NO: 23; alternatively, the antibody recognizing BCMA or a functional fragment thereof comprises the sequence as shown by SEQ ID NO:24.

18. The engineered T cell according to claim 16 or 17, wherein the exogenous protein-1 comprises an antibody recognizing NKG2A or a functional fragment thereof and an antibody recognizing a tumor antigen or a functional fragment thereof, and their linkage mode is that:
(1) the light chain/heavy chain (or light chain variable region/heavy chain variable region) of the antibody recognizing NKG2A—the heavy chain/light chain (or heavy chain variable region/light chain variable region) of the antibody recognizing NKG2A—the heavy chain/light chain (or heavy chain variable region/light chain variable region) of the antibody recognizing a tumor antigen-the light chain/heavy chain (or the light chain variable region/heavy chain variable region) of the antibody recognizing a tumor antigen;
(2) the light chain (or light chain variable region) of the antibody recognizing a tumor antigen-the heavy chain (or heavy chain variable region) of the antibody recognizing NKG2A—the light chain (or light chain variable region) of the antibody recognizing NKG2A—the heavy chain (or heavy chain variable region) of the antibody recognizing a tumor antigen; and/or
(3) the light chain (or light chain variable region) of the antibody recognizing NKG2A—the heavy chain (or heavy chain variable region) of the antibody recognizing a tumor antigen-the light chain (or light chain variable region) of the antibody recognizing a tumor antigen-the heavy chain (or heavy chain variable region) of the antibody recognizing NKG2A;
preferably, the exogenous protein-1 comprises the amino acid sequence as shown by SEQ ID NO:30 and/or SEQ ID NO:32.

19. The engineered T cell according to any one of claims 9 to 18, wherein the exogenous protein-1 and/or exogenous protein-2 is a chimeric antigen receptor (CAR), chimeric T cell receptor, T cell antigen coupler (TAC), or a combination thereof;
preferably, the CAR comprises:
(i) an antibody specifically binding to a target antigen or a functional fragment thereof, the transmembrane region of CD28 or CD8, and CD3ζ;
(ii) an antibody specifically binding to a target antigen or a functional fragment thereof, the transmembrane region of CD28 or CD8, the co-stimulatory signal domain of CD28, and CD3ζ;
(iii) an antibody specifically binding to a target antigen or a functional fragment thereof, the transmembrane region of CD28 or CD8, the co-stimulatory signal domain of CD137, and CD3ζ; and/or
(iv) an antibody specifically binding to a target antigen or a functional fragment thereof, the transmembrane region of CD28 or CD8, the co-stimulatory signal domain of CD28, the co-stimulatory signal domain of CD137, and CD3ζ.

20. The engineered T cell according to claim 19, wherein the exogenous protein-1 is a chimeric antigen receptor-1, and the target antigen recognized by the chimeric antigen receptor-1 comprises a molecular marker of a T cell, an NK cell, a Treg cell, a myeloid-derived immunosuppressive cell (MDSC), or a macrophage, or a combination thereof; alternatively, the target antigen recognized by the chimeric antigen receptor-1 further comprises a tumor antigen and/or a pathogen antigen; and wherein the exogenous protein-2 is a chimeric antigen receptor-2, and the target antigens recognized by the chimeric antigen receptor-2 comprises a tumor antigen and/or a pathogen antigen.

21. The engineered T cell according to claim 20, wherein the chimeric antigen receptor-1 targets NKG2A or targets NKG2A and BCMA; and the chimeric antigen receptor-2 targets BCMA;
preferably, the chimeric antigen receptor-1 comprises the sequence as shown by SEQ ID NO: 9, SEQ ID NO: 30, SEQ ID NO: 32, SEQ ID NO: 55, and/or SEQ ID NO: 57; and the chimeric antigen receptor-2 comprises the sequence as shown by SEQ ID NO: 25.

22. The engineered T cell according to any one of claims 1 to 21, wherein the engineered T cell further comprises:
the knockout of a gene encoding a TCR protein and/or low expression or no expression of an endogenous TCR molecule;
the knockout of a gene encoding an MHC protein and/or low expression or no expression of an endogenous MHC;
preferably, the TCR is a TRAC molecule, and the MHC is a B2M molecule.

23. The engineered T cell according to claim 22, wherein CRISPR/Cas9 technology is used to knock out the endogenous MHC and the endogenous TCR; preferably, when using CRISPR/Cas9 technology, the gRNA used to knock out B2M is selected from the sequence as shown by SEQ ID NO: 54, and the gRNA used to knock out TRAC is selected from the sequence as shown by SEQ ID NO:53.

24. The engineered T cell according to any one of claims 1 to 23, wherein compared with a T cell that has not been engineered, the engineered T cell has immune activity increased by about 10%, 20%, 30%, 40%, 50%, 60%, 70%, 80%, 90%, or 100%.

25. The engineered T cell according to any one of claims 1 to 24, wherein compared with a T cell that has not been engineered, the engineered T cell has resistance to NK cells and/or killing ability to NK cells increased by about 10%, 20%, 30%, 40%, 50%, 60%, 70%, 80%, 90% or 100%.

26. The engineered T cell according to any one of claims 1 to 25, wherein compared with a T cell that has not been engineered, the engineered T cell inhibits tumor cell proliferation, and/or inhibits tumor cell proliferation and tumor volume increase *in vivo* by about 10%, 20%, 30% 40%, 50%, 60%, 70%, 80%, 90%, or 100%.

27. The engineered T cell according to any one of claims 1 to 26, wherein compared with a T cell that has not been engineered, the expression, activity and/or signaling of NKG2A in the activated engineered T cell is reduced by greater than about 10%, 20%, 30%, 40%, 50%, 60%, 70%, 80%, 90%, 95%, or 100%.

28. The engineered T cell according to any one of claims 1 to 27, wherein the engineered T cell can improve the kiling of tumor cells by T cells and/or CAR-T cells targeting target antigens that are introduced into the subject earlier, simultaneously, and later, and the engineered T cell can also enhance the survival and proliferation of the T cells and/or CAR-T cells.

29. **A** nucleic acid, expression vector, or virus for use in preparing the engineered T cell according to any one of claims 1 to 28.

30. A method for preparing the engineered T cell according to any one of claims 1 to 28, or a method for preparing the nucleic acid, expression vector, or virus according to claim 29.

31. A method for producing an engineered T cell, comprising genetically engineering a T cell to reduce the expression, activity and/or signaling of NKG2A in the cell;
preferably, reducing the expression comprises:
disrupting or inhibiting a gene encoding NKG2A in the T cell, and/or disrupting or inhibiting a gene in the T cell to reduce the expression of NKG2A;
disrupting or inhibiting a gene encoding a protein regulating the expression or activity of NKG2A in the T cell, and/or disrupting or inhibiting a gene in the T cell to reduce the expression of a protein regulating the expression or activity of NKG2A; and/or
disrupting or inhibiting a gene encoding a protein involved in NKG2A-dependent signaling in the T cell, and/or disrupting or inhibiting a gene in the T cell to reduce the expression of a protein involved in NKG2A-dependent signaling.

32. The method according to claim 31, wherein gene disruption is achieved by introducing into the T cell an endonuclease targeting the gene;
preferably, the endonuclease is selected from the group consisting of: TAL nuclease, meganuclease, zinc finger nuclease, Cas9 and Argonaute;
preferably, CRISPR/Cas9 technology is used to knockout the endogenous NKG2A gene of the T cell.

33. The method according to claim 31 or 32, wherein compared with the expression in a T cell that has not been engineered, the expression, activity and/or signaling of NKG2A in the engineered T cell is reduced by greater than or greater than about 10%, 20%, 30%, 40%, 50%, 60%, 70%, 80%, 90%, 95% or 100%.

34. The method according to any one of claims 31 to 33, wherein the engineered T cell is derived from a T cell isolated from a human;
preferably, the T cell is isolated from peripheral blood mononuclear cell (PBMC);
preferably, the T cell is a primary T cell.

35. The method according to any one of claims 31 to 34, wherein the cell is genetically engineered to express chimeric antigen receptor (CAR), modified T cell (antigen) receptor (TCR), T cell fusion protein (TFP), T cell antigen coupler (TAC), aTCR-T, or a combination thereof;
preferably, the CAR comprises:
(i) an antibody specifically binding to a target antigen or a functional fragment thereof, the transmembrane region of CD28 or CD8, the co-stimulatory signal domain of CD28, and CD3ζ; and/or
(ii) an antibody specifically binding to a target antigen or a functional fragment thereof, the transmembrane region of CD28 or CD8, the co-stimulatory signal domain of CD28, and CD3ζ; and/or
(iii) an antibody specifically binding to a target antigen or a functional fragment thereof, the transmembrane region of CD28 or CD8, the co-stimulatory signal domain of CD137, and CD3ζ; and/or
(vi) an antibody specifically binding to a target antigen or a functional fragment thereof, the transmembrane region of CD28 or CD8, the costimulatory signal domain of CD28, the costimulatory signal domain of CD137, and CD3ζ.

36. The method according to claim 35, wherein the CAR comprises an antibody capable of specifically recognizing BCMA:
the antibody specifically recognizing BCMA comprises HCDR1 as shown by SEQ ID NO:16, HCDR2 as shown by SEQ ID NO:17, HCDR3 as shown by SEQ ID NO:18, LCDR1 as shown by SEQ ID NO:19, LCDR2 as shown by SEQ ID NO:20, and/or LCDR3 as shown by SEQ ID NO:21; or comprises the heavy chain variable region as shown by SEQ ID NO:22 and/or the light chain variable region as shown by SEQ ID NO:23; or comprises the sequence as shown by SEQ ID NO:24.

37. The method according to any one of claims 31 to 36, wherein it further comprises genetically engineering the T cell to reduce the expression, activity and/or signaling of TCR and MHC in the cell;
preferably, the TCR comprises TRAC, and the MHC comprises B2M.

38. The method according to claim 37, preferably, wherein an endonuclease targeting the gene is introduced into the T cell to achieve gene disruption; preferably, the endonuclease is selected from the group consisting of: TAL nuclease, meganuclease, zinc finger nuclease, Cas9, and Argonaute;
preferably, CRISPR/Cas9 technology is used to knock out TRAC and B2M genes in the T cell.

39. The method according to claim 37 or 38, wherein compared with the expression in a T cell that has not been engineered, the TCR and MHC expression, activity and/or signaling in the genetically engineered T cell is reduced by greater than or greater than about 50%, 60%, 70%, 80%, 90% or 95%.

40. The method according to any one of claims 31 to 39, wherein the T cell is genetically engineered to express the antibody recognizing NKG2A or a functional fragment thereof; and/or to express a chimeric antigen receptor (CAR), a modified T cell (antigen) receptor (TCR), T cell fusion protein (TFP), T cell antigen coupler (TAC) or aTCR-T that specifically binds to NKG2A, or a combination thereof;
preferably, the CAR comprises:
(i) an antibody specifically binding to NKG2A or a functional fragment thereof, the transmembrane region of CD28 or CD8, the co-stimulatory signal domain of CD28, and CD3ζ; and/or
(ii) an antibody specifically binding to NKG2A or a functional fragment thereof, the transmembrane region of CD28 or CD8, the co-stimulatory signal domain of CD28, and CD3ζ; and/or
(iii) an antibody specifically binding to NKG2A or a functional fragment thereof, the transmembrane region of CD28 or CD8, the co-stimulatory signal domain of CD137, and CD3ζ; and/or
(vi) an antibody specifically binding to NKG2A or a functional fragment thereof, the transmembrane region of CD28 or CD8, the costimulatory signal domain of CD28, the costimulatory signal domain of CD137, and CD3ζ.

41. The method according to claim 40, wherein the antibody specifically binding to NKG2A comprises HCDR1 as shown by SEQ ID NOG, HCDR2 as shown by SEQ ID NO:4, HCDR3 as shown by SEQ ID NO:5, LCDR1 as shown by SEQ ID NO:6, LCDR2 as shown by SEQ ID NO:7, and/or LCDR3 as shown by SEQ ID NO:8; alternatively, the antibody specifically binding to NKG2A comprises the heavy chain variable region as shown by SEQ ID NO:1 and/or the light chain variable region as shown by SEQ ID NO:2; alternatively, the antibody specifically binding to NKG2A comprises the sequence as shown by SEQ ID NO:10.

42. An engineered T cell produced by the method according to any one of claims 31 to 41.

43. A composition, comprising: an effective amount of the engineered T cells according to any one of claims 1-28 and 42, and/or an effective amount of the nucleic acid, expression vector, virus or a combination thereof for use in preparing the engineered T cell according to any one of claims 1-28 and 42; preferably, the composition further comprises a pharmaceutically acceptable carrier;
preferably, the carrier is saline solution, dextrose solution, or 5% human serum albumin;
preferably, the composition further comprises a cryoprotectant.

44. A kit, wherein it comprises the engineered T cell according to any one of claims 1-28 and 42, the nucleic acid, expression vector, or virus according to claim 29, and/or the composition according to claim 43 and an additional agent for treating a disease.

45. A method for treating a disease, comprising administering to a subject in need the engineered T cell according to any one of claims 1-28 and 42, the nucleic acid, expression vector or virus according to claim 29, the composition according to claim 43, and/or the kit according to claim 44;
preferably, before administering the engineered T cell, the engineered T cell is produced by the method according to any one of claims 31 to 41;
preferably, the subject is a human;
preferably, wherein the engineered T cell is an autologous or allogeneic T cell.

46. The method according to claim 45, wherein the disease comprises immune disease;
preferably, the immune disease comprises autoimmune disease and/or anti-graft immune rejection.

47. The method of claim 44, wherein the disease comprises tumor;
preferably, the tumor comprises hematologic tumor and/or solid tumor.
